# EUROPEAN PATENT APPLICATION

(11) **EP 4 803 126 A2**
(43) Date of publication of application: **09.09.2026**
(21) Application number: 26179409.3
(22) Date of filing: 13.02.2019
(51) Int. Cl.: A61M 16/10

(54) **A COLLAPSIBLE CONDUIT, PATIENT INTERFACE AND HEADGEAR CONNECTOR**

(30) Priority: 13.02.2018 US 201862629903 P; 13.06.2018 US 201862684371 P
(62) Document number(s) of the earlier application(s) in accordance with Art. 76 EPC: 19754802.7 / 3 752 232
(71) Applicant: Fisher & Paykel Healthcare Limited, 2013 Auckland (NZ)
(72) Inventor: EVANS, Alicia Jerram Hunter, 2013 Auckland (NZ); KLINK, German, 2013 Auckland (NZ); WHITE, Craig Karl, 2013 Auckland (NZ); PAYTON, Matthew Jon, 2013 Auckland (NZ); SMITH, Graeme Matthew, 2013 Auckland (NZ); OSBORNE, Hamish Adrian, 2013 Auckland (NZ); HOLYOAKE, Bruce Gordon, 2013 Auckland (NZ); FLYNN, Cormac Nicholas, 2013 Auckland (NZ); BURGESS, Aidan Robert, 2013 Auckland (NZ); ROA, Nathan James, 2013 Auckland (NZ)
(74) Representative: Forresters IP LLP

(57) **Abstract**

This invention relates to a patient interface comprising at least one nasal prong or an outlet of said patient interface to be received by a user's nare(s) or mouth; a gases delivery side member extending from a side of the at least one nasal prong or said outlet; and wherein the gases delivery side member comprises of a lumen for a flow of gases from an inlet of the patient interface to the at least one nasal prong or said outlet; a collapsible portion; at least one elbow portion or flexible portion, located substantially at or toward one or both of a downstream end of said gases delivery member or an upstream end of said gases delivery member.

## Description

### TECHNICAL FIELD

This disclosure relates to various patient interfaces and particularly to patient interfaces for use with a high flow system. The patient interfaces include deliberately collapsible features to facilitate the stopping of gas flow through the patient interface and/or allow a face mask to be used over the patient interface while positioned on a user's face. The disclosure also relates to headgear connectors, and to breathing conduits with a collapsible portion.

### BACKGROUND ART

Patients may lose respiratory function during anaesthesia, or sedation, or more generally during certain medical procedures. Prior to a medical procedure a patient may be pre-oxygenated by a medical professional to provide a reservoir of oxygen saturation, and this pre-oxygenation and CO2 flushing/washout may be carried out with a high flow therapy via a nasal cannula or other patient interface.

Once under general anaesthesia, patients must be intubated to ventilate the patient. In some cases, intubation is completed in 30 to 60 seconds, but in other cases, particularly if the patient's airway is difficult to traverse (for example, due to cancer, severe injury, obesity or spasm of the neck muscles), intubation will take significantly longer. While pre-oxygenation provides a buffer against declines in oxygen saturation, for long intubation procedures, it is necessary to interrupt the intubation process and increase the patient's oxygen saturation to adequate levels. The interruption of the intubation process may happen several times for difficult intubation processes, which is time consuming and puts the patient at severe health risk. After approximately three attempts at intubation the medical procedure, such as an intubation method will be abandoned.

In the event that manual ventilation of the apnoeic, non-intubated, patient is urgently required (such as due to unsuccessful intubation of the patient) it is necessary to quickly remove the high flow patient interface and then apply a non-invasive ventilation mask, e.g. a face mask and bag, to the patient. A cannula may be difficult to remove quickly from the patient, for example connectors between headgear and a cannula may be difficult to release quickly or manipulate with one hand. Failure to remove the patient interface may result in the cushion of the face mask overlying the patient interface or patient interface gases supply tube, disrupting the seal between the face mask and the patient's face. Gases may consequently leak from the face mask during ventilation, rendering ventilation ineffective or inefficient.

In this specification, where reference has been made to external sources of information, including patent specifications and other documents, this is generally for the purpose of providing a context for discussing the features of the present invention. Unless stated otherwise, reference to such sources of information is not to be construed, in any jurisdiction, as an admission that such sources of information are prior art or form part of the common general knowledge in the art.

### SUMMARY

It is an object of this disclosure to provide a breathing conduit or a patient interface or a headgear connector which goes at least some way towards overcoming one or more of the above mentioned problems or difficulties, or to provide the industry/public with a useful choice.

In accordance with at least one of the embodiments disclosed herein, a breathing conduit for providing a flow of respiratory gases, whether formed with a patient interface or as a separate conduit, comprises a collapsible portion, a lateral cross section of the collapsible portion comprising:
a first side comprising a flat portion for positioning against a user's face,
a second side opposite the first side and facing away from the user's face,
the first and second sides joined by first and second fold points, in an open configuration the fold points spaced away from the flat portion of the first side in a direction away from the user's face in use,
wherein an inner length of the first side between the fold points and an inner length of the second side between the fold points are substantially equal, and in a partially closed or closed configuration the second side being moved towards or against the first side with the collapsible portion folding at the first and second fold points.

In some embodiments, the first side comprises an outwardly curved portion between the flat portion and each of the first and second fold points when in the open configuration.

In some embodiments, the thickness of the outwardly curved portion tapers from the flat portion towards the respective fold point, from a greater thickness to a reduced thickness.

In some embodiments, the first side is curved outwardly when in the open configuration.

In some embodiments, the second side is curved outwardly when in the open configuration.

In some embodiments, in the closed configuration the fold points are moved to be against or adjacent the face of a user.

In some embodiments, the thickness of the first side and/or the second side tapers towards each fold point, from a greater thickness to a reduced thickness.

In some embodiments, a maximum thickness of the first side is at an apex of the first side and/or a maximum thickness of the second side is at an apex of the second side.

In some embodiments, the thickness of the fold points is less than the thickness of the remainder of the cross section of the collapsible portion.

In some embodiments, the second side is thinner than the first side.

In some embodiments, in the open configuration the first side adjacent each fold point is at an angle to the flat portion such that an external angle between the first side adjacent the fold point and the flat portion is less than 80 degrees, or less than 75 degrees, or less than 70 degrees, or less than 65 degrees, or less than 60 degrees, or less than 55 degrees, or less than 50 degrees, or less than 45 degrees, or less than 40 degrees, or less than 35 degrees, or less than 30 degrees, or is between 50 and 70 degrees, or is between 60 and 70 degrees, or about 62 to 68 degrees, or is about 64 to 66 degrees, or is about 65 degrees.

In some embodiments, in the open configuration a line tangential to the portion of the first side adjacent to each folding point is an angle to a line extending between the first and second fold points such that an angle (beta) between the line and the portion adjacent the fold point is less than 70 degrees, or less than 65 degrees, or less than 60 degrees, or less than 55 degrees, or less than 50 degrees, or less than 45 degrees, or less than 40 degrees, or less than 35 degrees, or less than 30 degrees, or is between 30 and 60 degrees, or is between 40 and 50 degrees, or may be about 45 degrees.

In some embodiments, the flat portion has a thickness of about 0.5mm, and the fold points have a thickness of about 0.2mm.

In some embodiments, the flat portion has a length of about 5mm to 10mm or about 7mm, and/or
wherein a lateral width of the cross section of the collapsible portion is between 10mm and 15mm or about 13mm.

In some embodiments, the first side tapers from a thickness of 0.5mm to a thinner thickness at the fold point.

In some embodiments, the ratio of:
i) the thickness of the (thicker) centre of the first and/or second sides of the lateral cross section and thickness of the (thinner) fold points is in the range of about 1 to 8, or about 1.5 to 3.5, or
ii) the thickest part of the lateral cross section to the thinnest part of the lateral cross section being the fold points is in the range of about 1 to 8, or about 1.5 to 3.5

In some embodiments, the ratio of the relative thicknesses between the (thicker) flat portion of the first side and the (thinner) fold points is in the range of about 1 to 8, or about 1.5 to 3.5.

In some embodiments, the first and second fold points delimit or define the extent of the first and second sides, or the first and second sides each extend fully between the fold points, e.g. from the first fold point to the second fold point.

In some embodiments, the collapsible section has reflective symmetry about a centre line of the cross section, the centre line extending through a centre of the first and second sides of the cross section.

In some embodiments, a distance between the fold points is greater than a width of the flat portion.

In some embodiments, a maximum width of the cross section is defined by a distance between the fold points.

In some embodiments, the first and second sides are curved outwardly, the lateral cross section being substantially oval or elliptical but with the first and second sides converging to a point at each fold point.

In some embodiments, the first side diverges outwardly either side of the flat portion towards the respective fold point.

In some embodiments, the conduit is a conduit portion of a patient interface.

In some embodiments, the patient interface is a nasal interface.

In some embodiments, the nasal interface is a nasal cannula.

In some embodiments, the collapsible portion is formed from an elastomeric/resilient material, for example silicone.

In accordance with at least one of the embodiments disclosed herein, a breathing conduit for providing a flow of respiratory gases, whether formed with a patient interface or as a separate conduit, comprises a collapsible portion, a lateral cross section of the collapsible portion comprising:
a first side for positioning against a user's face,
a second side opposite the first side to face away from the user's face,
the first and second sides joined by first and second fold points,
the conduit adapted to collapse from an open configuration to a closed configuration by folding at the fold points so that the first side and the second side are positioned in contact or adjacent each other to substantially occlude flow through the conduit when in the closed configuration.

In some embodiments, an inner length of the first side between the fold points and an inner length of the second side between the fold points are substantially equal.

In some embodiments, the lateral cross section has reflective symmetry about a line extending through the first and second fold points.

In some embodiments, the lateral cross section has reflective symmetry about a centre line of the cross section, the centre line extending through a centre of the first and second sides of the cross section.

In some embodiments, the second side is curved outwardly when in the open configuration.

In some embodiments, the first side is curved outwardly when in the open configuration.

In some embodiments, in the closed configuration the fold points are moved to be against or adjacent the face of a user.

In some embodiments, the thickness of the first side and/or the second side tapers towards each fold point, from a greater thickness to a reduced thickness, the maximum thickness being at an apex of each of the first side and second side respectively.

In some embodiments, the thickness of the fold points is less than the thickness of the remainder of the cross section of the collapsible portion.

In some embodiments, the second side is thinner than the first side.

In some embodiments, the ratio of:
i) the thickness of the (thicker) centre of the first and/or second sides of the lateral cross section and the thickness of the (thinner) fold points is in the range of about 1 to 8, or about 1.5 to 3.5, or
ii) the thickest part of the lateral cross section to the thinnest part of the lateral cross section being the fold points is in the range of about 1 to 8, or about 1.5 to 3.5.

In some embodiments, in the open configuration a line tangential to the portion of the first side adjacent to each folding point is an angle to a line extending between the first and second fold points such that an angle (beta) between the line and the portion adjacent the fold point is less than 70 degrees, or less than 65 degrees, or less than 60 degrees, or less than 55 degrees, or less than 50 degrees, or less than 45 degrees, or less than 40 degrees, or less than 35 degrees, or less than 30 degrees, or is about 30 to 60 degrees, or about 40 to 50 degrees, or may be about 45 degrees.

In some embodiments, the first and second fold points delimit or define the extent of the first and second sides, or the first and second sides each extend fully between the fold points, e.g. from the first fold point to the second fold point.

In some embodiments, a maximum width of the cross section is defined by a distance between the fold points.

In some embodiments, the first and second sides are curved outwardly, the lateral cross section being substantially oval or elliptical but with the first and second sides converging to a point at each fold point.

In some embodiments, the conduit is a conduit portion of a patient interface.

In some embodiments, the patient interface is a nasal interface.

In some embodiments, the nasal interface is a nasal cannula.

In some embodiments, the collapsible portion is formed from an elastomeric/resilient material, for example silicone.

In accordance with at least one of the embodiments disclosed herein, a breathing conduit for providing a flow of respiratory gases, whether formed with a patient interface or as a separate conduit, comprises a collapsible portion, a lateral cross section of the collapsible portion being substantially rhombus or parallelogram shaped, the four corners of the rhombus or parallelogram shaped cross section providing fold points, in an open configuration the four sides of the rhombus or parallelogram spaced apart, and in a closed configuration the cross section folding at the corners so that adjacent sides of the rhombus or parallelogram come together into contact and with the corners comprising acute internal angles located at edges of the cross section.

In some embodiments, the lateral cross section of the collapsible portion being substantially parallelogram shaped and adapted such that a long side of the parallelogram is located against a user's face in use.

In some embodiments, an acute angle of the rhombus or parallelogram is less than 70 degrees, or less than 65 degrees, or less than 60 degrees, or less than 55 degrees, or less than 50 degrees, or less than 45 degrees, or less than 40 degrees, or less than 35 degrees, or less than 30 degrees, or is between 45 and 65 degrees, or is between 55 and 65 degrees, or may be about 60 degrees.

In some embodiments, the thickness of the sides of the rhombus or parallelogram taper towards each corner (fold point) with an acute angle, from a greater thickness to a reduced thickness.

In some embodiments, the thickness of the corners (fold points) comprising an acute angle is less than the thickness of the sides or a remainder of the cross section of the collapsible portion.

In some embodiments, a side of the rhombus or parallelogram shaped cross section for locating against a user's face is thicker than other sides of the rhombus or parallelogram shaped cross section.

In some embodiments, the ratio of:
i) the relative thicknesses between the (thicker) sides of the lateral cross section and the (thinner) fold points is in the range of about 1 to 8, or about 1.5 to 3.5, or
ii) the thickest part of the lateral cross section to the thinnest part of the lateral cross section being the fold points is in the range of about 1 to 8, or about 1.5 to 3.5.

In some embodiments, the cross section comprises an internal notch at the corners comprising an acute angle so that the thickness at the corners comprising an acute angle is less than the thickness of the sides of the cross section.

In some embodiments, the sides of the rhombus or parallelogram have a thickness of about 0.5mm, and wherein the corners comprising an acute angle have a thickness of about 0.2mm.

In some embodiments, the cross section of the collapsible portion comprises a tail portion extending from one or both corners of the section comprising acute internal angles, each tail portion providing a ramp from the edge of the section onto a top of the section in the closed configuration.

In some embodiments, a side of the rhombus or parallelogram shaped cross section for locating against a user's face is thicker than other sides of the rhombus or parallelogram shaped cross section, and
the cross section comprises only one tail portion that extends from the corner of the cross section comprising an acute angle at the thicker side of the cross section.

In some embodiments, the thickness of the sides of the cross section taper to be thicker at at least one corner of the cross section comprising an obtuse angle.

In some embodiments, the thickness of the cross section provides for a tapering collapsed cross section that tapers in thickness from the edges of the cross section to a thicker section between the edges of the collapsed cross section.

In some embodiments, the cross section has reflective symmetry on a line extending through the corners comprising an obtuse angle.

In some embodiments, the conduit is a conduit portion of a patient interface.

In some embodiments, the patient interface is a nasal interface.

In some embodiments, the nasal interface is a nasal cannula.

In some embodiments, the collapsible portion is formed from an elastomeric/resilient material, for example silicone.

In accordance with at least one of the embodiments disclosed herein, a patient interface comprises a breathing conduit as described in any one or more of the above statements.

In some embodiments, the interface is a nasal interface comprising a single inlet, at least one nasal outlet, and the breathing conduit extending between the single inlet and the at least one nasal outlet.

In accordance with at least one of the embodiments disclosed herein, a connector adapted to connect a headgear to a patient interface comprises:
a first connector part (e.g. a male part) and a second connector part (e.g. a female part), the second connector part comprising a pair of spaced apart tines to receive the first part therebetween when the first and second parts are connected together.

In some embodiments, the tines each extend from a base of the second part, an end of each tine distal from the base free to deflect laterally relative to the base.

In some embodiments, one or both of the tines comprises an aperture or a lateral projection, and the first part comprises a corresponding lateral projection or aperture, such that with the first part received between the tines the lateral projection is received in the aperture to retain the first and second parts together.

In some embodiments, the aperture is a slot oriented with a major axis lateral to a longitudinal axis of a headgear strap to be attached to the patient interface.

In some embodiments, each tine comprises a said aperture and the first connector comprises a said lateral projection on each lateral side of the first part.

In some embodiments, the first and second parts are complementarily adapted to rotate relative to one another from a engaged position to disengage, the first and second parts comprising complementary features such that relative rotation between the first and second parts causes the tines to deflect and spread apart to release the second part from the first part.

In some embodiments, the aperture and projection are complementarily adapted so that relative rotation between the first and second parts causes the projection to release from the aperture and deflect a said tine over the projection.

In some embodiments, the lateral projection comprising a bevelled edge to deflect the tines apart when inserting the first part in between the tines of the second part in an axial direction of the connector parts.

In some embodiments, the second connector part is releasably coupled to a headgear.

In accordance with at least one of the embodiments disclosed herein, a connector adapted to connect a headgear to a patient interface comprises:
a first connector part (e.g. a male part) and a complementary second connector part (e.g. a female part) comprising a pair of spaced apart resilient tines to receive the first connector part therebetween, the second connector part adapted to be removably coupled to a headgear, the second connector part comprising a base, each tine extending from the base,
and an end of each tine distal from the base free to deflect laterally relative to the base so that the tines deflect laterally apart to release the first connector part from the second connector part.

In some embodiments, the first and second connector parts are adapted to be connected together by moving the second connector part axially towards the first connector part, and
the connector parts adapted to be disconnected by relative rotation about a lateral projection on one of the first and second parts received in an aperture on the other one of the first and second parts.

In accordance with at least one of the embodiments disclosed herein, a patient interface comprises:
a manifold and at least one nasal prong or an outlet extending from the manifold to be received by a user's nare or mouth,
a side member extending from each side of the manifold, each side member comprising a collapsible portion comprising a lumen, in an open configuration the lumen remaining open and the collapsible portion adapted to be pinched or flattened to a closed configuration (e.g. by an external force) to occlude or substantially occlude the lumen, and wherein at least one of the side members is a conduit for a flow of gases from an inlet of the patient interface to the manifold.

In some embodiments, the patient interface comprises a plug and a conduit connector, the plug adapted to fit to an end of one or both side members, and the conduit connector adapted to fit to an end of the other one or both side members.

In some embodiments, each side member is formed as a conduit, and the patient interface comprises a plug and a conduit connector, the plug and conduit connector both adapted to fit to an inlet end of both side members, so that the patient interface is configurable to a dual inlet patient interface or a left or right sided single patient interface.

In some embodiments, the patient interface comprises a wall near to and on an inlet side of a said nasal prong or outlet, to separate the lumen of one side member from the manifold and the other side member, such that only one of the side members acts as a conduit to provide a flow of gases from an inlet of the patient interface to the manifold.

In some embodiments, the lumen of the side member that is separate from the manifold comprises a relief hole so that the lumen of the side arm separate from the manifold is in communication with the atmosphere.

In some embodiments, the wall is curved or shaped to direct a flow from the manifold to the at least one nasal prong or outlet and/or to reduce resistance to flow.

In some embodiments, the side members, manifold and the at least one nasal prong or outlet are integrally formed as a unitary member.

In some embodiments, the side members are formed from a relatively soft or compliant material and the plug and/or conduit connector is formed from a relatively rigid or hard material.

In some embodiments, the patient interface comprises a removable shield to configure the patient interface for use without collapsing.

In some embodiments, the shield is adapted to fit over and cover a side member, or both side members and the manifold.

In some embodiments, the shield comprises one or more pair of jaws, each pair of jaws configured to grab around a portion of the patient interface to hold the shield to the patient interface.

In some embodiments, the patient interface is a nasal cannula comprising the manifold and at least one said nasal prong or a nasal outlet extending from the manifold to be received by a user's nare.

In some embodiments, a part of a headgear connector is integrally formed with each side member.

In some embodiments, when dependent on claim 13, wherein a part of a headgear connector is integrally formed with the conduit connector and/or a part of a headgear connector is integrally formed with the plug.

In some embodiments, the cannula comprises a pair of headgear connector parts (e.g. a pair of male parts or a pair of female parts), each part adapted to connect to a corresponding headgear connector part to attach a headgear to the cannula, and wherein each headgear connector part is arranged at an angle to the side members in a side view of the cannula, so that in use the cannula is positioned horizontally on the user's face and with a headgear extending above the user's ears.

In some embodiments, the angle is 10 to 30 degrees, or 15 to 25 degrees, or about 20 degrees.

In some embodiments, in plan view the cannula comprises an obtuse angle between the side members when in a neutral or unbent configuration.

In some embodiments, the obtuse angle in the range of 100 to 130 degrees, or about 100 to 120 degrees, or about 100 to 110 degrees, or about 105 degrees (e.g. 106 degrees).

In some embodiments, the side members are substantially straight in a neutral or unbent configuration, and the manifold is curved to provide the obtuse angle between the side members.

In some embodiments, the cannula comprises a pair of headgear connector parts (e.g. a pair of male parts or a pair of female parts), each part adapted to connect to a corresponding headgear connector part to attach a headgear to the cannula, and wherein each headgear connector part is arranged at an angle to the side members in a plan view of the cannula, wherein the angle is in the range of 130 degrees to 170 degrees, or 140 degrees to 160 degrees, or 145 degrees to 155 degrees.

In some embodiments, the cannula comprises a pair of headgear connector parts (e.g. a pair of male parts or a pair of female parts), each part adapted to connect to a corresponding headgear connector part to attach a headgear to the cannula, and wherein a distance between distal ends of the side arms, or between the pair of headgear connector parts is about 100mm to 150mm, or about 110mm to 140mm, or about 110mm to 130mm or about 120mm.

In accordance with at least one of the embodiments disclosed herein, a nasal cannula comprises:
a manifold and at least one nasal prong or outlet extending from the manifold to be received by a user's nare, and a side member extending from each side of the manifold, and wherein in plan view the cannula comprises an obtuse angle between the side members when in a neutral or unbent configuration.

In some embodiments, the obtuse angle in the range of 100 to 130 degrees, or about 100 to 120 degrees, or about 100 to 110 degrees, or about 105 degrees (e.g. 106 degrees).

In some embodiments, the side members are substantially straight in a neutral or unbent configuration, and the manifold is curved to provide the obtuse angle between the side members.

In some embodiments, the cannula comprises a pair of headgear connector parts (e.g. a pair of male parts or a pair of female parts), each part adapted to connect to a corresponding headgear connector part to attach a headgear to the cannula, and wherein each headgear connector part is arranged at an angle to the side members in a plan view of the cannula, wherein the angle is in the range of 130 degrees to 170 degrees, or 140 degrees to 160 degrees, or 145 degrees to 155 degrees.

In some embodiments, the cannula comprises a pair of headgear connector parts (e.g. a pair of male parts or a pair of female parts), each part adapted to connect to a corresponding headgear connector part to attach a headgear to the cannula, and wherein each headgear connector part is arranged at an angle to the side members in a side view of the cannula, so that in use the cannula is positioned horizontally on the user's face and with a headgear extending above the user's ears.

In some embodiments, the angle is 10 to 30degrees, or 15 to 25 degrees, or about 20degrees.

In some embodiments, the nasal cannula comprises a wall near to and on an inlet side of a said nasal prong or outlet, to separate the lumen of one side member from the manifold and the other side member, such that only one of the side members acts as a conduit to provide a flow of gases from an inlet of the cannula to the manifold.

In some embodiments, the lumen of the side member that is separate from the manifold comprises a relief hole so that the lumen of the side arm separate from the manifold is in communication with the atmosphere.

In accordance with at least one of the embodiments disclosed herein, a conduit for a respiratory support system comprises:
a collapsible portion, in an open configuration the collapsible portion remaining open and in a closed configuration the collapsible portion being pinched or flattened to occlude or substantially occlude the conduit, and
a relatively rigid component adapted to move from a first configuration in which the collapsible portion is in the open configuration to a second configuration in which the component presses against an outside of the collapsible portion to pinch or flatten the collapsible portion into the closed configuration.

In some embodiments, the component comprises a shield attached to the outside of the collapsible portion, the shield adapted to distribute an external force applied to the shield to a predetermined collapsible area of the collapsible portion.

In some embodiments, the component comprises a lever adapted to pivot from the first configuration to the second configuration.

In some embodiments, the conduit comprises the collapsible portion and a non-collapsible portion and the lever is pivotally attached to the non-collapsible potion of the conduit.

In some embodiments, the conduit comprises a vent aperture upstream of the collapsible portion and wherein the lever comprises a first arm extending from a first side of a pivot and a second arm extending from an opposite second side of the pivot, and in the first configuration the lever is pivoted about the pivot so that the first arm does not pinch or flatten the collapsible portion and the second arm substantially occludes the vent aperture, and in the second configuration the lever is pivoted about the pivot so that the first arm pinches or flattens the collapsible portion and the second arm lifts away from the vent aperture to allow gases in the conduit upstream of the collapsible portion to vent to atmosphere.

In some embodiments, the vent aperture is in the non-collapsible portion of the conduit.

In some embodiments, the lever comprises a projection or rim to press against the collapsible portion in the second configuration.

In accordance with at least one of the embodiments disclosed herein a patient interface comprises:
an interface portion for interfacing with a user's nasal or oral airway, and
a conduit as described in any one or more of the preceding statements, the conduit extending from the interface portion, and
the relatively rigid component attached to the conduit or interface portion to move from the first configuration to the second configuration.

In some embodiments, the component comprises a lever pivotally attached to the conduit or interface portion to pivot from the first configuration to the second configuration.

In some embodiments, the conduit comprises the collapsible portion and a non-collapsible portion and the lever is pivotally attached to the non-collapsible potion of the conduit, and wherein the collapsible portion is located between the interface portion and the non-collapsible portion of the conduit.

In some embodiments, the patient interface is a nasal cannula and the interface portion comprises a manifold and at least one nasal prong or outlet extending from the manifold.

In some embodiments, the patient interface is an oral interface to be received in a user's mouth.

In accordance with at least one of the embodiments disclosed herein a patient interface comprises:
a body comprising:
a manifold and at least one nasal prong or an outlet extending from the manifold, and
a left hand side member extending from a left side of the manifold and a right hand side member extending from a right side of the manifold, each side member comprising an inlet portion and a lumen to provide a conduit for a flow of gases from the inlet portion to the manifold, and
a frame comprising a tube connector and a blanked hollow projection, the tube connector and the blanked hollow projection adapted to receive a said inlet portion of the body with the frame attached to the body, the frame movably attached to the body to selectively configure the patient interface between a left hand inlet configuration and a right hand inlet configuration,
in the left hand inlet configuration the inlet portion of the left hand side member received in the tube connector and the inlet portion of the right hand side member received in the blanked hollow projection, and
in the right hand inlet configuration the inlet portion of the right hand side member received in the tube connector and the inlet portion of the left hand side member received in the blanked hollow projection.

In some embodiments, the conduit of each side member comprises a collapsible portion, in an open configuration the collapsible portion remaining open and in a closed configuration the collapsible portion being pinched or flattened to occlude or substantially occlude the conduit.

In some embodiments, the frame is adapted to deform so that a force applied to the front of the frame elastically bends the frame to collapse the conduit of a said member to the closed configuration.

In some embodiments, the frame is rotationally attached to the body rotate the frame relative to the body to selectively configure the cannula between the left hand inlet configuration and the right hand inlet configuration.

In some embodiments, the frame comprises a concave interior to receive a correspondingly shaped convex shape of the body.

In some embodiments, the patient interface is a nasal cannula, said body being a cannula body comprising the manifold and said at least one nasal prong or a nasal outlet extending from the manifold to be received by a user's nare.

In accordance with at least one of the embodiments disclosed herein a patient interface comprises a headgear, the headgear comprising a pair of arms, each arm comprising an ear plug, each ear plug adapted to fit within a user's ear to retain the patient interface in position on the user's face.

In some embodiments, one or both arms is length adjustable

In some embodiments, one or both arms is telescopic, one or both arms comprising a first portion slidingly received in a second portion, relative movement between the first and second arms adjusting the length of the arm.

In some embodiments, one of the first and second portions of each arm is integrally formed with a frame attached to a body of the patient interface.

In some embodiments, one of the first and second portions of the arm is more rigid than the other one of the first and second portions of the arm.

In some embodiments, the patient interface is a nasal cannula.

In another aspect, this disclosure relates to a breathing conduit for providing a flow of respiratory gases, e.g. whether formed with a patient interface or as a separate conduit, the conduit comprising:
a collapsible portion being collapsible by an external force applied to the conduit; and
a flexible insert provided to an inside of the collapsible portion, where in a collapsed configuration the insert substantially blocks a flow path of the conduit through the collapsible portion.

In one form the insert is an annular member and/or the annular member is arranged with a central axis lateral to a longitudinal axis of the conduit. In some embodiments the annular member is arranged with a central axis aligned with a longitudinal axis of the conduit, the annular member located around an inner surface of the conduit.

In some embodiments the insert is circular. In some embodiments the insert is an o-ring. In some embodiments the insert has a lateral dimension greater than a lateral inner dimension of the conduit, the insert elastically deflected to fit within the conduit. In some embodiments the insert has a height less than a height of the inside of the collapsible portion to allow a flow of gases through the conduit and around the insert when the collapsible portion is in an open configuration.

In some embodiments the insert is biased between opposite sides of the inside of the collapsible portion, the opposite sides forming folded edges of the collapsible portion when in the collapsed configuration, the insert adapted to expand between the opposite sides of the collapsible portion as the collapsible portion is collapsed from an open configuration to the collapsed configuration.

In some embodiments the insert contacts between opposite sides of the inside of the collapsible portion. In some embodiments the insert has rounded end profile located at the sides of the inside of the collapsible portion, in the collapsed configuration the collapsible portion wrapping around the end profile of the insert.

In some embodiments the insert is adapted to fill gaps adjacent folded edges of the collapsible portion formed when the collapsible portion is in the collapsed configuration. In some embodiments the insert comprises a gel material and/or is a fluid filled member.

In some embodiments the breathing conduit is a conduit portion of a patient interface. The patient interface may be a nasal interface, e.g. a nasal cannula.

In some embodiments the patient interface may comprise a breathing conduit according to any one of the preceding embodiments.

In some embodiments the patient interface may comprise:
a manifold and at least one nasal prong or an outlet extending from the manifold to be received by a user's nare or mouth;
a gases delivery side member extending from a side of the manifold;
a non-gases delivery side member extending from another side of the manifold;
wherein at least a part of the non-gases delivery side member comprises an outer wall for, in use, contact with a mask, and an inner wall for, in use, contact with a user's face, where a cross-sectional profile of the at least a part of the non-gases delivery side member comprises the inner wall having a more pronounced outward curve than the outer wall.

In some embodiments the cross-sectional profile of the at least part of the non-air delivery side is an elongated asymmetric lens shape, e.g. the inner wall has a curved profile with a central, flat region and/or the outer wall has a curved profile with a central, flat region. In some embodiments the outer wall has a flat profile relative to the outward curve of the inner wall.

In some embodiments, relative to a plane taken through meeting edges of the inner and outer walls, the outward curve of the inner wall extends from 1 to 3mm and the outward curve of the outer wall extends 0 to 0.9mm.

In some embodiments edges of the inner and outer walls meet at a point, a taper or a rounded area.

In some embodiments the gases delivery side member is or forms part of a conduit for a flow of gases from an inlet of the patient interface to the manifold. In an example the gases delivery side member comprises a collapsible portion adapted to be pinched or flattened to a closed configuration to occlude or substantially occlude the conduit.

In some embodiments a cross -sectional profile of the collapsible portion includes a thinned region. In some embodiments the gases delivery side member has an obround shaped cross-sectional profile.

In some embodiments the cross-sectional profile has a_constant wall thickness over a substantive length, excluding the collapsible portion. For example, opposing walls of the collapsible portion that, in use, contact the user's face and a mask respectively, progressively narrow until they meet at an offset region having the thinnest wall thickness of the cross sectional profile. For example, opposing walls of the collapsible portion that, in use, contact the user's face and a mask respectively, progressively narrow until they meet at a thinnest wall thickness region of the cross sectional profile.

In some embodiments the opposing walls have a substantive portion with constant thickness and the thinnest wall thickness region promotes collapsing.

In some embodiments the collapsible portion extends 3 to 10mm, preferably about 5 mm, or about 10mm, along a portion of the length of the gases or air-delivering side.

In some embodiments an elbow in a region of the air or gases delivery side member proximate where it is attachable to an inspiratory tube, e.g. a cross-sectional wall thickness of the elbow is increased or varied compared to a wall thickness of the air delivery side member. The wall thickness of the elbow may be in the range of about 0.2mm to about 1mm, or about 0.05mm to 2mm.

In some embodiments both the non-air delivery side member and air or gases delivery side member are contoured for placement across a user's upper lip.

In some embodiments an outward facing surface of either or both of the non-air delivery side member and air or gases delivery side member is roughened.

In some embodiments an internal wall of the collapsible portion has some stickiness for providing an amount of hysteresis which contributes to holding it (being the internal wall of the collapsible portion) together when collapsed.

In some embodiments there is a retention mechanism for support upon a user's face, e.g. wherein the retention mechanism comprises one or more of a headband, an elastic strap and/or one or more adhesive pads

In a further form there may be a patient interface comprising:
a manifold and at least one nasal prong or an outlet extending from the manifold to be received by a user's nare or mouth;
a gases delivery side member extending from a side of the manifold;
wherein the gases delivery side member comprises:
   a lumen for a flow of gases from an inlet of the patient interface to the manifold;
   a collapsible portion, with a cross -sectional profile having a thinned region, adapted to be pinched or flattened to a closed configuration to occlude or substantially occlude the lumen.

In some embodiments a cross-sectional profile of a substantive length of the gases delivery side member, excluding the collapsible portion, has a constant wall thickness. In some embodiments the cross-sectional profile(s) have an obround shape.

In some embodiments opposing walls of the collapsible portion that, in use, contact the user's face and a mask respectively, progressively narrow until they meet at the thinned region, the thinned region being an offset region having the thinnest wall thickness of the cross-sectional profile.

In some embodiments opposing walls of the collapsible portion that, in use, contact the user's face and a mask respectively, progressively narrow until they meet at the thinned region, the thinned region being a thinnest wall thickness region of the cross sectional profile.

In some embodiments the opposing walls have a substantive portion with constant thickness and the thinned region promotes collapsing.

In some embodiments the collapsible portion extends 3mm to 10mm, preferably about 5 mm, along a portion of the length of the air-delivering side.

In some embodiments the air or gases delivery side member further comprises an elbow in a region proximate where it (being said elbow) is attachable to an inspiratory tube.

In some embodiments a cross-sectional wall thickness of the elbow is increased or varied compared to a wall thickness of the air or gases delivery side member. The wall thickness of the elbow may be in the range of about 0.2mm to about 1mm, or about 0.05mm to about 2mm.

In some embodiments there is a second side member extending from an opposite side of the manifold, wherein the second side member is either a non-gases delivery side member or another gases delivery side member, e.g. the second side member has or comprises a collapsible portion.

In some embodiments both the air or gases delivery side member and second side member are contoured for placement across a user's upper lip.

In some embodiments an outward facing surface of the air or gases delivery side member is roughened.

In some embodiments the air or gases delivering side member is the only member extending from the manifold and further comprises an adhesive side for securing on user.

In some embodiments the manifold comprises reversible nasal prongs.

In some embodiments the internal surfaces of opposing walls of the collapsible portion provide an amount of hysteresis which contributes to holding it together when collapsed.

In some embodiments there is a retention mechanism for support upon a user's face, e.g. the retention mechanism comprises one or more of a headband, an elastic strap and/or one or more adhesive pads

In a further form there may be a patient interface comprising:
a manifold and at least one nasal prong or an outlet extending from the manifold to be received by a user's nare or mouth;
at least one non-gases delivery side member extending from a side of the manifold;
wherein at least a part of the non-gases delivery side member comprises an outer wall for, in use, contact with a mask, and an inner wall for, in use, contact with a user's face, where a cross-sectional profile of the at least a part of the non-gases delivery side member comprises the inner wall having a more pronounced outward curve than the outer wall.

In accordance with at least one of the embodiments disclosed herein, a patient interface comprising:
at least one nasal prong or an outlet of said patient interface to be received by a user's nare(s) or mouth;
a gases delivery side member extending from a side of the at least one nasal prong or said outlet;
wherein the gases delivery side member comprises:
   a lumen for a flow of gases from an inlet of the patient interface to the at least one nasal prong or said outlet;
   a collapsible portion;
   at least one elbow portion or flexible portion, located substantially at or toward one or both of a downstream end of said gases delivery member or an upstream end of said gases delivery member.

In some embodiments, the collapsible portion allows for a leakage gases flow through the collapsed portion of the conduit when in a collapsed condition, optionally said leakage gases flow is about 15L/min or less; or about 10 L/min or less, or about 10L/min, or about 5L/min to about 10L/min, optionally the leakage gases flow may be measured by a sensor.

In some embodiments, the patient interface comprises a base portion to support the at least one nasal prong, the base portion being substantially the same, or near the same width as a base of the nasal prongs, such that in use the base of the nasal prongs is located on or near a patient's face, optionally to provide for a low profile cannula in a region under a patients nose, optionally the base portion comprises a hollowed section located on the rear side of the base portion.

In some embodiments, wherein the base portion is a manifold, the manifold configured to provide a flow of gases to the at least one nasal prong or outlet from the air delivery side member.

In some embodiments, the manifold comprises a ramp portion, the ramp portion configured to direct said flow of gases to a nasal prong or a pair of nasal prongs.

In some embodiments, the ramp portion is positioned to protrude into said flow of gases and to direct said flow of gases to a nasal prong or a pair of nasal prongs.

In some embodiments, the ramp portion is positioned along a base of said manifold.

In some embodiments, the ramp portion provides for a graduated wall region, the wall region extending substantially towards or upwardly to said nasal prongs or to a more downstream nasal prong of a pair of nasal prongs.
In some embodiments, the ramp portion extends from a substantially upstream location, with respect to the direction of said flow of gases, towards a substantially downstream location, with respect to the direction of said flow of gases.

In some embodiments, the ramp portion extends from a substantially upstream location, with respect to the direction of said flow of gases, said ramp graduated so as to be inclining from said upstream location.

In some embodiments, the ramp portion initiates a ramp incline from a position upstream of an upstream nasal prong of a pair of nasal prongs.

In some embodiments, the ramp portion initiates a ramp incline from a position at a position substantially aligned with an upstream nasal prong of a pair of nasal prongs.

In some embodiments, the ramp portion initiates a ramp incline from a position substantially aligned with a nasal prong, said alignment is with respect to a mid-line of a base of said nasal prong.

In some embodiments, the ramp portion initiates a ramp incline from a position downstream of an upstream nasal prong of a pair of nasal prongs.

In some embodiments, the ramp portion initiates a ramp incline from a position downstream of an upstream nasal prong of a pair of nasal prongs, and upstream of a downstream nasal prong.

In some embodiments, the ramp portion terminates at a base of a downstream nasal prong of a pair of nasal prongs.

In some embodiments, the ramp portion terminates at a most downstream end point of a downstream nasal prong of a pair of nasal prongs.

In some embodiments, a prong outlet is s-shaped or elongate.

In some embodiments the prong outlet has edges of a gases outlet cut-out, or is a shaped cut-out to confirm to a surface that has a substantially reverse S-shape, the S-shape being aligned substantially vertically.

In some embodiments the prong outlet is substantially elliptical or elongated in a substantially vertical direction, or a lower edge of the surface cuts extend across the rear of the prongs to create the cut-out, the surface being a reverse S-shape to obtain the cut-out shape.

In some embodiments, a lower portion of a front face of the base portion is rounded or bevelled.

In some embodiments, the patient interface comprises a dipped portion (i.e. being a substantially downwardly dipped portion) between a pair of nasal prongs.

In some embodiments, the dipped portion is at least partially protruding into a flow of gases directed toward said nasal prongs.

In some embodiments, the dipped portion is configured to direct at least a portion of a flow of gases to an upstream nasal prong of the pair of nasal prongs.

In some embodiments, the air or gases delivery side member, and/or at least one elbow is connected to, or connectable to an inspiratory tube connector (optionally the inspiratory connector comprises a barb or a barb-shaped portion for connection with the air delivery side member and/or at least one elbow).

In some embodiments, the gases delivery side member comprises an inwardly extending portion to retain the barb or barb shaped portion when connected.

In some embodiments, the gases delivery side member comprises a thickened sidewall to retain the barb or barb shaped portion when connected.

In some embodiments, the gases delivery side member comprises a thickened sidewall in a region between an end of the delivery side member and the inwardly extending portion to retain the bard or bard shaped portion when connected.

In some embodiments, the inwardly extending portion is a relatively thinned or thinner sidewall portion.

In some embodiments, when said inwardly extending portion is subjected to an elongation force, said inwardly extending portion substantially stretches or deforms substantially radially inwardly, thereby increasing a retention force upon said barb.

In some embodiments, the inspiratory tube connector is rigid.

In some embodiments, the inspiratory tube comprises a barbed or barb-shaped portion, and/or a threaded connection, for connection with an inspiratory conduit.

In some embodiments, the inspiratory tube connector comprises a connection feature for connection of the inspiratory tube connector to a head strap and/or an associated headgear, optionally the connection feature is one or a pair, or a plurality of slots.

In some embodiments, one or more of the slots is a substantially t-shaped slot, a substantially T-shaped slot, a substantially U-shaped slot, or a substantially L-shaped slot.

In some embodiments, one or more of the slots is located at a headgear strap connection end of the inspiratory tube connector is an open T-shaped slot.

In some embodiments, one or more of the slots located at a headgear strap connection end of the inspiratory tube connector is a closed T-shaped slot, enclosed within said headgear strap connection end of the inspiratory tube connector.

In some embodiments, one or more of the slots are narrowed in relation to other of the slots.

In some embodiments, the narrowed slot provides for a narrowed opening for receiving a headgear strap.

In some embodiments, one or more of the slots are wider in relation to other of the slots.

In some embodiments, the wider slot provides for a wider opening for receiving a headgear strap.

In some embodiments, the at least one elbow portion, or flexible portion comprises a first elbow or a first flexible portion, the first elbow or first flexible portion is located at an end of the gases delivery side member, and/or collapsible portion near or adjacent an inspiratory tube or the or an inspiratory tube connector, optionally the first elbow or first flexible portion is located between the gases delivery side member and/or the collapsible portion, and an inspiratory tube or the or an inspiratory tube connector.

In some embodiments, further comprising an intermediate section located between the first elbow or the first flexible portion and an inspiratory tube or an inspiratory tube connector.

In some embodiments, the intermediate section is located at an angle of about -25 degrees to about 45 degrees from a centreline of the patient interface, optionally, the intermediate section is located at an angle of about 10 degrees from a centreline of the patient interface.

In some embodiments, the intermediate section is located at an angle of about 0 degrees to about 90 degrees from a front face, or a rear face, or a longitudinal axis of the collapsible portion and/or the gases delivery side member, optionally the intermediate section is located at an angle of about 20 degrees to about 60 degrees from a front face, or a rear face, or a longitudinal axis of the collapsible portion and/or the gases delivery side member, optionally the intermediate section is located at an angle of about 25 degrees to about 35 degrees from a front face, or a rear face, or a longitudinal axis of the collapsible portion and/or the gases delivery side member.

In some embodiments, the intermediate section is located at an angle of about 0 degrees to about 30 degrees from a lower face, or an upper face, or a longitudinal axis of the gases delivery side member and/or the collapsible portion, optionally the intermediate section is located at an angle of about 5 degrees to about 25 degrees from a lower face, or an upper face, or a longitudinal axis of the gases delivery side member and/or the collapsible portion, optionally the intermediate section is located at an angle of about 15 degrees from a lower face, or an upper face, or a longitudinal axis of the gases delivery side member and/or the collapsible portion, optionally the angle between the intermediate section and the lower face, or an upper face, or a longitudinal axis of the gases delivery side member and/or the collapsible portion is an angle of elevation.

In some embodiments, the intermediate section provides for a flow path from the inspiratory tube or inspiratory tube connector to the gases delivery side member and/or the collapsible portion.

In some embodiments, the intermediate section is about 0mm to about 30mm in length, or about 12mm to about 25mm in length.

In some embodiments, the first elbow or first flexible portion provides for or comprises a pivot portion and/or a hinging portion, optionally to allow for relative movement about at least one axis (for example axis A1, or axis A2, or axis A3).

In some embodiments, said at least one axis comprises a first axis (for example axis A1), the first axis being oriented substantially parallel with, or along a height of the gases delivery side member and/or the collapsible portion, optionally said first axis being oriented substantially parallel to a patient's face in use, optionally said first axis being oriented substantially parallel to a portion of the gases delivery side member and/or the collapsible portion configured to contact a user's face, optionally said first axis being oriented substantially parallel to a front face and/or a rear face of the gases delivery side member and/or the collapsible portion, optionally, said first axis being located through (optionally so as to bisect) said the first elbow or first flexible portion.

In some embodiments, said at least one axis comprises a second axis (for example axis A2 or axis A3), the second axis being oriented parallel with, or along a length of the gases delivery side member and/or the collapsible portion and/or an intermediate portion.

In some embodiments, a cross-sectional wall thickness of the elbow is increased or varied compared to, or relative to, a wall thickness of the air or gases delivery side member, optionally the wall thickness may be a thickness of about 0.2mm to about 1mm, or about 0.05mm to about 2mm.

In some embodiments, the gases delivery side member and/or the collapsible portion is a substantially straight section.

In some embodiments, the gases delivery side member and/or the collapsible portion is of a substantially constant cross-section.

In some embodiments, the gases delivery side member is about 50mm to about 100mm in length, or about 20mm to about 70mm in length, or about 25mm to about 35mm in length.

In some embodiments, the collapsible portion is about 3mm to about 10mm, or about 5 mm, or about 10mm in length.

In some embodiments, the collapsible portion is located substantially in a central portion of the gases delivery side member.

In some embodiments, a cross-sectional profile of a substantive length of the gases delivery side member, excluding the collapsible portion, has a constant wall thickness.

In some embodiments, the cross-sectional profile(s) is an obround shape.

In some embodiments, opposing walls of the collapsible portion that, in use, contact the user's face and a mask respectively, progressively narrow or reduce to meet at, or until said opposing walls meet at, a thinned region, the thinned region being an offset region having the thinnest wall thickness of the cross-sectional profile.

In some embodiments, opposing walls of the collapsible portion that, in use, contact the user's face and a mask respectively, progressively narrow or reduce to meet at, or until said opposing walls meet at, a thinned region, the thinned region being a thinnest wall thickness region of the cross-sectional profile.

In some embodiments, the opposing walls meet at an angle to each other in the thinned region on an inner surface of the collapsible portion, optionally said wall thickness at the location where the opposing walls meet is about 0.1mm, or about 0.05mm to about 2mm.

In some embodiments, the external surface of the collapsible portion is substantially round or rounded or is a continuous surface in the area of the thinned region.

In some embodiments, the or opposing walls have a substantive portion with constant thickness and the thinned region promotes collapsing.

In some embodiments, the thickness of the opposing walls is about 1mm, optionally the wall thickness of the opposing walls is about 0.2mm to about 1mm, or about 0.05mm to about 2mm.

In some embodiments, a cross-sectional profile of the collapsible portion comprises at least one substantially thinned region.

In some embodiments, a or the thinned region is located at one or both of an upper portion or a lower portion of the collapsible portion, optionally the thinned region may be provided at alternative or substantially opposing locations about an internal perimeter or inner surface of said collapsible portion.

In some embodiments, an inner surface of the collapsible portion comprises an angled section in the thinned region.

In some embodiments, inner surfaces of the collapsible portion converge to a or said thinned region.

In some embodiments, inner surfaces of the collapsible portion meet at a or said thinned region to provide for a pre-determined bending or hinging point.

In some embodiments, the external surface of the collapsible portion is substantially round or rounded or is a substantially continuous surface in the area of a or the thinned region.

In some embodiments, a or the wall thickness of the collapsible portion in the thinned region is about 0.1mm, or about 0.05mm to about 2mm.

In some embodiments, the collapsible portion extends along a portion of the length of the air or gases-delivering side.

In some embodiments, the gases delivery side has a gases delivery side member wall thickness of about 0.2mm to about 1mm, or about 0.05mm to about 2mm, or about 1mm.

In some embodiments, the gases delivery side member transitions from a gases delivery side member wall thickness that is substantially greater than said the wall thickness of the or a thinned region of said collapsible portion.

In some embodiments, the gases delivery side member wall thickness remains of a constant wall thickness or is of a substantially greater wall thickness than the wall thickness of the gases delivery side member at the or a thinned region of the collapsible portion.

In some embodiments, the wall thickness of the gases delivery side member along a length of said lumen remains substantially constant or is of a substantially greater wall thickness than the or a wall thickness of the gases delivery side member at the or a thinned region of the collapsible portion.

In some embodiments, the or a wall thickness of the gases delivery side member varies along a length of the lumen, the wall thickness reducing or being reduced or substantially less in the region of the thinned region relative to the wall thickness of the gases delivery side member along the length of the lumen.

In some embodiments, the wall thickness of gases delivery side member outside of the or a thinned region transitions via a step or a taper to the wall thickness at the thinned region.

In some embodiments, at least one elbow portion, or flexible portion comprises a second elbow, or second flexible portion, the second elbow, or second flexible portion located substantially in a region of the gases delivery side member proximate where said second flexible portion is attachable to the at least one nasal prong or outlet, and/or said base portion.

In some embodiments, the second elbow or second flexible portion provides for, or comprises, a pivot portion and/or a hinging portion, optionally to allow for relative movement about at least one axis (for example axis A4, or axis A2, or axis A6).

In some embodiments, said at least one axis comprises a first axis (for example A4), the first axis being oriented parallel with, or along a height of the gases delivery side member and/or the collapsible portion, optionally said first axis being oriented substantially parallel to a patient's face in use, optionally said first axis being oriented substantially parallel to a portion of the gases delivery side member and/or the collapsible portion configured to contact a user's face, optionally said first axis being oriented substantially parallel to a front face and/or a rear face of the gases delivery side member and/or the collapsible portion, optionally, said first axis being located through (optionally so as to bisect) said the second elbow or second flexible portion.

In some embodiments, said at least one axis comprises a second axis (for example axis A2 or axis A6), the second axis being oriented parallel with, or along a length of the gases delivery side member and/or the collapsible portion and/or the base portion.

In some embodiments, the gases delivery side member and/or the collapsible conduit is located about 30 degrees to about 80 degrees from a centreline of the patient interface, optionally the gases delivery side member and/or the collapsible conduit is located about 55 to about 60 degrees from a centreline of the patient interface.

In some embodiments, the gases delivery side member and/or the collapsible conduit is located about 10 degrees to about 60 degrees from a centreline of the base portion, optionally the gases delivery side member and/or the collapsible conduit is located about 30 degrees to about 35 degrees from a centreline of the base portion.

In some embodiments, the gases delivery side member and/or the collapsible portion is relatively stiffer or less flexible or more resilient to a flex, along a length of the gases delivery side member and/or the collapsible portion than along a length of the at least one elbow or flexible portion (optionally one or more of the first elbow, or first flexible portion and/or the second elbow or second flexible portion).

In some embodiments, the gases delivery side member and/or the collapsible portion is relatively stiffer or less flexible or more resilient to a flex, along a height of the gases delivery side member and/or the collapsible portion than along a height of the at least one elbow or flexible portion (optionally one or more of the first elbow, or first flexible portion and/or the second elbow or second flexible portion).

In some embodiments, the gases delivery side member and/or the collapsible portion is relatively stiffer or less flexible or more resilient to a flex, than the at least one elbow or flexible portion about at least one axis.

In some embodiments, the gases delivery side member and/or the collapsible portion is relatively stiffer or less flexible or more resilient to a flex, than one or more of the first elbow, or first flexible portion (optionally one or more of the first elbow, or first flexible portion and/or the second elbow or second flexible portion) about at least one axis.

In some embodiments, the gases delivery side member and/or the collapsible portion is relatively stiffer or less flexible or more resilient to a flex, than the at least one elbow or flexible portion about a gases delivery side member and/or the collapsible portion axis (for example axis A5),
optionally, said gases delivery side member and/or the collapsible portion axis being oriented parallel with, or along a height of the gases delivery side member and/or the collapsible portion,
optionally said gases delivery side member and/or the collapsible portion axis being oriented substantially parallel to a portion of the gases delivery side member and/or the collapsible portion configured to contact a user's face,
optionally said gases delivery side member and/or the collapsible portion axis being oriented substantially parallel to a front face and/or a rear face of the gases delivery side member and/or the collapsible portion,
optionally, the gases delivery side member and/or the collapsible portion axis being located through (optionally so as to bisect) the gases delivery side member and/or the collapsible portion,
optionally, the gases delivery side member and/or the collapsible portion axis being located through a centre of the gases delivery side member and/or the collapsible portion.

In some embodiments, said at least one axis comprises a first axis (for example A1), the first axis being oriented parallel with, or along a height of the gases delivery side member and/or the collapsible portion,
optionally said first axis being oriented substantially parallel to a patient's face in use,
optionally said first axis being oriented substantially parallel to a portion of the gases delivery side member and/or the collapsible portion configured to contact a user's face,
optionally said first axis being oriented substantially parallel to a front face and/or a rear face of the gases delivery side member and/or the collapsible portion,
optionally, said first axis being located through (optionally so as to bisect) said the first elbow or first flexible portion.

In some embodiments, said at least one axis comprises a second axis (for example axis A2 and/or axis A3), the second axis being oriented parallel with, or along a length of the gases delivery side member and/or the collapsible portion and/or an intermediate portion.

In some embodiments, the gases delivery side member and/or the collapsible portion is relatively stiffer or less flexible or more resilient to a flex, than one or more of the second elbow, or second flexible portion about at least one axis

In some embodiments, said at least one axis comprises a first axis (for example A4), the first axis being oriented parallel with, or along a height of the gases delivery side member and/or the collapsible portion,
optionally said first axis being oriented substantially parallel to a patient's face in use,
optionally said first axis being oriented substantially parallel to a portion of the gases delivery side member and/or the collapsible portion configured to contact a user's face,
optionally said first axis being oriented substantially parallel to a front face and/or a rear face of the gases delivery side member and/or the collapsible portion, optionally, said first axis being located through (optionally so as to bisect) said the second elbow or second flexible portion.

In some embodiments, said at least one axis comprises a second axis (for example axis A2 and/or axis A6), the second axis being oriented parallel with, or along a length of the gases delivery side member and/or the collapsible portion and/or the base portion.

In some embodiments, the at least one elbow (optionally one or more of the first elbow, or first flexible portion and/or the second elbow or second flexible portion) are configured to substantially deform before the gases delivery side member and/or the collapsible portion when a force is applied to the or an end (whether an upstream end or a downstream end or both of these ends) of the air delivery arm.

In some embodiments, the at least one elbow (optionally one or more of the first elbow, or first flexible portion and/or the second elbow or second flexible portion) is/are configured to isolate or attenuate the gases delivery side member and/or the collapsible portion from forces applied (for example force applied the non-air delivery arm, of the gases delivery side member) to the patient interface,
optionally to maintain the profile of the collapsible portion,
optionally to prevent the collapsible conduit from kinking.

In some embodiments, the patient interface further comprises a non-air delivering side member extending from a second side of at least one nasal prong or outlet, said gases delivery side member and non-air delivering side member are at different angles with respect to the at least one nasal prong or outlet.

In some embodiments, the patient interface further comprises a or the non-air delivering side member, and wherein the non-air delivering side member a connection feature for connection of the non-air delivering side member to a head strap and/or an associated headgear, optionally the connection feature is one or a pair, or a plurality of slots.

In a further form there may be a system for providing a flow of respiratory gases to a user, the system comprising:
a gases flow source;
a patient interface according to any one of the preceding embodiments;
a conduit providing gases flow between the flow source and the patient interface.

In some embodiments there may be a flow-compensated pressure relief valve upstream of the patient interface, e.g. wherein the resistance to flow of the flow-compensated pressure relief valve is altered to a predetermined value for a specific patient interface.

In some embodiments an air or gases delivery side member of the patient interface incorporates a predetermined restriction in the gases flow, e.g. the predetermined restriction of an adult patient interface is determined to match the comparative flow restriction of a child patient interface.

In some embodiments the flow-compensated pressure relief valve provides a hysteresis effect of flow venting with respect to force applied to the collapsible portion.

In some embodiments the force required to collapse or block the collapsible portion is greater than the force required to unblock the collapsible portion.

In some embodiments the patient interface is a cannula.

In a further form there may be a headstrap connector, the strap connector comprising of a connector body, a terminal end of the connector body configured to receive a strap of a headgear, wherein said connector body comprises of at least two strap receiving slot portions, a first slot portion located substantially adjacent to said terminal end and provided with an opening or access to said first slot through or from said terminal end, and a second slot portion is located further away from said terminal end than said first slot portion, and wherein said second slot portion is configured to extend substantially transversely across a width of said connector body.

In some embodiments, the second slot portion is a single slot provided as a substantially rectangular opening, wherein a long side of said rectangular opening extends substantially in a direction being aligned with said transverse width of said connector body, and wherein a short side of said rectangular opening extends substantially in a direction being aligned with a longitudinal length of said connector body.

In some embodiments, the first slot portion is a single slot provided as a substantially rectangular opening, excluding said opening or access to said first slot portion through or from said terminal end of said connector body.

In some embodiments, the second slot portion has a short side dimension that is substantially less than a short side dimension of said first slot portion.

In some embodiments, the second slot portion has a short side dimension that is substantially greater than a short side dimension of said first slot portion.

In some embodiments, the first slot portion has a short side dimension that is substantially less than a short side dimension of said second slot portion.

In some embodiments, the first slot portion has a short side dimension that is substantially greater than a short side dimension of said second slot portion.

In some embodiments, the second slot is enclosed or bound within said connector body.

In some embodiments, an edge or edges or edge portion(s) of at least one of said first or second slot portions comprises of a tapered or chamfered edge profile.

In some embodiments, an edge portion of at least one of said first and/or second slot portions comprises one or more retention features configured to, in-use, retain or grip a strap.

In some embodiments, the one or more retention features comprise one or more of: teeth features, a jagged profile.

In some embodiments, the connector body further comprises a pneumatic connection portion at an end of said connector body being substantially proximal or longitudinally displaced away from said terminal end.

In some embodiments, the connector body comprise of a first face and a second face, in-use, the first face of said connector body to be located substantially adjacent to a patient's face, and said second face of said connector body to comprises of said pneumatic connection portion to displace said pneumatic connection portion from said patient's face.

In some embodiments, the pneumatic connection portion comprises a connector portion to connect to a first end of a breathing circuit, said connector portion optionally being of a threaded connection.

In some embodiments, a second end of the pneumatic connector is configured to connect to a patient interface.

In some embodiments, the second end of the pneumatic connector comprises a barb to engage a recess in a wall of a pneumatic connector connecting end portion of the patient interface.

In some embodiments, the pneumatic connector connecting end portion of the patient interface is substantially elastically deformable to receive said pneumatic connector.

In some embodiments, the second end of the pneumatic connector and/or the pneumatic connector connecting end portion of the patient interface comprises of an alignment feature.

In some embodiments, the second end of the pneumatic connector and/or the pneumatic connector connecting end portion of the patient interface comprises of an alignment feature.

In some embodiments, the alignment feature comprises of complementary male and female receiving portions configured to mate with each other upon alignment of said pneumatic connector with said pneumatic connector connecting end portion of the patient interface.

In some embodiments, the male and female receiving portions are a notch and a projection, or a keyway and a key, configured for corresponding mating to ensure connection between said pneumatic connector and said pneumatic connector connecting end portion of the patient interface.

In some embodiments, the second end of the pneumatic connector comprises a rigid connector.

In a further form there may be a headstrap connector, the strap connector comprising of a connector body, a terminal end of the connector body configured to receive a strap of a headgear, wherein said connector body comprises of at least two strap receiving slot portions, a first slot portion located substantially adjacent to said terminal end and provided with an opening or access to said first slot through or from said terminal end, and a second slot portion is located further away from said terminal end than said first slot portion, and wherein said second slot portion is configured as an intersection of at least two substantially opposingly arranged slots through said connector body.

In some embodiments, the second slot portion comprises of at least two slot portions arranged substantially transversely or arranged to intersect with each other so as to form said second slot.

In some embodiments, each of said first slot portion and said second slot portion are of:
- substantially the same width with respect to the connector body, and/or
- substantially the same longitudinal length with respect to the connector body.

In some embodiments, each of said first slot portion and said second slot portion are of:
- substantially different widths with respect to each other, and/or
- substantially different longitudinal length with respect to each other.

In some embodiments, the second slot portion is formed as at least one of the following: a substantially t-shaped, a substantially T-shaped, a substantially U-shaped, or a substantially L-shaped slot.

In a further form there may be a system for providing gases to a user comprising:
a gases flow source,
the patient interface of any of the embodiments above, and
a conduit connection between the gases flow source and the patient interface.

In a further form there may be a patient interface, comprising
a manifold,
a gases delivery side arm, said gases delivery side arm integrally formed with and extending from a first side of the manifold,
at least one or a pair of nasal prongs, and
a headstrap connector of any of the embodiments above.

In some embodiments, the gases delivery side arm comprises a collapsible portion.

In some embodiments, the gases delivery side arm further comprises a flexible portion or an elbow.

In some embodiments, the flexible portion or elbow is positioned at an upstream end of the gases delivery side arm.

In some embodiments, a non-gases delivery side arm extends from a second side of the manifold.

In some embodiments, a headstrap is configured to connect the headstrap connector, the gases delivery side arm, and the non-gases delivery side arm.

In a further form there may be a patient interface comprising:
a manifold and at least one nasal prong or an outlet extending from the manifold to be received by a user's nare or mouth;
a patient conduit configured to supply a flow of gases to the at least one nasal prong;
an engagement portion, the engagement portion configured to engage the patient conduit, optionally a surface or portion of the patient interface, the engagement portion being connected to the patient interface by a bridging section.

In some embodiments, the patient interface further comprises:
a gases delivery side member extending from a side of the manifold;
a non-gases delivery side member extending from another side of the manifold;
wherein the patient conduit is connected to an end of the gases delivery side member.

In some embodiments, the engagement portion is configured to engage with a surface of the patient conduit, at a location away, or distal from, the end of the gases delivery side member and/or where the patient conduit is connected to the gases delivery side member or the patient interface.

In some embodiments, the bridging portion provides for, or comprises, a pivoting portion and/or a hinging portion, optionally to allow for relative movement about at least one axis.

In some embodiments, the at least one axis is located at or bisecting the end of the gases delivery side member and/or where the patient conduit is connected to the gases delivery side member or the patient interface.

In some embodiments, the at least one axis is oriented parallel with, or along a height of the gases delivery side member and/or the collapsible portion,
optionally said axis being oriented substantially parallel to a patient's face in use,
optionally said axis being oriented substantially parallel to a portion of the gases delivery side member and/or the collapsible portion configured to contact a user's face,
optionally said axis being oriented substantially parallel to a front face and/or a rear face of the gases delivery side member and/or the collapsible portion.

In some embodiments, the bridging section is configured to deform before the gases delivery side member when a force is applied to the conduit in a direction towards, or away from, a patient's face.

In some embodiments, the bridging section is configured to be deformable when the patient conduit engages with the engagement portion.

In some embodiments, the bridging section is configured to be deformable in a direction towards or away from a patient's face, in use, when the patient conduit engages with the engagement portion.

In some embodiments, deformation of the bridging section is configured to isolate the gas delivery side member from forces applied to the conduit.

In some embodiments, the engagement portion is configured to engage with an internally facing surface and/or a patient facing surface of the patient conduit.

In some embodiments, the engagement portion is configured to engage with an externally facing surface or a surface facing away from a patient of the patient conduit.

In some embodiments, the engagement portion is configured to be freely moveable relative to the patient conduit, optionally the engagement portion is configured to be freely moveable along a length of the patient conduit.

In some embodiments, the engagement portion is configured to be connected, or connectable to the patient conduit, optionally so as to prevent relative movement.

In some embodiments, the engagement portion comprises one or more connection features configured to allow for connection between the engagement portion and the patient conduit.

In some embodiments, the bridging section comprises a relatively flexible section, optionally the relatively flexible section comprises a material with shape memory.

In some embodiments, the bridging section is relatively more flexible torsionally about an axis along the bridging section, optionally the axis extends along a length of the bridging section, optionally the axis extends in a direction along an axis of the patient conduit.

In some embodiments, the gases delivery side member comprises a collapsible portion.

In some embodiments, the engagement portion engages the patient conduit at a location away from the gas delivery arm and/or a location away from where the patient conduit is connected to the gases delivery side member or the patient interface.

In some embodiments, the engagement portion comprises a loop, wherein the loop extends around at least part, or the entirety of the patient conduit.

In some embodiments, the patient conduit is configured to be located within the loop.

In some embodiments, a diameter of the loop is larger than a diameter of the patient conduit.

In some embodiments, the patient conduit is configured to be moveable within the loop, to allow relative movement between the patient conduit and the loop.

In some embodiments, a diameter of the loop is the same, or smaller than, a diameter of the patient conduit.

In some embodiments, the patient conduit is configured to be retained within the loop, to prevent relative movement between the patient conduit and the loop.

In some embodiments, the engagement portion comprises a C-shaped portion.

In some embodiments, the engagement portion and bridging section are integrally formed.

In some embodiments, the engagement portion and bridging section are integrally formed with the patient interface and/or the gases delivery side member.

In some embodiments, the patient conduit is configured to be directly connected to the end of the gases delivery side member

In some embodiments, the patient conduit is configured to be connected to the end of the gases delivery side member by one or more connector.

In some embodiments, the patient conduit is integrally formed with the gases delivery side member.

In some embodiments, the gases delivery side member is connected to the patient interface by one or more threaded connection.

In some embodiments, the gases delivery side member is internally threaded.

In some embodiments, the interface comprises a connection feature for connection of the gases delivery side member to a head strap and/or an associated headgear, optionally the connection feature is one or a pair, or a plurality of slots or a clip.

In some embodiments, the bridging section and/or the engagement portion comprises the connection feature.

In some embodiments, the connection feature is integrally formed with the bridging section and/or the engagement portion.

In some embodiments, the connection feature is connectable and disconnectable from the bridging section and/or the engagement portion.

In some embodiments, the connection feature is connected to the head strap and/or the associated headgear at a location rearward to the engagement portion and/or the bridging portion and/or further from the at least one nasal prongs.

In some embodiments, the connection feature provides for a pivotable connection with the head strap and/or the associated headgear, optionally pivotable in a direction towards or away from a patient's face in use.)

In a further form there may be a patient interface comprising:
a manifold,
a pair of nasal prongs extending from the manifold to be received by a user's nares,
a dipped region substantially between the pair of nasal prongs, and
a ramp portion disposed in the manifold,
wherein the dipped region and the ramp portion work in combination to direct a flow of gases to the pair of nasal prongs.

In some embodiments, the dipped region and ramp portion work in combination to provide a substantially equal distribution of gases flow to each of the pair of nasal prongs.

In some embodiments, the manifold and said ramp disposed within said manifold are integrally formed or are a single piece.

As mentioned, the cross-sectional profile of the non-air delivering side is preferably an elongated asymmetric lens, e.g. a bottom portion (skin side) has a depth greater than a top portion (outer side), such that the patient's skin and the mask conform to the contours of the bottom and top portions, respectively, under the force of a mask when pressed over the cannula onto the patient's face. This advantageously allows for a substantially airtight seal to be formed around the non-air delivering side of the nasal cannula between the patient's skin and the bag mask.

The cross-sectional profile of the collapsible region of the air delivering side includes a thinned portion. It is intended that the material of the collapsible region of the nasal cannula will fold in the vicinity of this thinned region (as opposed to at a deliberately formed point in the internal wall). This thinned region will therefore advantageously provide a range of fold points for the material of the collapsible region to collapse.

When used in combination with the cannula, a flow-compensated pressure relief valve (FCPRV) provides a hysteresis effect of flow venting from the valve with respect to force applied to the collapsing cannula. As a result, the force required to collapse (or block) the collapsing cannula is greater than the force required to unblock the collapsing cannula. This provides the advantage of requiring that the medical professional make a very deliberate and intentional action to both apply (causing cannula collapse) and then subsequently remove (causing cannula uncollapse) the resuscitation mask.

The invention preferably has a provision of some predetermined restriction of the air delivering side of the cannula. For example, the FCPRV can be set to accommodate a specific predetermined system resistance to flow. However, different sizes of cannula (i.e. small, medium, large) will present different resistances to flow. Therefore, it is desirable for different sizes of cannula to be provided with different sized restrictions to compensate for differences in resistance to flow. This is such that the system resistance to flow will be the same regardless of the size of the cannula and will ensure that all sizes of the cannula are compatible with the same size of FCPRV.

Preferably, the external surface of the collapsing cannula is slightly roughened. This may be achieved by any means known in the art, such as bead-blasting, chemically etching, laser etching, or roughening with sandpaper the surfaces of the mould tool. A slightly roughened external surface advantageously prevents the resuscitation mask sticking to the collapsing cannula and pulling the cannula away from the patient's face when applied and subsequently removed. Preferably, the mould tool would have a roughness value (Ra) between 0.1 - 100µm, or 0.1 - 10µm

In another aspect, this disclosure relates to a patient interface comprising a breathing conduit as described in any one or more of the above statements.

In some embodiments, the interface is a nasal interface comprising a single inlet, at least one nasal outlet, and the breathing conduit extending between the single inlet and the at least one nasal outlet.

Any of the aforementioned features or embodiments or aspects may be combined with one or more of the other features or embodiments or aspects as described herein.

The term 'conduit' as used in this specification and claims is intended to broadly mean, unless the context suggests otherwise, any member that forms or provides a lumen for directing a flow of gases. For example, a conduit or conduit portion may be part of a patient interface or may be a separate conduit attachable to a patient interface to provide a flow of gases to the patient interface

The phrase 'lateral cross section' of a conduit means a cross section transverse to the flow path of the conduit, e.g. perpendicular to a flow path or longitudinal axis of the conduit. As a further example, the lateral cross section may be viewed from an end of the conduit.

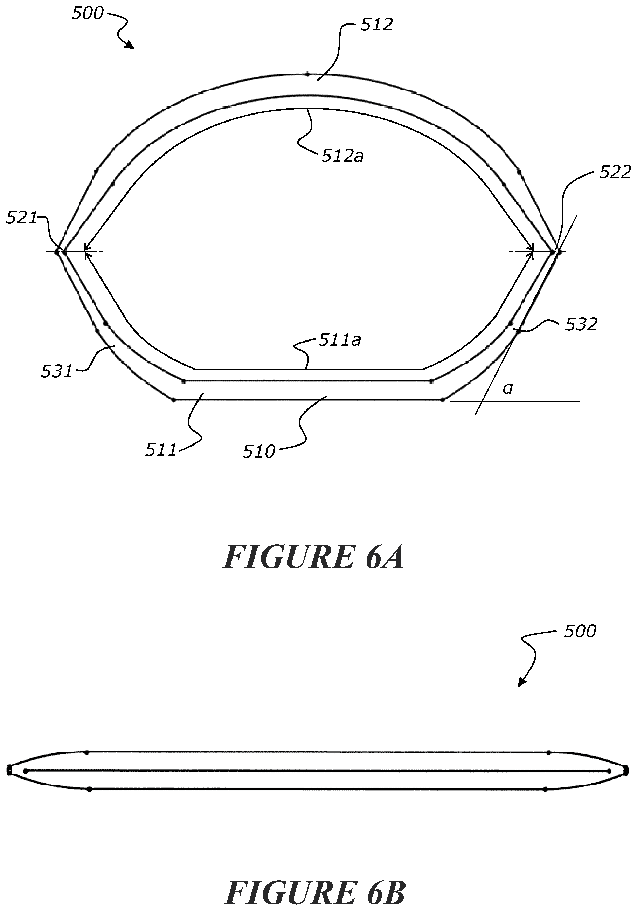

Unless the context suggests otherwise, the thickness of a side or a portion of a lateral cross section of a conduit is the lateral wall thickness of the side or portion of the cross section. For example, the thickness at a point (i.e. a fold point) on the cross section is the smallest distance across the wall of the cross section from an outer surface to an inner surface of the wall at that point. For example, in Figure 6A, the thickness of a fold point 522 is the distance from the outside surface to the inside surface at the fold point 522, for example along a line extending through a centre of the cross section, or a line extending across the cross section extending through both fold points 522 of the cross section.

In this specification and claims, a conduit described as being in a collapsed configuration such that a flow of gases through a conduit is blocked, sealed or occluded, may allow an acceptable level of leak through the collapsed portion of the conduit. An acceptable level of leaking gases flow through the conduit in a collapsed configuration may be 10L/min or less.

The term "comprising" as used in this specification and claims means "consisting at least in part of". When interpreting each statement in this specification and claims that includes the term "comprising", features other than that or those prefaced by the term may also be present. Related terms such as "comprise" and "comprises" are to be interpreted in the same manner.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

As used herein the term "and/or" means "and" or "or", or both.

As used herein "(s)" following a noun means the plural and/or singular forms of the noun.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

The disclosure consists in the foregoing and also envisages constructions of which the following gives examples only. Features disclosed herein may be combined into new embodiments of compatible components addressing the same or related inventive concepts. For example, a retention mechanism of one illustrated embodiment may be combined with a patient interface having an air delivery side and collapsible wall of a cannula of another illustrated embodiment. Likewise, one embodiment of a collapsible wall on the air delivery side can be combined with another embodiment of a collapsible wall on the other side, or a non-air delivery side as necessary.

### BRIEF DESCRIPTION OF THE FIGURES

Preferred embodiments of the disclosure will be described by way of example only and with reference to the following drawings.
**Figure 1** shows a respiratory therapy system.
**Figure 2** shows a patient wearing a patient interface.
**Figure 3** shows a patient wearing a patient interface (a first patient interface) and a face mask (a second patient interface).
**Figure 4** shows a cross-section of a portion of a patient interface or conduit.
**Figure 5** shows a typical airway of a patient.
**Figures 6A and 6B** show a lateral (across the flow path of the conduit) cross section of a collapsible portion of a conduit. Figure 6a shows the conduit in a first or open configuration, and Figure 6b shows the conduit in a second or closed configuration.
**Figures 7A to 7C** show alternative lateral cross sections for a collapsible conduit portion.
**Figures 8A and 8B** show alternative lateral cross sections for a collapsible conduit portion.
**Figure 8C** illustrates a possible collapsed profile for the cross section of Figure 8a.
**Figure 8D** illustrates a possible collapsed profile for the cross section of Figure 8b.
**Figures 9A to 9H** show a nasal cannula. Figure 9A is a perspective view, Figure 9B is an exploded perspective view, Figure 9C is a top (plan) view, Figure 9D is a front view, Figure 9E is a side view, Figures 9F to 9H are 'transparent top, front and side views respectively.
**Figures 10A to 10D** show a nasal cannula. Figure 10A is a perspective view, Figure 10B is an exploded perspective view, Figure 10C is a top (plan) view, Figure 10D is a side view on a connector of the cannula.
**Figures 11A to 11D** illustrate parts of a headgear connector engaging and disengaging. Figure 11A shows the connector parts separated, Figure 11B shows the connector parts connected together, Figure 11C shows the parts rotated relative to one another to disengage, and Figure 11D is a cross section on line I-I in Figure 11C.
**Figures 12A and 12B** illustrate soft portions of a user's face, and **Figure 12C** illustrates a cannula positioned across a soft portion of a user's face.
**Figures 13A and 13B** illustrate geometries of two embodiments of a cannula.
**Figures 14A to 14B** illustrate a cannula with alternative headgear.
**Figure 15** is a perspective view of a cannula and a shield configured to attach to the cannula to support the cannula against collapse.
**Figures 16A to 16E** show a nasal cannula. Figure 16A is a perspective view, Figure 16B is a front view, Figure 16C is an exploded view, Figure 16D is a perspective view showing a compliant cannula body rotated relative to a relatively rigid cannula member, and Figure 16E is a cross section on line I-I in figure 16B.
**Figure 17** is a perspective view of a nasal cannula.
**Figures 18A to 18D** show a nasal cannula. Figure 18A is a front perspective view, Figure 18B is a rear perspective view, Figure 18C is a cross sectional view showing an actuating lever in an open position to allow a flow of gases to flow from nasal prongs of the cannula, and Figure 18D is a cross sectional view showing an actuating lever in a closed position to block a flow of gases flowing from nasal prongs of the cannula.
**Figure 19** is an exploded view of an oral interface.
**Figure 20** illustrates a collapsible conduit portion in a collapsed configuration and with gaps or leak paths adjacent folded edges of the conduit.
**Figures 21A(i) to 21A(iii)** illustrate a collapsible conduit with an insert. Figure 21A(i) is a view on a front side of the conduit, i.e. a side of the conduit that faces away from a user's face in use. Figure 21A(ii) is a front end perspective view. Figure 21A(iii) is an end view.
**Figures 21B(i) to 21B(iii)** illustrate the conduit and insert of Figures 21A(i)-(iii) but in a collapsed configuration with the insert blocking flow through the conduit.
**Figures 22A and 22B** are sectional views of a collapsible conduit with an insert. Figure 22A shows the conduit in an open configuration allowing flow past the insert, and Figure 22B shows the conduit in a collapsed configuration with the insert blocking flow through the conduit.
**Figures 23A to 23D** are sectional views of a collapsible conduit with an insert. Figures 23A and 23C show the conduit in an open configuration allowing flow past the insert, and Figures 23B and 23D show the conduit in a collapsed configuration with the insert blocking flow through the conduit. Figures 23A and 23C are cross sectional views on a longitudinal axis of the conduit and Figures 23B and 23D are cross sectional views across the longitudinal axis of the conduit.
**Figure 24** is a cross sectional view of a collapsible conduit.
**Figure 25A** illustrates a ring member for use with or forming part of a collapsible conduit.
**Figure 25B** illustrates a conduit with the ring member of Figure 25A, viewed from a direction lateral to a user's face.
**Figure 25C** illustrates another ring for use with or forming part of a collapsible conduit.
**Figures 25D(i) and 25D(ii)** illustrates a conduit with two ring members. Figure 25D(i) shows the conduit in an open configuration and Figure 25D(ii) shows the conduit in a collapsed configuration.
**Figure 25E** illustrates a double ring member or assembly for use with or forming part of a collapsible conduit.
**Figure 25F(i) and 25F(ii)** illustrate a ring member with pivoting lever for use with or forming part of a collapsible conduit. Figure 25F(i) shows the conduit in an open configuration and Figure 25F(ii) shows the conduit in a collapsed configuration.
**Figures 26A to 26C** are cross sectional views of a conduit comprising a flap valve. Figure 26A shows the conduit in an open no-flow configuration, Figure 26B shows the conduit in an open flow configuration, and Figure 26C shows the conduit in a collapsed configuration.
**Figure 27A** is a view of a conduit supported on a patient interface side arm or headgear strap comprising a profile to assist in bending and/or kinking the conduit into a collapsed configuration.
**Figures 27B(i) and 27B(ii)** illustrate a conduit supported on a profile comprising two spaced apart projections to assist in bending and/or kinking the conduit into a collapsed configuration. Figure 27B(i) shows the conduit in an open configuration and Figure 27B(ii) shows the conduit in a collapsed configuration.
**Figures 28A(i) and 28A(ii)** illustrate a conduit comprising complementary internal profiles. Figure 28A(i) shows the conduit in an open configuration and Figure 28A(ii) shows the conduit in a collapsed configuration.

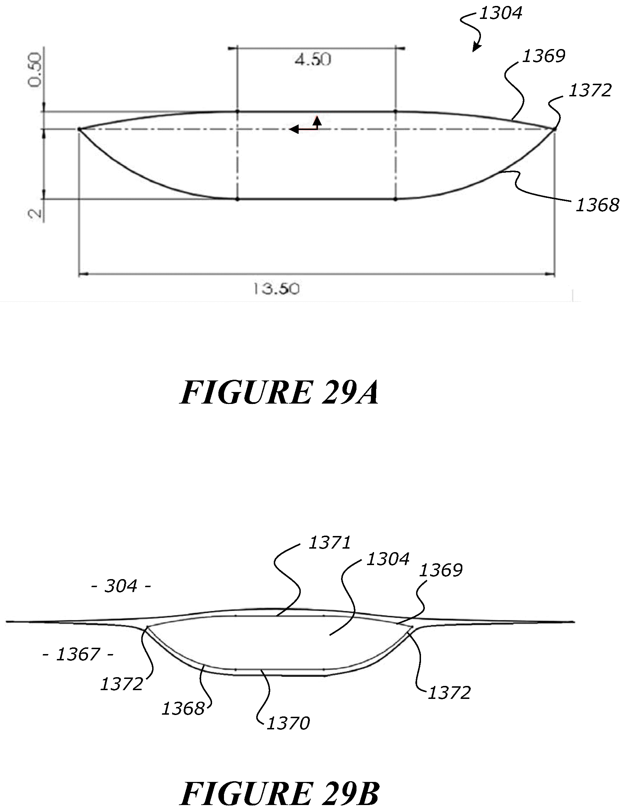

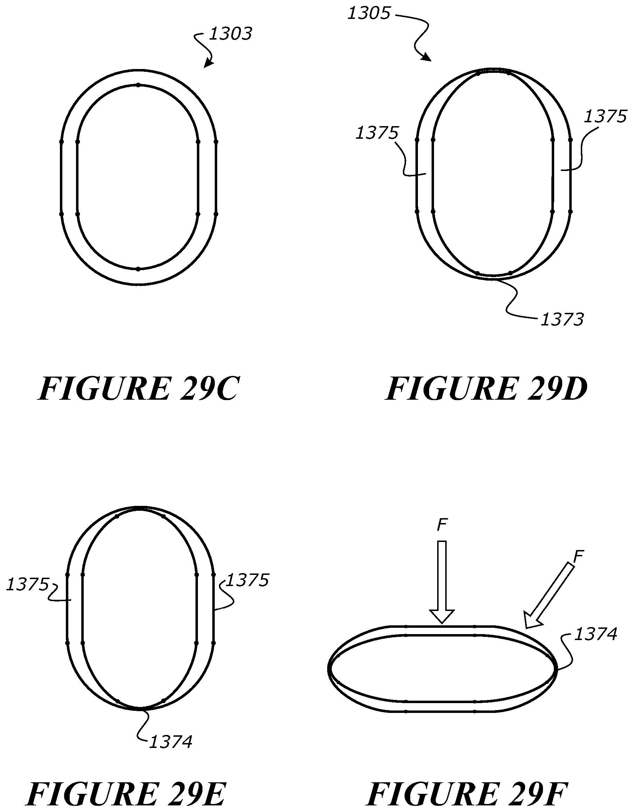

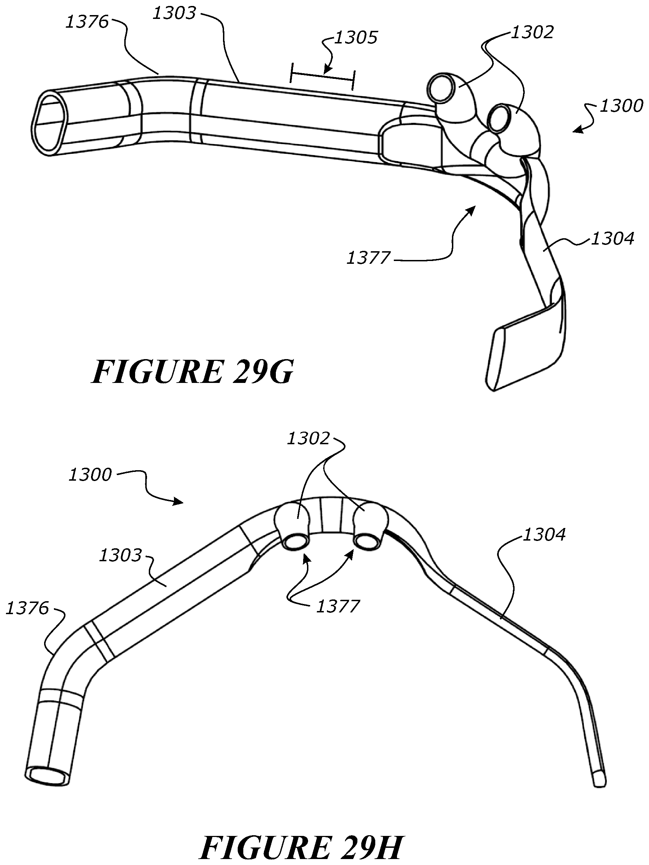

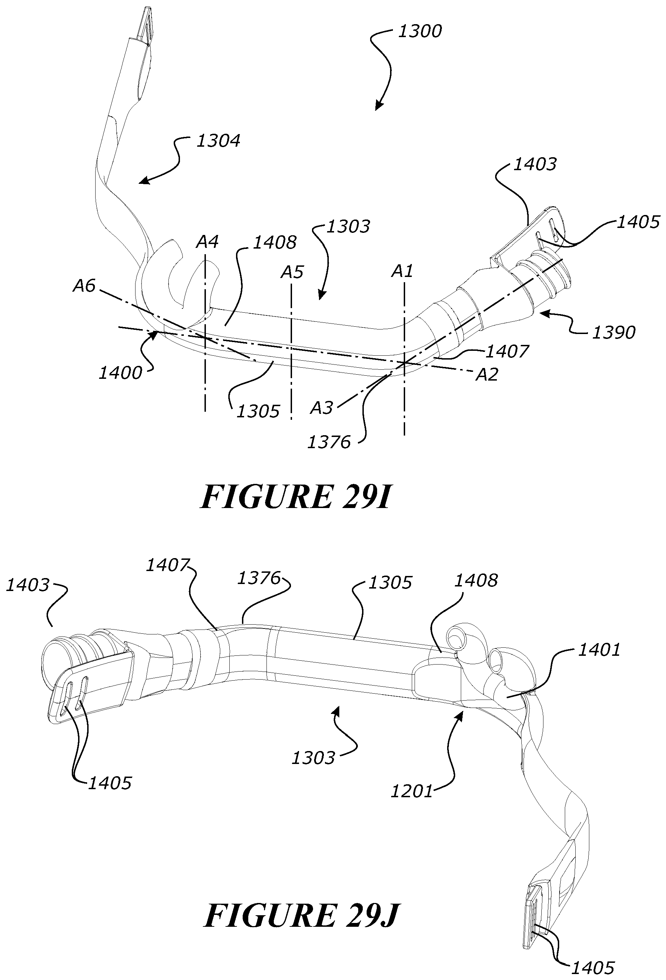

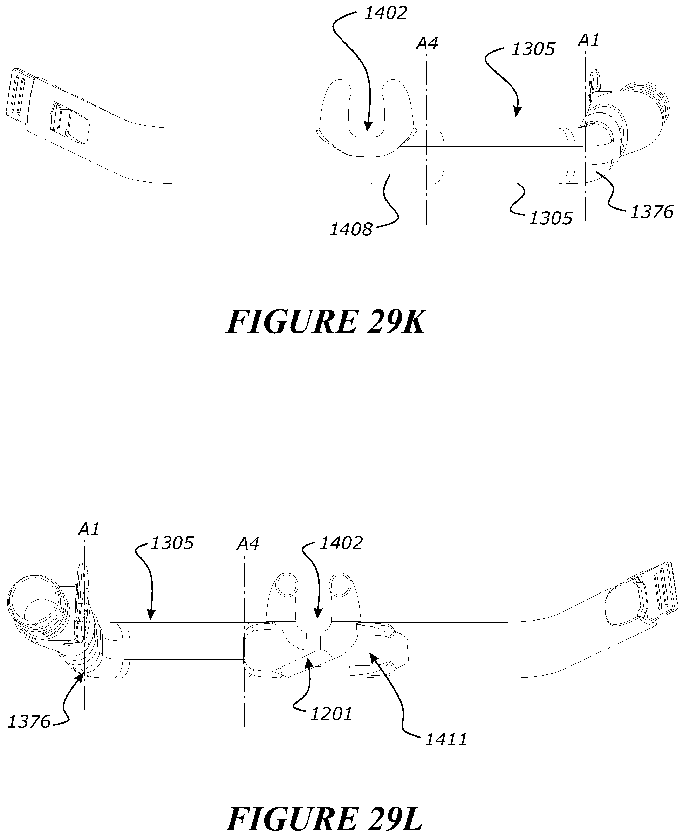

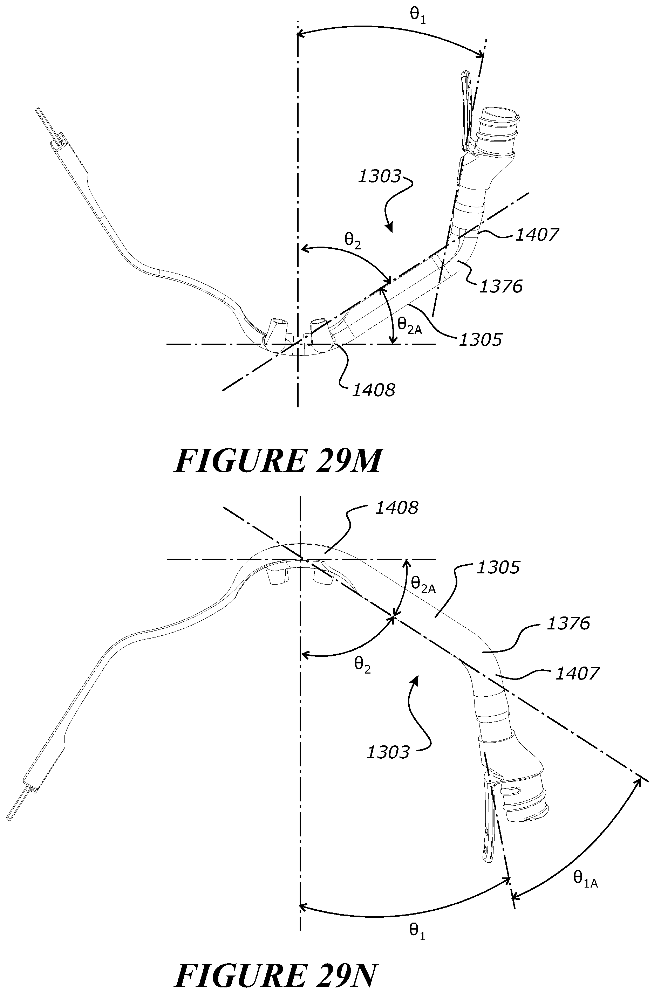

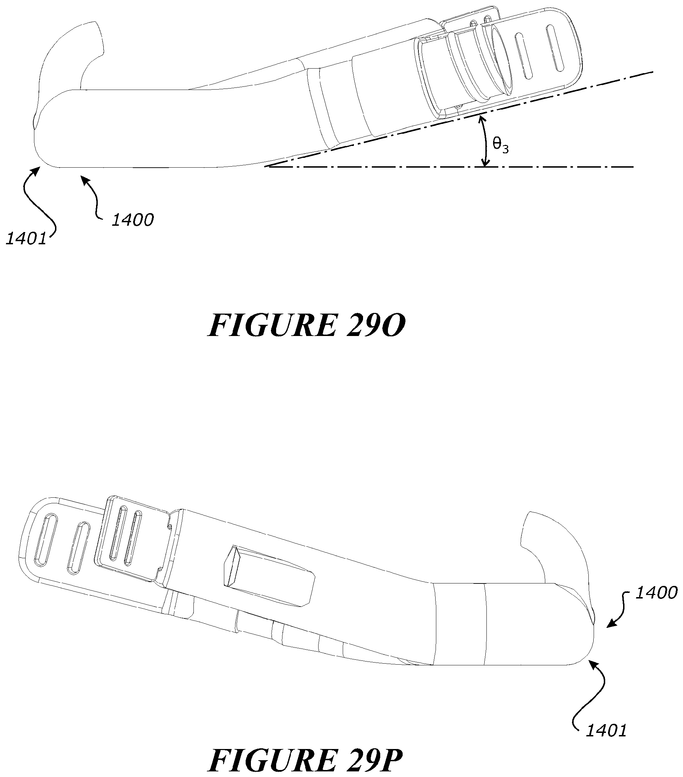

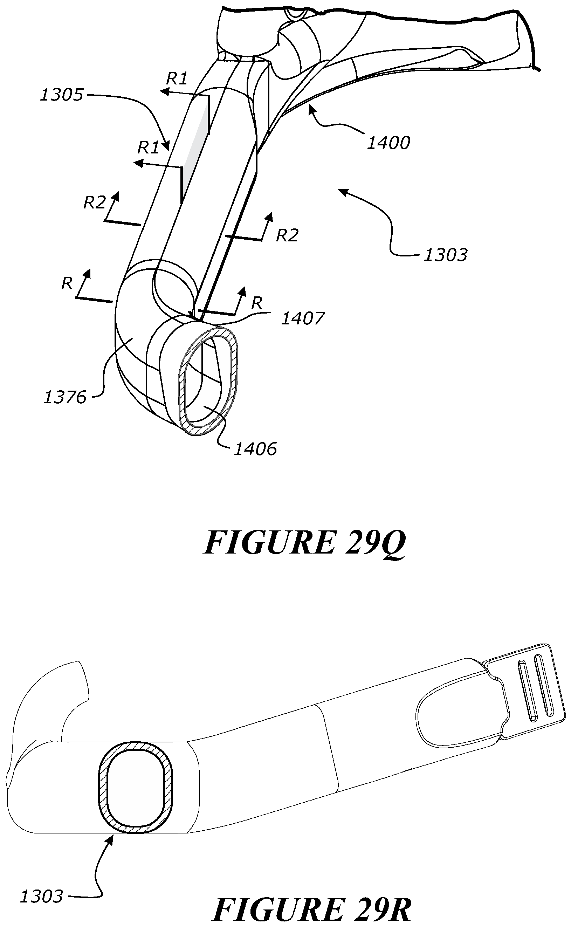

**Figures 29A to 29R2** show a nasal cannula. Figures 29A and 29B show cross section views of a non-air delivering side of the nasal cannula, Figures 29C to 29F show cross section views of an air delivering side of the nasal cannula, Figure 29G shows a rear perspective view of the nasal cannula, Figure 29H shows a plan view of the nasal cannula, Figures 29I and 29J show perspective views of the nasal cannula, Figures 29K and 29L show front and rear views of the nasal cannula, Figures 29M and 29N show top and bottom views of the nasal cannula, Figures 29O and 29P show left and right views of the nasal cannula, Figure 29Q shows another perspective view of the nasal cannula, Figures 29R-29R2 show various cross sections of the nasal cannula.
**Figures 29S to 29T** show an inspiratory connector for a nasal cannula.
**Figures 30A to 30C** show a series of side perspective views of a nasal cannula with possible headgear options, i.e. a headband, strap and pads respectively.
**Figures 31** shows a graph of pressure on the mask versus flow through the cannula providing a visual depiction of a hysteresis effect.
**Figure 32** shows a system including a collapsible cannula and flow compensated pressure relief valve (FCPRV) in combination.
**Figures 33A to 33I** show a series of views of alternative types of adhesive pad compatible with the nasal cannula and headgear of Figure 30C.
**Figures 34A to 34D** show a nasal cannula. Figure 34A and 34B show perspective views of the nasal cannula, 34C shows a side view of the nasal cannula, 34D shows a top view of the nasal cannula.
**Figures 35A to 35C** show a series of configurations of a strap connector portion for a headgear of a patient interface.
**Figures 36A and 36B** show bottom and side views of a connector, including sectional views of a connection joint between a inspiratory tube connector and a portion of a gases delivery side arm member.
**Figure 37A** shows a front perspective view illustrating internal details of a patient interface.
**Figures 37B, and 37C and 37D** show illustrative cross sectional views of alternative configurations of the patient interface of Figure 37A.
**Figures 38 and 39** shows a further embodiment of figures 29L and 29M, illustrating an arrangement of a relatively smaller nasal prong configuration.
**Figures 40A and 40B** show details of nasal prongs of a further embodiment of a nasal prong configuration.

### DETAILED DESCRIPTION

Various embodiments are described with reference to the Figures. Throughout the Figures and specification, the same reference numerals may be used to designate the same or similar components, and redundant descriptions thereof may be omitted.

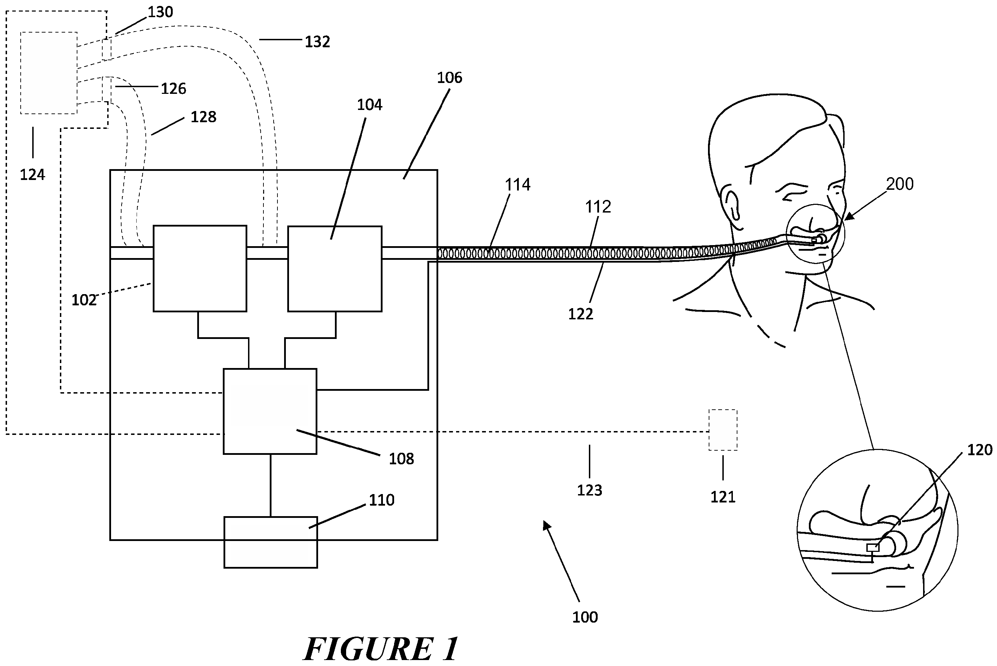

Figure 1 shows a respiratory therapy system 100. The respiratory therapy system 100 comprises a flow generator 102. The flow generator 102 is configured to generate gas flows that are passed through the respiratory therapy system 100. The flow generator 102 passes the air to a humidifier 104. The humidifier 104 is configured to heat and humidify gas flows generated by the flow generator 102. In some configurations, the flow generator 102 comprises a blower adapted to receive gases from the environment outside of the respiratory therapy system 100 and propel them through the respiratory therapy system 100. In some configurations, the flow generator 102 may comprise some other gas generation means. For example, in some configurations, the flow generator 102 may comprise a source available from a hospital gas outlet (e.g. oxygen or air), or one or more containers of compressed air and/or another gas and one or more valve arrangements adapted to control the rate at which gases leave the one or more containers. As another example, in some configurations, the flow generator 102 may comprise an oxygen concentrator. In some configurations, the flow generator 102 may be adapted to deliver a high flow therapy.

According to various configurations and embodiments described herein, a flowrate of gases supplied or provided to an interface or via a system, such as through a flowpath, may comprise, but is not limited to, flows of at least about 5, 10, 20, 30, 40, 50, 60, 70, 80, 90, 100, 110, 120, 130, 140, 150 L/min, or more, and useful ranges may be selected between any of these values (for example, about 40 to about 80, about 50 to about 80, about 60 to about 80, about 70 to about 100 L/min, about 70 to 80 L/min). Flowrates above about 15 L/min in some embodiments may be used in such configurations or embodiments, in particular but not limited to flowrates of about 60-70 L/min. 'High flow' or 'high flow therapy' may refer to the delivery of gases to a patient at a flow rate of between about 5 or 10 L/min and about 100 L/min, or between about 15 L/min and about 95 L/min, or between about 20 L/min and about 90 L/min, or between about 25 L/min and about 85 L/min, or between about 30 L/min and about 80 L/min, or between about 35 L/min and about 75 L/min, or between about 40 L/min and about 70 L/min, or between about 45 L/min and about 65 L/min, or between about 50 L/min and about 60 L/min.

Gases delivered may comprise a percentage of oxygen. In some configurations, the percentage of oxygen in the gases delivered may be between about 20% and about 100%, or between about 30% and about 100%, or between about 40% and about 100%, or between about 50% and about 100%, or between about 60% and about 100%, or between about 70% and about 100%, or between about 80% and about 100%, or between about 90% and about 100%, or about 100%, or 100%.

High flow therapy has been found effective in meeting or exceeding the patient's normal peak inspiratory demand, to increase oxygenation of the patient and/or reduce the work of breathing. Additionally, high flow therapy may generate a flushing effect in the nasopharynx such that the anatomical dead space of the upper airways is flushed by the high incoming gas flows. This creates a reservoir of fresh gas available of each and every breath, while minimising re-breathing of carbon dioxide, nitrogen, etc.

As relatively high gas delivery flow rates may be used with the embodiments or configurations described herein, the gases being supplied or delivered to the user or patient may be delivered to different parts of the user's or a patient's airway.

Such relatively high flow rates of gases may assist in providing the supplied gases into a user's airway, or to different parts of a user's airway, for example such flow rates may allow for a delivery of such gases to the upper or lower airway regions. Upper airway region typically includes the nasal cavity, pharynx and larynx, while the lower airway region typically includes the trachea, primary bronchi and lungs.

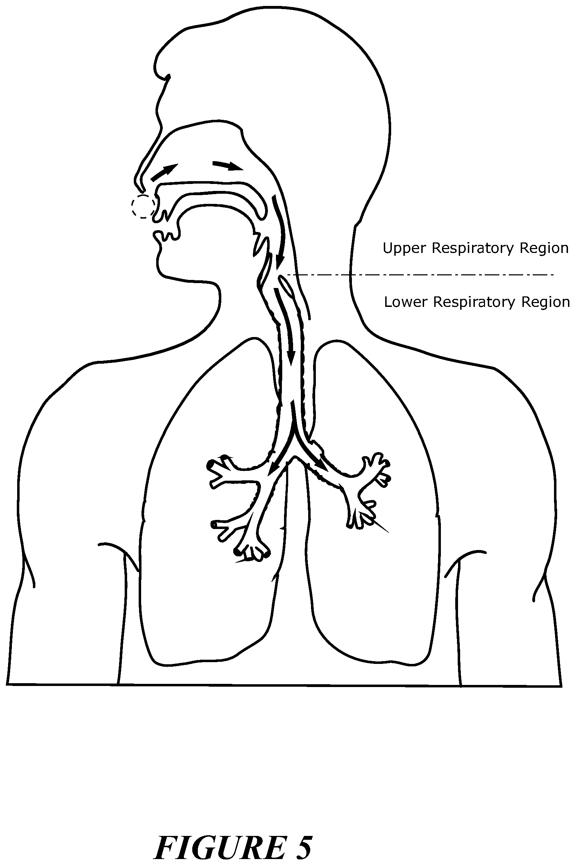

Figure 5 shows a typical airway of a person, and includes arrows to indicate how a relatively high flow rate of gases supplied to a user may be utilised to effectively push or drive the supplied gases further or deeper into a user's airway than when the person is under normal or typical self-driven respiratory conditions, or when a patient has a diminished respiratory drive.

The respiratory therapy system 100 comprises a housing 106 that at least partially houses both the flow generator 102 and the humidifier 104 (e.g. the respiratory therapy system 100 may comprise an integrated flow generator/humidifier apparatus). In other configurations the flow generator 102 and humidifier 104 may have separate housings. A hardware controller 108 is shown to be in electronic communication with the flow generator 102 and the humidifier 104, although in some configurations the hardware controller 108 might only communicate with the flow generator 102 or the humidifier 104. The hardware controller 108 may comprise a microcontroller or some other architecture configured to direct the operation of controllable components of the respiratory therapy system 100, including but not limited to the flow generator 102 and/or the humidifier 104. An input/output module 110 is shown to be in electronic communication with the controller 108. The input/output module 110 may be configured to allow a user to interface with the controller 108 to facilitate the control of controllable components of the respiratory therapy system 100, including but not limited to the flow generator 102 and/or the humidifier 104, and/or view data regarding the operation of the respiratory therapy system 100 and/or its components. The input/output module 110 might comprise, for example, one or more buttons, knobs, dials, switches, levers, touch screens, speakers, displays and/or other input or output peripherals that a user might use to view data and/or input commands to control components of the respiratory therapy system 100.

As further shown in Figure 1, a supplementary gas source 124 may be used to add one or more supplementary gases to the gases flowing through the respiratory therapy system 100. The one or more supplementary gases join the gas flow generated by the flow generator 102. The supplementary gas source 124 may be configured to deliver one or more supplementary gases including but not limited to air, oxygen (O₂), carbon dioxide (CO₂), nitrogen (N₂), nitrous oxide (NO), and/or heliox (a mixture of helium and oxygen). The supplementary gas source 124 may deliver the one or more supplementary gases via a first supplementary gas conduit 128 to a location upstream of the flow generator 102, and/or may deliver the one or more supplementary gases via a second supplementary gas conduit 132 to a location downstream of the flow generator 102 and/or upstream of the humidifier 104. One or more supplementary flow valves 126, 130 may be used to control the rates at which the one or more supplementary gases can flow from the supplementary gas source 124 and through the first and/or second supplementary gas conduits 128, 132. One or more of the supplementary flow valves 126, 130 may be in electronic communication with the controller 108, which may in turn control the operation and/or state of the one or more of the supplementary flow valves 126, 130. In other configurations, the supplementary gas source 124 may be configured to add one or more supplementary gases downstream of the humidifier 104.

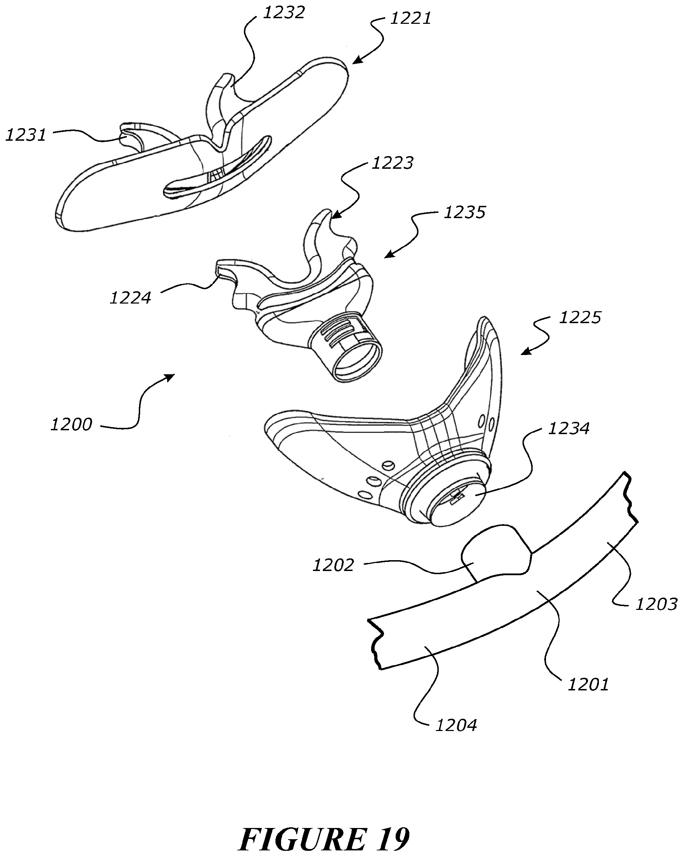

As shown in Figure 1, a conduit 112 extending from the humidifier 104 links the humidifier 104 to a patient interface 200. The conduit 112 may comprise a conduit heater 114 adapted to heat gases passing through the conduit 112. In other configurations the conduit heater 114 may not be present. The patient interface 200 is shown to be a nasal cannula, although it should be understood that in some configurations, other patient interfaces may be suitable. For example, in some configurations, the patient interface 200 may comprise a sealing or non-sealing interface, and may comprise a nasal mask, an oral mask, an oro-nasal mask, a full face mask, a nasal pillows mask, a nasal cannula, an endotracheal tube, tracheostomy tube, a combination of the above or some other gas conveying system. In a preferred embodiment, the patient interface 200 is a non-sealing interface such as a nasal cannula, which allows gases to be exchanged with the environment. For example, the non-sealing cannula allows carbon dioxide to be removed and/or cleared from the patient's airways while the patient receives flow therapy from the system 100. Further, in some preferred embodiments, the patient interface 200 is in the form of a nasal interface, such that the system does not interfere with other oral airway equipment and/or devices, for example, a tracheal tube in an intubation procedure. Accordingly, the patient may continue to receive flow therapy throughout the intubation procedure. In other embodiments, the patient interface 200 is an oral interface, for example an oral interface that is received in a user's mouth. An oral interface may be preferred in situations involving medical procedures via the nose, such that the interface does not interfere with nasal airway equipment and/or devices, for example a tracheal tube used in a nasal intubation procedure. In other embodiments the interface may be suitable for both nasal and oral placement or may be adapted between a nasal and an oral configuration. An example oral interface is illustrated in Figure 19.

As shown, in some configurations the patient interface 200 may also comprise a gas sensing module 120 adapted to measure a characteristic of gases passing through the patient interface 200. In other configurations the gas sensing module 120 could be positioned and adapted to measure the characteristics of gases at or near other parts of the respiratory therapy system 100. The gas sensing module 120 may comprise one or more sensors adapted to measure various characteristics of gases, including but not limited to pressure, flow rate, temperature, absolute humidity, relative humidity, enthalpy, gas composition, oxygen concentration, carbon dioxide concentration, and/or nitrogen concentration. Gas properties determined by the gas sensing module 120 may be utilized in a number of ways, including but not limited to closed loop control of parameters of the gases. For example, in some configurations flow rate data taken by a gas sensing module 120 may be used to determine the instantaneous flow, which in turn may be used to determine the respiratory cycle of the patient to facilitate the delivery of flow in synchronicity with portions of the respiratory cycle. The gas sensing module 120 may communicate with the controller 108 over a first transmission line 122. In some configurations, the first transmission line 122 may comprise a data communication connection adapted to transmit a data signal. The data communication connection could comprise a wired data communication connection such as but not limited to a data cable, or a wireless data communication connection such as but not limited to Wi-Fi or Bluetooth. In some configurations, both power and data may be communicated over the same first transmission line 122. For example, the gas sensing module 120 may comprise a modulator that may allow a data signal to be 'overlaid' on top of a power signal. The data signal may be superimposed over the power signal and the combined signal may be demodulated before use by the controller 108. In other configurations the first transmission line 122 may comprise a pneumatic communication connection adapted to transmit a gas flow for analysis at a portion of the respiratory therapy system 100.

Additionally as shown a physiological sensor module 121 may be present. The physiological sensor module 121 may be configured to detect various characteristics of the patient or of the health of the patient, including but not limited to heart rate, EEG signal, EKG/ECG signal, inertial sensors attached to the patient (e.g.: chest) to detect movement, blood oxygen concentration (via, for example, a pulse oximeter), blood CO₂ concentration, transcutaneous CO₂ (TcCO2) and/or blood glucose. Similarly, the physiological sensor module 121 may communicate with the controller 108 over a second transmission line 123. The second transmission line 123 may comprise wired or wireless data communication connections similarly to the first transmission line 122, and power and data may be communicated similarly. The physiological sensor module 121 may be used, for example, to determine the blood oxygen saturation of the patient.

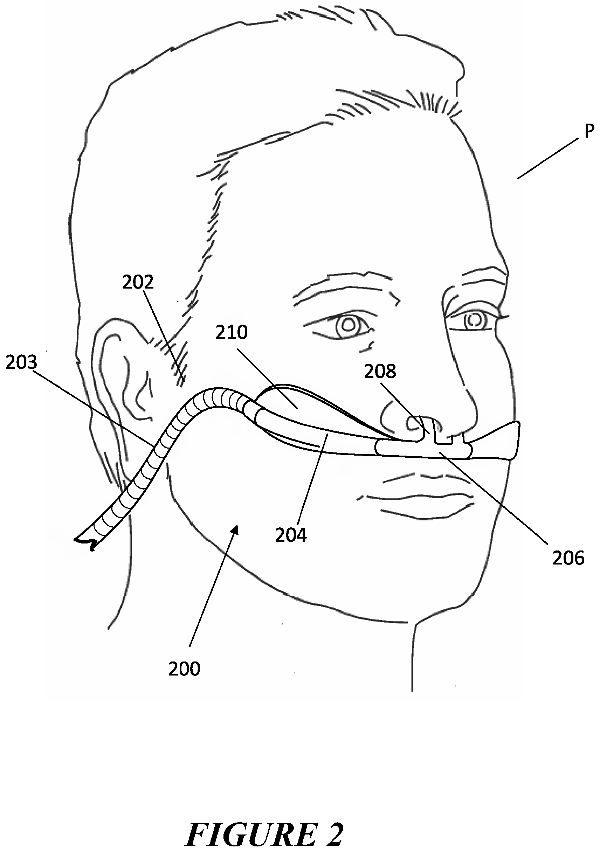

Figure 2 shows a user or patient P wearing a patient interface 200, for example the patient interface 200 of the respiratory system of Figure 1. The patient depicted is an adult, however, the patient may be an infant or juvenile. In the illustrated non-limiting configuration, the patient interface 200 is a nasal cannula. The patient interface 200 comprises a first gas conduit 202. The first gas conduit 202 is adapted to receive gases from the respiratory therapy system 100 (for example, via the conduit 112 shown in Figure 1) and channel the gases to the patient P. The first gas conduit 202 may comprise a reinforcement element 203 adapted to strengthen and/or add rigidity to the first gas conduit to prevent deformation or collapse of the first gas conduit 202 arising due to the application of forces against the first gas conduit 202. The reinforcement element 203 may include a number of structures, including but not limited to plastic or metallic reinforcing beads that lie in or on the wall of the first conduit lumen 202.

The first gas conduit 202 is in pneumatic communication with a flow manifold 206. The flow manifold 206 receives gases from the first gas conduit 202 and passes them to one or more nasal delivery elements 208 (e.g. nasal prongs). The one or more nasal delivery elements 208 extend outwardly from the flow manifold 206. The one or more nasal delivery elements 208 are adapted to be non-sealingly positioned in one or more nares of the patient P. As shown, the patient interface 200 comprises two nasal prongs 208 adapted to be positioned one in each of the patient's nares. Each nasal prong 208 may be shaped or angled such that it extends inwardly towards a septum of the patient's nose. Alternatively the first patient interface 200 may be a sealing nasal interface.

In the embodiment shown in Figure 2, the flow manifold 206 receives flow from one lateral side of the flow manifold 206 (e.g. with respect to an imaginary vertical plane bisecting the face of the patient P) and channels flow to the manifold and each of the nasal prongs 208. In some embodiments a conduit may extend from the left hand side or from the right hand side of the manifold. In some situations providing the conduit on the left hand side of the patient interface may be preferred for access for a clinician, for example for intubation. Alternatively, a conduit extending from the right hand side may be preferred, for example in procedures such as endoscopies where the patient is typically lying on his or her left hand side. In other configurations, the patient interface 200 may comprise greater (for example, three or four) or fewer (for example, one) nasal delivery element 208. In other configurations, each nasal delivery elements 208 can have different properties. For example, one of a pair of nasal delivery elements 208 can be relatively long and the other nasal delivery element 208 can be relatively short.

In some configurations, the flow manifold 206 may be configured to receive flow from two lateral sides of the flow manifold 206 (e.g. from a 'left' and 'right' of the flow manifold 206 instead of just the patient's right hand side of the flow manifold 206 as seen in Figure 2). In some such configurations, multiple gas conduits may be used to provide for pneumatic communication between the flow manifold 206 and the respiratory therapy system 100. For example, the patient interface may comprise dual conduits, the first gas conduit 203 extending from a first side of the interface (in the illustrated example the right hand side of the patient) and a second gas conduit extending from a second opposite side of the interface. In some configurations, the flow manifold 206 may be configured to receive flow from a non-lateral side of the flow manifold 206 (e.g. from a 'bottom' or 'top' of the flow manifold 206).

The patient interface may further comprise mounts and/or supports, e.g., cheek supports 210, for attaching and/or supporting the gas conduit 202 or conduits on the patient's face. Alternatively or additionally, the patient interface may be held in place via one or more headstraps or headgear.

The first gas conduit 202 of the patient interface 200 comprises a first portion 204 configured to transition from a first configuration in which a first level of gases is able to pass through the first portion 204 to a second configuration in which a second level of gases is able to pass through the first portion 204.

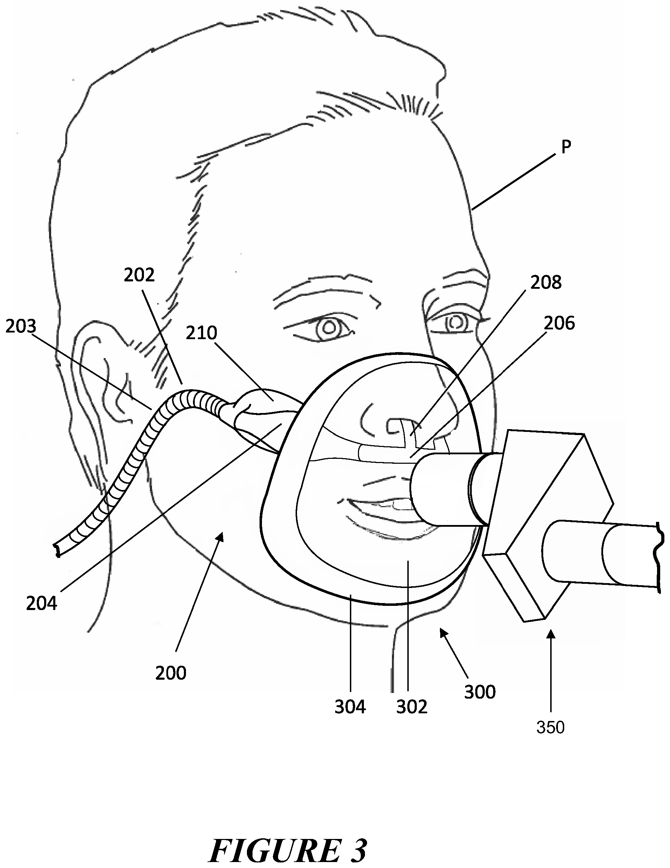

Figure 3 shows a non-limiting exemplary embodiment of a patient P wearing the patient interface 200 as shown in Figure 2 (a first patient interface) underneath a face mask 300 assembly (a second patient interface). Figure 3 schematically shows the face mask as a transparent structure in order to illustrate the patient interface 200 under it. The first patient interface 200 may be used with a first respiratory support subsystem and the second patient interface 300 may be used together with a second respiratory support subsystem.

A system may find benefit in the selective delivery of separate therapies to a patient using different patient interfaces, and/or in stopping or ceasing the delivery of a therapy from an interface and/or allowing gases provided by an interface to be sampled. The system and devices as described find particular application in emergency resuscitation, around intubation of a patient receiving high flow therapy, ear, nose, and throat (ENT) surgery, in assisting with conditioning of a patient in a pre-operative state prior to administration of anaesthetics, and during post-extubation and recovery.

Face mask assembly 300 may be used as or with a second respiratory support subsystem and/or to deliver one or more substances other than a substance delivered by the cannula 200, for example anesthetic agents or oxygen, to the patient, or the same substance but at different flow and/or pressure levels. Alternatively, the face mask assembly 300 may be used to stop the delivery of therapy from a first respiratory support subsystem. The face mask assembly 300 may also be adapted to measure respiratory gases, for example exhaled carbon dioxide from the patient, the measurements of which may otherwise be affected by flow from the patient interface 200 of the first respiratory support subsystem.

Accordingly, the embodiment shown in Figure 3 allows for the alternation between the two different respiratory support subsystems. Additionally, this configuration may allow the patient interface 200 to be left on the patient throughout the surgical procedure and/or into recovery (whether or not the patient continues to receive flow therapy through the patient interface 200 throughout the procedure) without interfering with other clinical practices.

In the embodiment shown, face mask assembly 300 comprises a full face mask 302 configured to cover both the patient's nose and mouth. In other configurations, the face mask 300 may be a nasal mask which is placed over the patient interface 200 to cover only the patient's nasal region.

As shown, the face mask 302 comprises a seal region 304 adapted to seal against the patient's face. The face mask assembly 300 is connected to a second gas source, for example via a filter element 350, which supplies the one or more other gases to the patient via the face mask. That is, the second gas source is preferably different from the source supplying gas (for example, supplementary gas source 124/flow generator 102) to the patient interface 200.

In a preferred embodiment, the face mask assembly 300 is connected to a separate gas source or a separate respiratory support device. For example, the respiratory support can be a ventilator or a CPAP or a high flow therapy device or a manual resuscitator (for example a hand held face mask with bag). Alternatively or additionally the face mask assembly 300 may be connected to a device for measuring a characteristic of respiratory gases.

Alternatively the mask assembly 300 could be connected to an anesthetic device and anesthetic gas, or air, or oxygen, or a combination of gases, can be delivered via the mask 302.

The embodiment shown in Figure 3 allows for the delivery of gas from multiple sources via at least two different respiratory support modes, and further allows a doctor, clinician or medical professional to quickly and easily change the type of respiratory support mode.

In one particular application, a patient preparing for anaesthesia can be pre-oxygenated by delivering a high flow of oxygen or humidified gases or mixture of both via a nasal cannula. In some circumstances, anaesthesiologists managing the sedation of a patient may want to switch between delivery of gas flow from one patient interface (for example a nasal cannula 200) and delivery of gas flow from another patient interface, such as via a face mask 300.

Anaesthesiologists also use a mask with a bag to oxygenate a patient, and in some instances find it more beneficial to use a bag mask if a patient's vital signs begin to drop for example to deliver more pressure or have greater control over the variation in delivered pressure. In some situations a medical professional may wish to switch between different respiratory systems or support modes. In first mode respiratory support may be provided by first respiratory support system (for example via the patient interface 200) and in a second mode respiratory support may be provided by a second respiratory support system (for example via the patient interface 300), with the support from the first system switched off. For example, the additional flow from a high flow provided by nasal interface 200 may also modify the expected behaviour of the anaesthetic circuit provided by the face mask 300, and therefore it may be advantageous to be able to turn the additional flow from the first respiratory system off.

In some configurations, the switching between two respiratory support modes or subsystems may be facilitated by a structure of the first gas conduit 202, which has first portion 204 configured to transition from a first configuration in which a first level of gases is able to pass through the first portion 204 to a second configuration in which a second level of gases is able to pass through the first portion 204.

In some configurations, the first portion 204 is configured to be more collapsible or otherwise better adapted at changing the flow of gas through the first portion 204 (therefore reducing the flow of gas through the conduit and to the patient) than other portions of the conduit 202, and/or allowing a seal of a mask to seal over the top of the conduit. In other configurations the entire conduit may be configured to be collapsible. In some configurations a vent arrangement may be provided upstream of a collapsible portion, to vent gases from the conduit upstream of the collapsible portion to atmosphere.

In some embodiments, the first configuration or first condition is a substantially open configuration and the second configuration or second condition is a substantially closed configuration. That is, the conduit 202 is configured to be more collapsible, deformable or otherwise adapted to fully close off the flow at the first portion 204 than at other portions of the conduit 202.

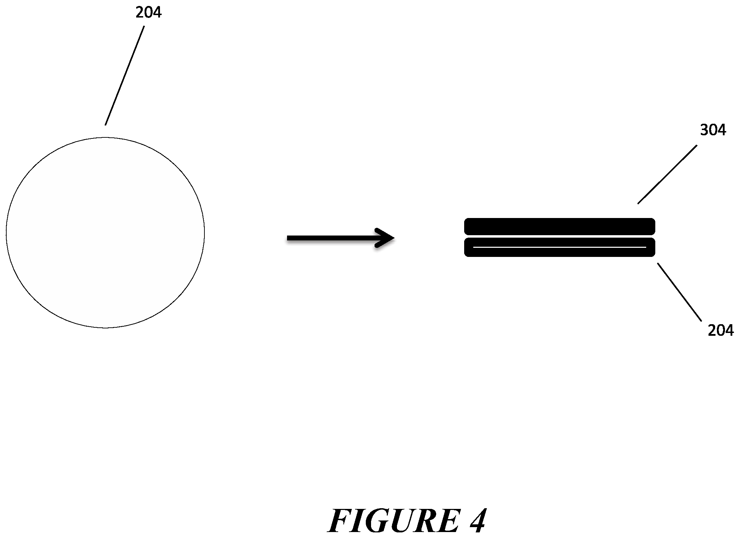

Figure 4 shows one example of this configuration, in which the conduit (for example the conduit 202 of the nasal cannula 200 of figure 3) at a first portion 204 is substantially closed by the seal 304 of face mask 302. In such an embodiment, the first portion (i.e. the more collapsible or deformable section) of the first gas conduit should be of a length that is greater than a width of a section of a seal of the face mask that bears over the first portion of the first gas conduit. This ensures the seal of the face mask does not bear over a non-collapsible section of the first gas conduit. For example, the first portion may extend from a distance of 35mm or less from the centre of a user's nose to at least 50mm from the centre of a user's nose, the first portion having a length of at least 15mm. In some embodiments the length of the first portion may be at least 15mm, 20mm, 25mm, 30mm, 35mm, 40mm, 45mm, 50mm or greater.

The first portion 204 may progress between the first and second configurations based on a relative level of force applied to the wall of the first portion 204. For example, as shown in Figure 3, the force may be applied by the seal 304 of face mask 302. In this example, first portion 204 is configured to be positioned under the seal 304 of the face mask 302.

Alternatively, the force may be applied to first portion 204 by other means, e.g., clamps (not shown), or alternatively a medical practitioner may compress the conduit by pressing on the conduit wall with a finger or thumb.

In some embodiments, the seal of the face mask acting on the first portion of the gas conduit causes the first portion to form a seal or at least a partial seal between the nasal outlets of the first patient interface 200 and the flow generator 102. Additionally, the seal of the face mask forms a seal or at least a partial seal over the first portion of the gas conduit.

Switching between respiratory support therapies is therefore achieved simply by applying a mask to the patient's face so that the seal of the mask collapses (partially or completely) the first portion of the gas conduit of the first interface 200 to 'turn off' or reduce the therapy supplied by the first interface 200 and also provides a seal between the face mask 300 and the external surface of the first portion 204 of the conduit 202 such that therapy can be provided by the mask 300 with the therapy provided by the first interface shut off. The cannula with a collapsible conduit portion allows a user, e.g. an anesthetist or a nurse or a clinician to use a mask and prevent delivery of gases from multiple sources (e.g. the mask and cannula). The first interface 200 is structured and functions in a manner to prevent the delivery of high flow and other respiratory therapy or anesthesia gases through a mask. In some embodiments the removal of the mask from the patient's face allows the therapy supplied by the first interface to recommence, as the conduit returns from the collapsed configuration to the open configuration.

Figures 6a and 6b illustrate a lateral cross section of a collapsible portion of a conduit 500, which may be integrally formed with and as part of a patient interface, for example a high flow cannula. Figure 6a shows the conduit 500 in a first or open configuration, and Figure 6b shows the same conduit 500 cross section in a second or collapsed/closed configuration. In the open configuration gases may flow along the conduit and in the closed configuration the collapsible portion is substantially sealed/occluded so that gases flow along the conduit substantially ceases.

In the embodiment of Figure 6a, the lateral cross section of the collapsible portion of the conduit 500 comprises a first side 511 with a flat portion 510 for positioning against a user's face. The flat portion is thought to assist with positioning the conduit on a user's face, for example assisting with holding the tube in a correct orientation on the user's face for when a mask is applied over the tube, and/or assist with the conduit moving from the open to the closed configuration. The flat portion may help the conduit to rest on the user's face and provide a stable mounting surface. A second side 512 of the conduit is opposite to the first side and faces away from the user's face. The first and second sides are joined by first and second fold points 521 and 522. In the open configuration the fold points are spaced away from the flat portion of the first side and therefore away from the user's face. In an alternative embodiment (not illustrated) the fold points may be coplanar with the flat section. The collapsible cross section may be such that the fold points are adjacent or rest on the face. In the configuration shown in Figure 6A the internal length of the first side and the second side being equal allows for flat folding of the collapsible portion.

In a partially closed or closed configuration the second side is moved towards or against the first side with the collapsible portion folding at the first and second fold points. In the closed configuration the fold points 521, 522 may be moved to be against or adjacent the face of a user. In order to assist with the conduit sealing closed in the closed configuration to substantially prevent a flow of gases along the conduit and/or to assist with providing a substantially flat conduit over which the mask seal may seal against, an inner length of the first side between the fold points and an inner length of the second side between the fold points are substantially equal. This configuration may assist the collapsible portion to achieve a substantially flat configuration, or a configuration in which the inner surfaces of the first and second sides of the conduit come together in contact substantially fully along their lengths (e.g. without bubbles, ripples or wrinkles), when in the closed or collapsed configuration, as shown in Figure 6b. The first and second fold points delimit or define the extent of the first and second sides. In other words, the first and second sides each extend fully between the fold points, e.g. from the first fold point to the second fold point. For example, the length of the first side 511 between the fold points 521, 522 is illustrated in Figure 6A by double ended arrow 511a, and the length of the second side 512 between the fold points 521, 522 is illustrated in Figure 6A by double ended arrow 512a

In some embodiments, the first side may comprise an outwardly curved or arcuate portion 531, 532 between the flat portion 510 and each of the first and second fold points 521, 522 when in the open configuration. The two curved portions 531, 532 preferably have the same radius of curvature, such that the collapsible section has reflective symmetry about a centre line of the cross section. Preferably the cross section has reflective symmetry about a centreline of the cross section, the centreline extending through a centre of the first and second sides of the cross section. Having a symmetrical cross section may help to ensure that the collapsible portion collapsed to a flat shape with a minimum height profile to promote sealing of the mask seal over the collapsed conduit. In some embodiments, the outward curvature or curvature of the arcuate portions 531, 532 has a radius that is sufficiently large to prevent or reduce the occurrence of creases in the conduit or gaps between the first and second sides when in the collapsed configuration. In some embodiments the outward curvature or curvature of the arcuate portions 531, 532 may help to maintain the cross-section in the open configuration when no external force is applied. In some embodiments the outward curvature or curvature of the arcuate portions 531, 532 may help to reduce the resistance to flow of the cross-section by increasing the cross-sectional area and reducing the sharpness of internal corners.

In an alternative configuration, the curved or arcuate portions 531, 532 may be inwardly curved, or the portion of the cross section between the flat portion and between each fold point may be straight or without curvature.

In some embodiments the thickness of the curved portions 531, 532 tapers from the flat portion 510 towards the respective fold point 521, 522, from a greater thickness to a reduced thickness. The change in thickness is preferably gradual along the length of the side of the cross section to reduce or prevent the occurrence of folds or creases in the side away from the fold points 521, 522. Preferably the flat section is of a thickness that is greater than the thickness of the remainder of the first side of the cross section. The thicker flat portion provides additional structure to the conduit in an area in contact with the user's face so that the conduit does not crease or buckle or fold on the user's face which may reduce the effectiveness of the conduit sealing closed when in the closed configuration.

In some configurations, the second side 512 of the conduit is curved outwardly when in the open configuration, as shown in Figure 6a. In some configurations the second side 512 is a continuous curved or arcuate portion, e.g. the second side is curved outwardly from the first fold point to the second fold point. In some embodiments the curvature of the second side may be about a single radius of curvature. In some configurations, the thickness of the second side 512 tapers towards each fold point 521, 522, from a greater thickness to a reduced thickness. In some embodiments, the second side is thickest at the centre or apex of the curved second side. The outward curve of the second side reduces a resistance to flow as compared to having a feature of the cross section that curves or extends inwardly towards a centre of the cross section.

Preferably the thickness of the fold points 521, 522 is less than the thickness of the remainder of the cross section of the collapsible portion. The relatively thin section of the fold points allows the section to be particularly adapted to fold or bend at the fold points to transition between the open and closed configurations. The conduit preferentially bends or folds at the fold points to move between the open and closed configurations. The thickness of the fold points relative to the thickness of other sections of the collapsible portion allows the collapsible portion to collapse flat so that preferably the collapsed portion substantially seals to substantially stop flow through the conduit, and additionally, to also facilitate the mask seal sealing over the top of the conduit and with the user's face at the edges of the collapsed portion. The thin fold points combined with the outwards curve of the second side may encourage a gradual tapering of the collapsed portion from the centre of the cross section towards the user's face in the collapsed configuration, reducing the chance of leaks between the conduit and the seal of the mask and the user's face.

In some embodiments, in the open configuration the first side adjacent each fold point is at an angle to the flat portion 510 such that an external angle (alpha) between the first side adjacent the fold point and the flat portion 510 is less than 80 degrees, or less than 75 degrees, or less than 70 degrees, or less than 65 degrees, or less than 60 degrees, or less than 55 degrees, or less than 50 degrees, or less than 45 degrees, or less than 40 degrees, or less than 35 degrees, or less than 30 degrees, or is between 50 and 70 degrees, or is between 60 and 70 degrees, or may be about 65 degrees. For example the angle is illustrated as being 62.6 degrees in Figure 6a.

In some configurations, the first side diverges outwardly either side of the flat portion 510 towards the respective fold point 521, 522. Preferably the first side curves into (or from) the flat portion 510 so that the cross section is without a defined 'corner' at each extent of the flat portion. A sharp corner in the first side at the edge of the flat portion may cause upwards buckling on a lower surface of the cross section, creating a gap between the conduit and the face when in the collapsed configuration.

As described above, in some embodiments the flat portion may be thicker than other portions of the cross section. For example, in some embodiments, the flat portion may have a thickness of about 0.5mm. In some embodiments, the fold points have a thickness of about 0.2mm. In some embodiments, the flat portion may have a thickness of 0.5mm to 1.5mm. In some embodiments the fold point may have a thickness of 0.2 to 0.4mm.

In some configurations, the ratio of the relative thicknesses between the (thicker) centre of the first and/or second sides of the lateral cross section and the (thinner) fold points is in the range of about 1 to 8, or about 1.5 to 3.5. In some configurations, the ratio of the relative thicknesses between the (thicker) flat portion of the first side and/or the apex of the second side of the lateral cross section and the (thinner) fold points is in the range of about 1 to 8, or about 1.5 to 3.5. In some configurations, the ratio of the thickest part of the lateral cross section to the thinnest part of the lateral cross section being the fold points is in the range of about 1 to 8, or about 1.5 to 3.5. If the ratios stated are greater than the stated range the thickest parts of the cross section may reduce the flexibility of the collapsible portion. If the ratios are less than the stated range the conduit may be too think and may collapse under its own weight and/or may result in creases, folds or wrinkles in areas outer than the fold points, which is undesirable for sealing of the conduit and also sealing with the seal of the mask over the top of the conduit. However, the walls of the collapsible section are sufficiently thin to ensure suppleness when used with the user so that the conduit is comfortable against the user's face. The above stated ratios relate to tested materials being silicone with a Shore hardness of 60 to 70A and thermoplastic polyurethane.

As an exemplary embodiment, in some configurations, the flat portion has a length of about 5mm to 10mm or about 7mm, and/or a lateral width of the cross section of the collapsible portion is between 10mm and 15mm or about 13mm. A distance between the fold points is greater than a width of the flat portion. In an alternative embodiment, the first side is without a flat portion 510. For example the first side may be curved between the fold points, the curvature (may include one or more radius of curvatures) extending from one fold point to the other fold point.

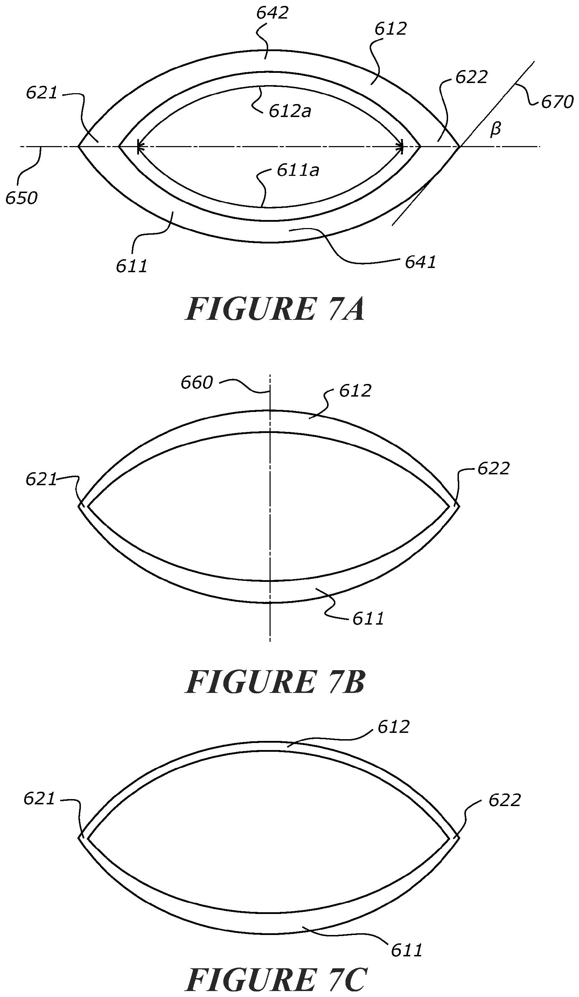

Figures 7a to 7c illustrate alternative cross sections for a collapsible conduit portion. With reference to Figure 7a, the cross section has a first side 611 for positioning against a user's face, and a second side 612 opposite the first side to face away from the user's face. Unlike in the earlier described embodiment with a flat portion 510, in the embodiments of figures 7a to 7c, the first side 611 is without a flat portion. The first and second sides are joined by first and second fold points 621, 622. The first and second fold points delimit or define the extent of the first and second sides, or in other words the first and second sides each extend fully between the fold points, e.g. from the first fold point to the second fold point. In the illustrated embodiments, a maximum width of the cross section is defined by a distance between the fold points.

In the illustrated embodiment the cross section is shaped so that the fold points 621, 622 are spaced away from the user's face in an open configuration. The first and second sides 611, 612 are curved outwardly when in the open configuration, so that the lateral cross section is substantially oval or elliptical; but in contrast to a true oval or elliptical shape which have rounded ends on a major axis of the oval or ellipse, in the lateral cross sections of Figures 7a to 7c the first and second sides converge to a point at each fold point of the cross section. When in the open configuration the fold points are spaced from the user's face and in the closed configuration the fold points are moved to be against or adjacent to the user's face.

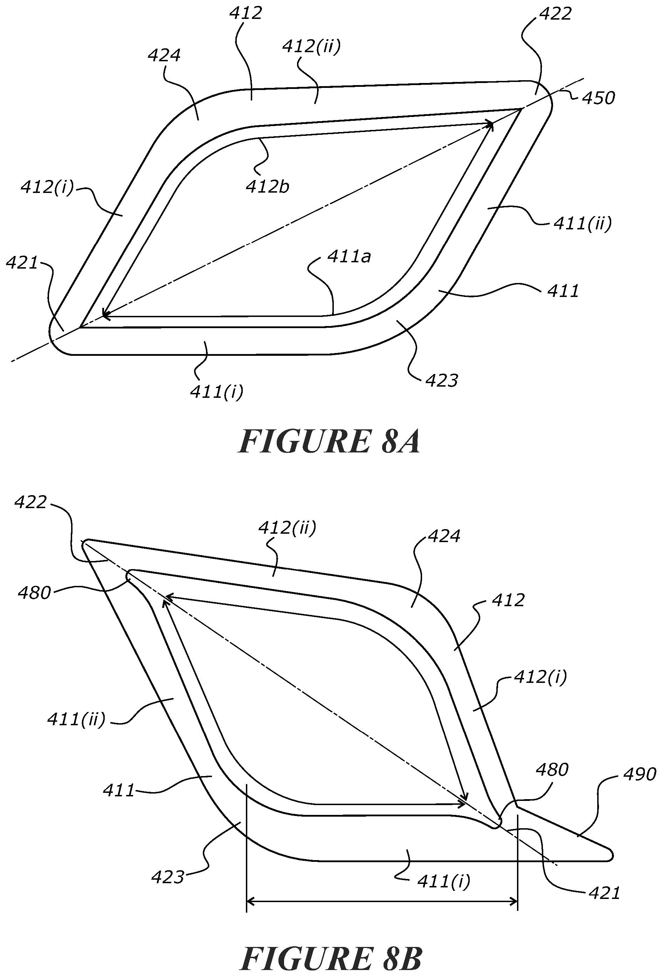

In some embodiments, for example as shown in Figures 7b and 7c, the fold points are the thinnest points of the lateral cross section, such that the collapsible portion preferentially folds at the fold points when collapsing to a closed configuration. In some configurations, for example as shown in Figures 7b and 7c, the thickness of the first side and/or the second side tapers towards each fold point, from a greater thickness to a reduced thickness. The maximum thickness is preferably at an apex 641, 642 of each of the first side and second side respectively. In some configurations, the thickness of the fold points is less than the thickness of the remainder of the cross section of the collapsible portion. In some configurations, for example as described below in relation to the embodiment of Figure 8b, the cross section may comprise an internal notch (780 on figure 8b) at the fold points so that the thickness at the fold points is reduced compare to other portions of the cross section. In some configurations, as shown in Figure 7c, the second side may be thinner than the first side, to promote collapsing of the second side towards the first side when pressed by the seal of a mask.

In some embodiments, the ratio of the relative thicknesses between the (thicker) centre of the first and/or second sides of the lateral cross section and the (thinner) fold points is in the range of about 1 to 8, or about 1.5 to 3.5. In some configurations, the ratio of the thickest part of the lateral cross section to the thinnest part of the lateral cross section being the fold points is in the range of about 1 to 8, or about 1.5 to 3.5. As described above in relation to earlier embodiments, if the ratios stated are greater than the stated range the thickest parts of the cross section may reduce the flexibility of the collapsible portion. If the ratios are less than the stated range the conduit may be too think and may collapse under its own weight and/or may result in creases, folds or wrinkles in areas outer than the fold points, which is undesirable for sealing of the conduit and also sealing with the seal of the mask over the top of the conduit.

As described above in relation to the earlier described embodiments, it is desirable that the cross section achieves a flat shape when in the closed configuration, to substantially occlude a lumen of the conduit and present a flat shape over which a seal of a mask can rest and seal against the conduit and the user's face. To assist with achieving a flat shape when in the closed configuration the cross section may comprise a number of other features. For example, in some embodiments, the lateral cross section has reflective symmetry on a line 650 extending through the first and second fold points. In some embodiments, an inner length 611a of the first side between the fold points and an inner length 612a of the second side between the fold points are substantially equal. In some embodiments, the collapsible section has reflective symmetry about a centre line (e.g. line 660 in Figure 7b) of the cross section, the centre line extending through a centre of the first and second sides of the cross section. Such features may assist with achieving a flat shape by avoiding creasing or folding other than at the fold points.

With reference to figure 7a, in some configurations, when in the open configuration a line 670 tangential to the portion of the first side adjacent to each folding point is an angle to a line 650 extending through the first and second fold points such that an angle (beta) between the line and the portion adjacent the fold point is less than 70 degrees, or less than 65 degrees, or less than 60 degrees, or less than 55 degrees, or less than 50 degrees, or less than 45 degrees, or less than 40 degrees, or less than 35 degrees, or less than 30 degrees, or is between 30 and 60 degrees, or is between 40 and 50 degrees, or may be about 45 degrees. Making this angle (beta) acute may assist in the cross section collapsing to a flat state.

Figures 8a and 8b illustrate further alternative cross sections for a collapsible conduit portion. In some embodiments, the cross section may be substantially a rhombus or parallelogram shape. In Figures 8a and 8b the shape of the illustrated cross sections is substantially parallelogram shaped, however, one skilled in the art will understand features of the parallelogram shaped cross sections described below may be applied in a rhombus shaped cross section, unless the context suggests otherwise. The four corners of the parallelogram shaped cross section provide fold points 421 to 424. In an open configuration the four sides of the parallelogram are spaced apart, and in a closed configuration the cross section folds at the corners 421, 422, 423, 424 so that adjacent sides of the parallelogram come together into contact. In the closed configuration the corners 421 and 422 comprising acute internal angles are located at edges of the collapsed cross section.

In some embodiments, the cross section is arranged so that a long side of the parallelogram is located against a user's face in use. Having a long side positioned against a user's face may assist to ensure the conduit is correct situated to be collapsed by the seal of a mask pressing over the conduit. Having the long side resting against face also reduces the profile of the collapsible portion of the conduit on the user's face and provides a cleaner more aesthetically pleasing, less intrusive look. In some embodiments however, the cross section may be configured such that a short side of the parallelogram rests against the user's face. This may be particularly useful for use with infants, as an infant or juvenile provides limited facial area to support the conduit.

To assist with collapsing of the conduit, preferably the acute corner angle of the parallelogram is less than 70 degrees, or less than 65 degrees, or less than 60 degrees, or less than 55 degrees, or less than 50 degrees, or less than 45 degrees, or less than 40 degrees, or less than 35 degrees, or less than 30 degrees, or is between 45 and 65 degrees, or is between 55 and 65 degrees, or may be about 60 degrees.

As described previously, preferably the fold points have a thinner cross section that other portions of the lateral cross section. In some configurations the thickness of the sides of the parallelogram (or rhombus) taper towards each corner (fold point) 421, 422 with an acute angle, from a greater thickness to a reduced thickness. In some configurations, the thickness of the acute angled corners (fold points) is less than the thickness of the remainder of the cross section of the collapsible portion. As shown in Figure 8b, the cross section may comprise an internal notch 480 at the corners 421, 422 comprising an acute angle so that the thickness at the corners 421, 422 comprising an acute angle is less than the thickness of the sides 411, 412 of the cross section. The thickness of the corners 423, 424 with an obtuse angle may have a thickness similar to sides of the cross section. In some configurations, the sides of the rhombus or parallelogram have a thickness of about 0.5mm to 1.0mm, or about 0.7mm, and wherein the corners comprising an acute angle have a thickness of about 0.2mm.

In some embodiments, a first side 411 of the lateral cross section extends between the two corners 421, 422 or fold points comprising an acute angle, the first side comprising two adjacent sides 411(i) and 411(ii) and an obtuse angled corner 423 of the parallelogram. An opposite second side 412 of the lateral cross section extends between the two corners 421, 422 or fold points comprising an acute angle, the second side comprising two adjacent sides 412(i) and 412(ii) and an obtuse angled corner 424 of the parallelogram. In some embodiments, an inner length 411a of the first side between the fold points 421, 422 and an inner length 412a of the second side between the fold points 421, 422 are substantially equal. In some configurations the second side 422 is thinner than the first side 421.

In some configurations, a ratio of the relative thicknesses between the (thicker) sides of the lateral cross section and the (thinner) fold points is in the range of about 1 to 8, or about 1.5 to 3.5, or the ratio of the thickest part of the lateral cross section to the thinnest part of the lateral cross section being the fold points is in the range of about 1 to 8, or about 1.5 to 3.5.

In some embodiments, a side 411(i) of the parallelogram cross section that rests against a user's face is thicker than other sides. For example, side 411(i) may be thicker than the adjacent side 411(ii) joined to side 411(i) by an obtuse angle of the parallelogram, and/or side 411(i) may be thicker than the adjacent side 412(ii) joined to side 411(i) by an acute angle of the parallelogram, and/or side 411(i) may be thicker than the side 412(i) opposite to side 411(i). In some embodiments, the ratio of the thickness of a thinner side of the cross section to the thickness of side 411(i) (i.e. the base of the cross section) is in the range of 0.3 to 0.7. In one preferred embodiment, the ratio is 0.5. For example, in one embodiment the base 411(i) of the parallelogram is about 1.4mm and the thickness of the other sides is about 0.7mm.

In some embodiments, the ratio of the length of the base (the side 411(i) in contact with the user's face) between an obtuse corner and an acute corner of the cross section and the thickness of the base is in the range of 4 to 6.

With reference to figure 8b, in some configurations the cross section of the collapsible portion comprises a tail portion 490 extending from one or both corners 421, 422 of the section comprising acute internal angles. The tail portion 490 provides a ramp at the edge of the collapsed section, from the user's face onto a top of the collapsed section in the closed configuration. The ramp or tail portion 490 thus provides a tapering or transitioning thickness at the edge of the collapsed section, allowing the seal of a mask to gradually compress from the user's face onto the collapsed section to provide an improved seal against the user's face and the collapsed portion of the conduit. In some configurations the cross section has reflective symmetry on a line 450 extending through the corners 423, 424 comprising an obtuse angle, for example such that the same ramp feature is provided at each acute angled corner 421, 422.

In some embodiments, the tail portion tapers from a height of approximately the thickness of the side of the cross section that contacts the user's face to a reduced height, for example 0.5mm or less, or may taper to a point. The height of the tail portion (where the tail portion connects to a side of the cross section, for example side 412(ii)) may be about 1mm to 3mm, or about 1mm to 2mm, or 1mm to 1.5mm, or about 1.2mm. In some configurations the height of the tail portion may be similar to the thickness of the side of the cross section that contacts the user's face. In some configurations, the height of the tail portion may be similar to the thickness of the side of the cross section that contacts the user's face plus the thickness of the opposite side of the cross section (e.g. side 412(i)) that comes into contact with the side that contacts the user's face when the cross section is in a collapsed configuration. In some configurations the height of the tail portion is about the same as the height of the collapsed cross section. A ratio of the height of the tail portion and the thickness of the thinnest section of the cross section may be about 1.2 to 1.9.

The tail portion may have a length of about 2mm to 6mm, or about 10% to about 50% of the width of the collapsible cross section, or about 30% of the width of the collapsible cross section.

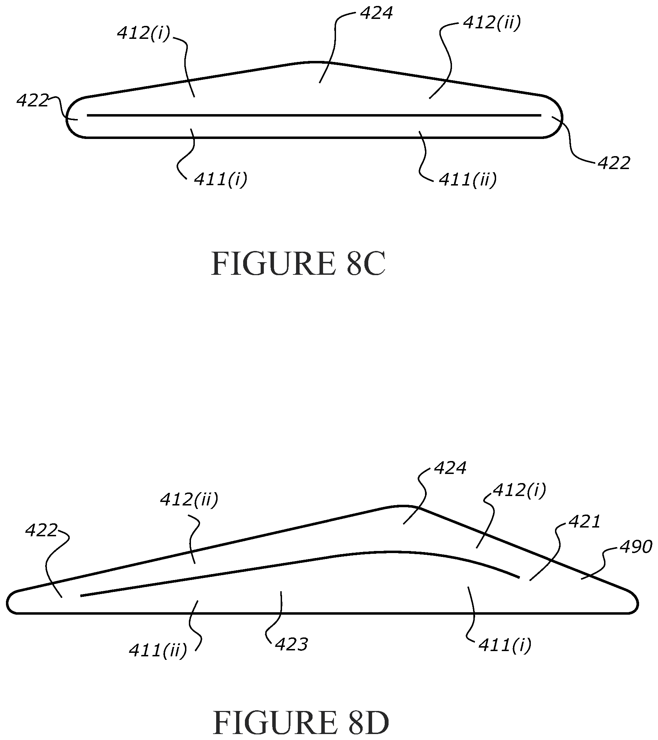

In some embodiments, the collapsible portion may have a lateral cross section configured so that in a collapsed or closed configuration the cross section forms a profile that tapers from a deeper or thicker cross section to thinner section at the edges of the collapsed section, for example as shown in Figures 8c and 8d.

Figure 8c represents a possible collapsed profile of the cross section of Figure 8a or a cross section similar to that of figure 8a. Figure 8d represents a possible collapsed profile of the cross section of Figure 8b or a cross section similar to that of figure 8b. In some embodiments, one or both of the sides of the cross section may taper in thickness from the acute angled corner(s) 421, 422 that form the fold points of the profile towards the corner(s) comprising an obtuse angle. Once collapsed the acute angle corners are located at the edges of the collapsed section. The tapering of the thickness of the side or sides from the acute angled corners to the obtuse angled corners creates a collapsed section that has a greater thickness in a portion in between the edges collapsed section, as shown in Figures 8C and 8D. In figures 8c and 8d the tapering of the thickness of the sides from the acute to the obtuse angled corners is exaggerated to illustrate the concept of having a tapering thickness to achieve a tapering collapsed section. In figure 8a, the second side 412 of the cross section comprising two adjacent sides 412(i) and 412(ii) and an obtuse angle 424 of the parallelogram tapers in thickness, from the acute angled corner 422 to the obtuse angled corner 424. The thickness of side 412 is greatest at the obtuse angled corner. The resulting collapsed profile should taper in thickness from a maximum thickness at or adjacent to the flattened obtuse angled corner 424 towards the folded acute angled corners at the edges of the collapsed section. Similarly, in the cross section of Figure 8b, the thickness of the side 412 tapers to be greater at the obtuse corner 424.

As described above, in some embodiments, a side 411(i) of the parallelogram cross section that rests against a user's face may be thicker than other sides of the parallelogram, for example as shown in Figure 8b. In a collapsed configuration, the cross section is thinner through the thinner adjacent sides 411(ii) and 412(ii) when collapsed together, and the cross section is thicker through the thicker side 411(i) and adjacent side 412(ii) when collapsed together. The thinner section through the thinner sides may provide a tapering of the cross section from the edge 422 to the thicker section resulting from the thicker side 411(i). To provide a tapering of the thickness from the other edge 421 of the collapsed section, preferably, the cross section has the ramp portion 490 at the acute angled corner at the thicker side of the cross section.

The collapsible conduit of any one of the above described embodiments may be formed from any suitable material but in one preferred embodiment may be formed from a elastomeric/resilient material such as for example silicone. The material is substantially soft and is biocompatible. In some embodiments, the collapsible portion is formed so that a natural or undeformed cross section of the collapsible portion is the open configuration. The collapsible portion is elastically deformed to move from the open configuration to the collapsed configuration, by an external force applied to a side of the conduit. When the force is removed, the conduit returns to its undeformed open configuration due to the resiliency of the conduit material. Furthermore, the collapsible portion is biased to move from the collapsed configuration to the open configuration by an internal pressure of a gases flow within the conduit expanding the conduit to the open configuration.

The above described collapsible cross-sections may form only a portion of a length of a conduit. Remaining portions of the conduit may have relatively thicker wall sections or have a different cross-section (for example round) to prevent unintended collapse of portions of the conduit other than the collapsible portion. The shape and/or wall thickness may gradually change from a cross section in a non-collapsible portion to the cross section in the collapsible portion. In some embodiments the internal cross-sectional area (e.g. the cross sectional area of the lumen of the conduit) along the collapsible portion is similar to the cross-sectional area of the inspiratory tube 112 to avoid large changes in area that could lead to turbulence and an increased resistance to flow.

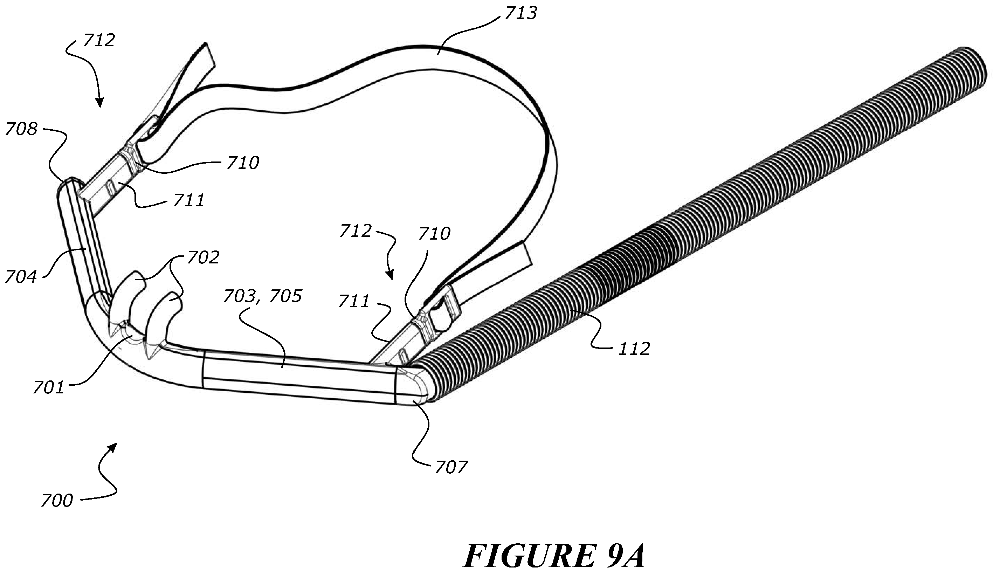

As described earlier, in some embodiments, a collapsible conduit or collapsing portion of a conduit may be integrally formed with and as part of a patient interface. An example of a nasal cannula 700 comprising a collapsible conduit portion is now described with reference to Figures 9A to 9H.

The nasal cannula 700 comprises a manifold portion 701 from which nasal prongs 702 extend. A side arm or member 703, 704 extends from one or each side of the manifold portion 701. A collapsible conduit portion 704 may be integrally formed in or with a side member of the cannula, and/or may be arranged to lie along and be supported by a side member. In some embodiments, a side member 703 is a conduit 705 for transporting a flow of gases from a patient conduit 112 to the manifold 701, e.g. the cannula comprises a conduit 705 extending from a side of the manifold 701. Substantially a full length of the conduit 705 may be configured to collapse, or a portion of the length of the conduit 705 may be configured to collapse.

In an embodiment where the cannula comprises a left side member 703 (left with respect to a patient) and a right side member 704, one or both side members may form a conduit for transporting gases to the conduit. Where both side members are conduits, two patient conduits 112 are provided, one conduit to a distal end of each side member. In some embodiments, as illustrated in Figures 9A to 9H, one side member 703 may be configured as a conduit 705 and the other side member 704 may be configured to collapse but in use to not provide a flow of gases to the manifold. In such an embodiment, the non-conduit side member 1704 may be formed as a hollow shell or body. With both the conduit 705 and the side member 704 configured to collapse, a face mask may be provided over the cannula with the cannula remaining on the face of the user. A seal of the face mask presses against a portion of each of the side member 704 and conduit 705 so that they collapse, allowing the seal of the face mask to form a satisfactory seal with the user's face and the cannula, as described earlier with reference to Figure 3. The non-conduit side member may include an inner cavity in fluid communication with ambient (i.e. via an aperture in a wall of the side arm) to allow the side member to collapse without an increase in pressure within the cavity. In some embodiments, the side arm on one side is without a conduit, such that the mask seal is required to collapse a conduit portion on one side of the interface only.

In some embodiments, the left and right side members 703, 704 may comprise the same cross section. For example, the side members 703, 704, whether used as a conduit or not, may have a collapsible cross section as described with reference to Figure 6A. If both side members have the same cross section this may allow the members to exhibit the same collapsing behaviour when the user applies an even force onto the patient's face and thus a similar seal of the facemask over both cannula side members may be achieved. In some embodiments, each of the side members may be a conduit, and a plug or cap 708 may be provided to a distal end of one side member and a conduit connector 707 may be provided to the distal end of the other side member, so that the cannula is configured as a single inlet cannula for use with a single patient conduit 112. The cannula may be configured to a dual inlet cannula by replacing the plug 708 with another connector to connect a second patient conduit. Alternatively, the cannula may be configured to a single inlet cannula with a patient conduit attached to the distal end of either the left side member or the right side member, and a plug attached to the distal end of the other one of the left and right side members. In other words, the cannula may be configurable between a left hand inlet and a right hand inlet cannula, by connecting a conduit connector and plug to the appropriate side member. In some embodiments, the conduit connector and plug and cannula side members may be configured so that the connector 707 and plug 708 may each be fitted to both the left and right side members, such that the cannula may be configured between a left or right inlet by swapping the connector and plug from the left and right side members. The plug may comprise an aperture to allow flow between ambient and the inside of the side arm, to allow the plugged side arm to move between collapsed and 'non-collapsed' states without significant pressure change within the side arm.

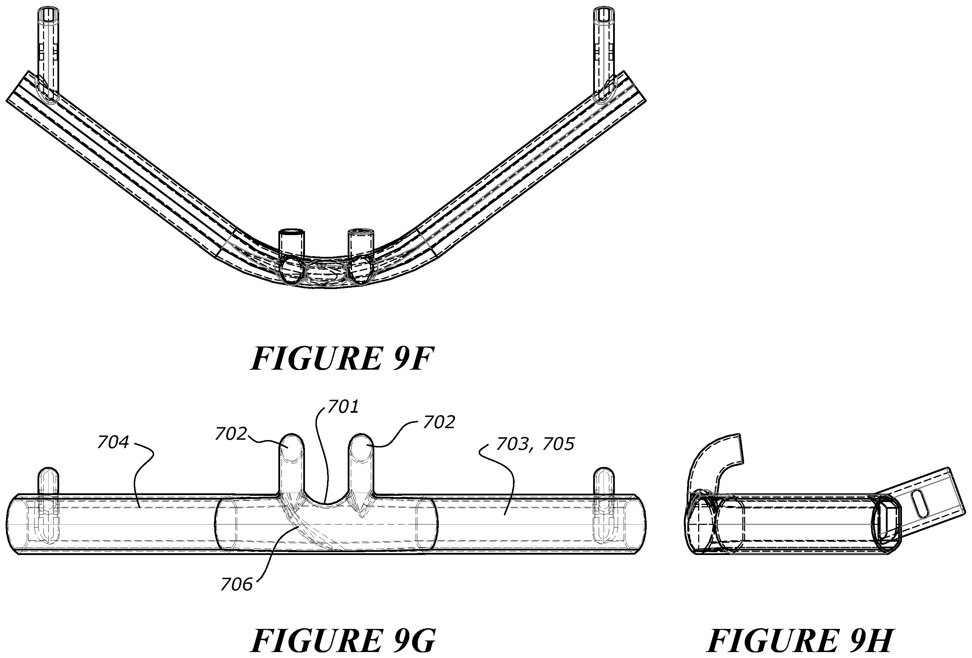

As best illustrated in Figure 9G, in some embodiments, the cannula 700 may comprise a barrier or wall 706 to separate a lumen of one side member from a lumen of the other side member. In the illustrated configuration, the cannula is a single inlet cannula. However, the cross section of the two side members may be the same, but with only one side member used as a conduit to provide a flow of gases from the patient conduit 112 to the manifold 701. In some embodiments the wall is curved to assist with directing a flow of gases from the conduit 705 into the prongs 702. This may assist with reducing the resistance to flow compared to a sharp corner. Further, this wall may act as a rib to help keep the gas path open near the prongs and prevent kinking of the cannula (for example if the cannula is bent around a patient with a small or narrow face). The wall separates the lumen or interior volume of one side arm 704 from the other 703. One side arm that is not in fluid communication with the nasal prongs 702 may have a relief hole or holes, so that the interior volume or lumen of the side member is open to the atmosphere, to allow air to escape from the interior of the side member as it is collapsed. Alternatively a relief hole may be provided in the plug 708, or no plug may be provided, e.g. the distal end of the side member may be left open. In some embodiments the interior volume of the side member not in communication with the nasal prongs may be in fluid communication with a user's exhaled breath, e.g. via a CO2 sampling tube, and a hole in the side member may be used to sample exhaled breath.

In some embodiments, the side members 703, 704 and manifold may be a unitary integrally formed member, for example from a thermoplastic elastomer (TPE), silicone or the like. In some embodiments, the side members 703, 704, manifold 701 and nasal prongs 702 may be a unitary integrally formed member. In some embodiments, the plug and/or conduit connector may be formed from a rigid material, for example HTPE, polypropylene, ABS, polycarbonate, or the like. The term rigid is used relatively with respect to the material that is used to form the side arms, which is substantially less rigid (more resilient or compliant to elastic deformation). A relatively more rigid plug or conduit connector may assist in maintaining the tube cross section in a normally open configuration. In some embodiments the side members may be formed separately to the manifold and attached or connected to the manifold. The manifold may comprise a relatively rigid material, to be more rigid that the soft or compliant side members.

In some embodiments, a headgear connector 712 is provided to each side member 703, 704. The headgear connector comprises a first part 710 and a second part 711 that releasably mate together. For example there may be a female connector part 711 and a male connector part 710 that releasably fit together. The female part 711 of the connector 712 may be formed of a resilient or flexible/compliant material, and the male part 710 may be formed from a relatively rigid material. In some embodiments, one of the connector parts may be attached to a side member of the cannula, and the other one of the connector parts attached to a headgear strap. In some embodiments, one of the connector parts may be integrally formed with a side member of the cannula, for example as illustrated in Figures 9A to 9H. In the embodiment of figures 9A to 9H, a female part or half 711 of the headgear connector is integrally formed with a side member 703, 704, and a male part or half 710 of the connector is attached to a headgear strap 713. In such an embodiment the plug 708 and conduit connector 707 may pneumatically block an opening through the female connector half that would otherwise communicate with a lumen of the side members. For example, the plug and conduit connector 708, 707 each comprises a projection 708A, 707A that fits into an inside of the female connector half 711. The projection 708A, 707A may be a projection that is received in a recess within the side arm to assist retaining the plug or connector 708, 707 within the side member against a pulling force to remove the plug or connector.

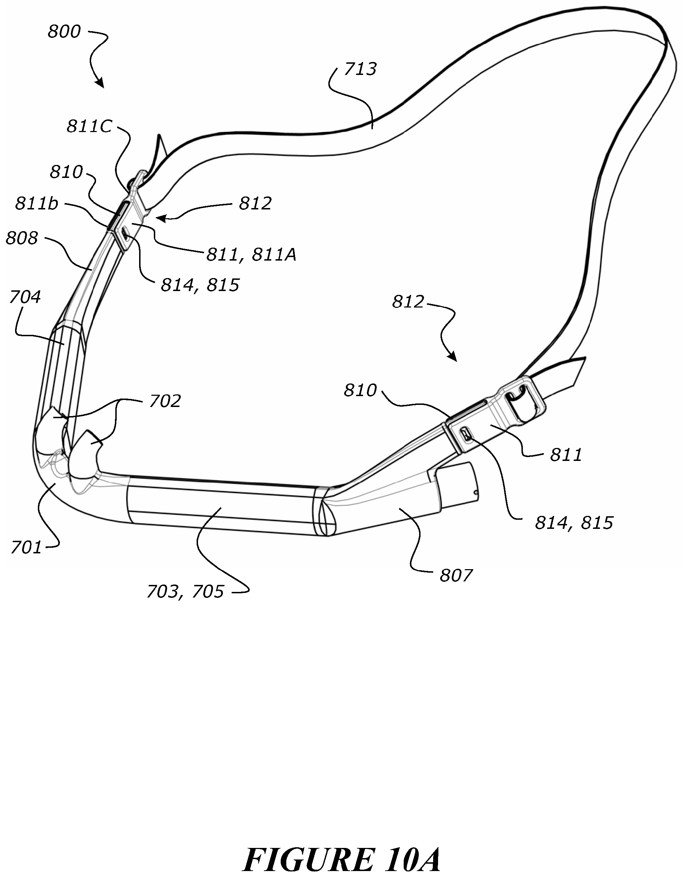

As shown in Figures 10A to 10D, in some embodiments a headgear connector half 810 is integrally formed with a conduit connector 807 or plug 808 fitted/attached to a distal end of a side member 703, 704 of the cannula 800. Having the headgear connector 812 and the conduit connector 807 integrally formed together in one relatively rigid component may increase stability compared to an arrangement where the headgear connector and conduit connector are separately connected to the relatively soft cannula body.

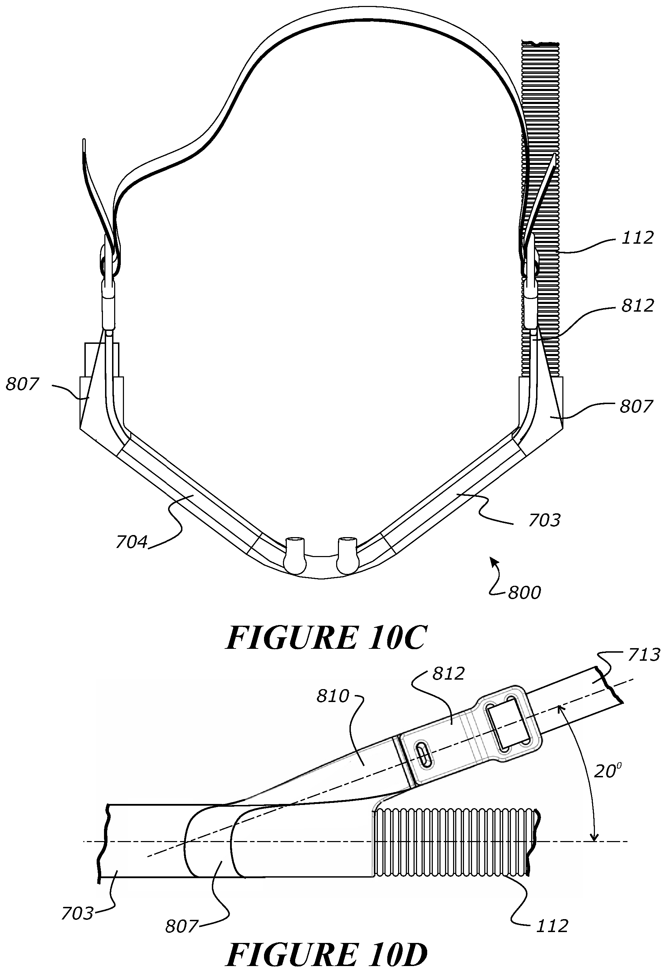

In Figure 10C the cannula is illustrated with a conduit connector 807 fitted to both side members which in some embodiments can configure the cannula into a dual inlet cannula. In such an embodiment, a wall (e.g. like wall 706 in Figure 9G) may be provided in the manifold 701 between the prongs 702, so that the prongs are pneumatically separate. A left hand prong or outlet 702 is provided a flow of gases via the lumen of the left hand side member703, and a right hand prong or outlet 702 is provided a flow of gases via the lumen of the right hand side member 704. Alternatively both the left and right hand conduits 703, 704 may be in fluid communication with both nasal outlets 702.

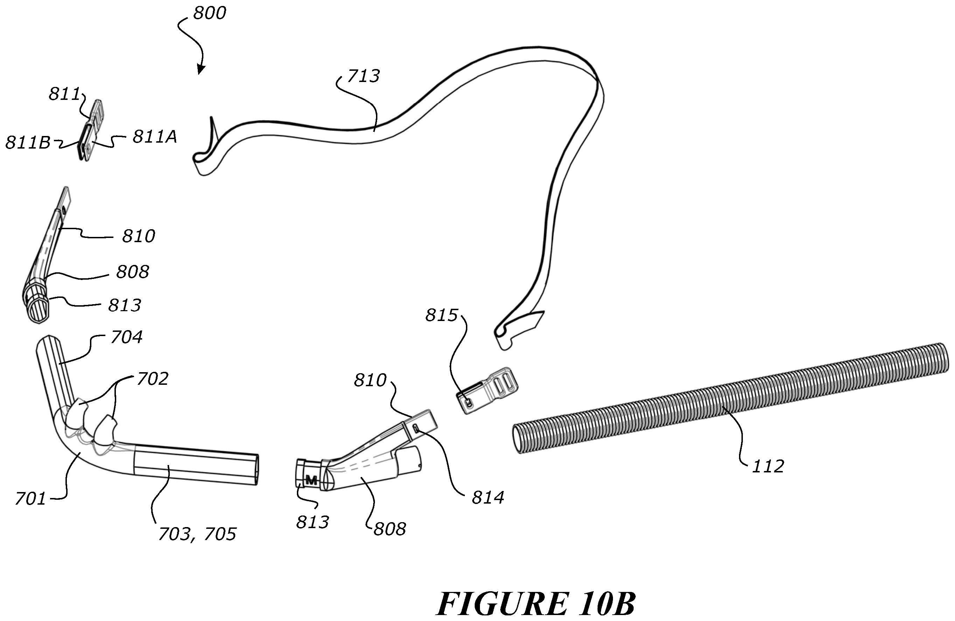

In some embodiments, as shown in Figure 10B, the plug 708, 808 and/or the connector 707, 807 may comprise a raised rib 813 to be received in a corresponding recess within the side member (not shown) to retain the plug or connector within the side member. In the illustrated embodiment of Figure 10B, the raised rib 813 is a continuous cannula rib or rim about the connector and plug, to fit in an annular recess within the side member. The rib or rim and corresponding recess may also act as a seal to prevent or minimise leakage of gases from the lumen of the side member between the side member and the connector/plug.

As shown in Figures 9A to 9H, and in figures 10A to 10D, in some embodiments the female connector half 711, 811 may comprise an aperture 715, 815 to receive a lateral projection 714, 814 of the male connector half 711, 811 to secure the connector halves together. In some embodiments the male connector half 710, 810 has a lateral projection 714, 814 on each of two lateral sides of the male connector half, and the female connector half 711, 811 has two corresponding apertures 715, 815, each configured to receive a said lateral projection 714, 814. Alternatively, in some embodiments, the male connector part may comprise an aperture to receive a lateral projection of the female part. For example, the female part may comprise a lateral projection extending from one or each lateral internal side, to mate with a corresponding aperture in the sides of the male part. The male part may comprise an aperture that extends laterally through the male part and lateral projections of the female part may engage the aperture of the male part from either side. Each lateral projection 714, 814 preferably has a bevelled edge to deflect the lateral sides of the female part. This allows the parts to be easily connected when pushed together axially. In some embodiments the or each aperture 715, 815 is a slot oriented with a major axis lateral to a longitudinal axis of a headgear strap to be attached to the patient interface, as illustrated.

In the embodiment of Figures 9A to 9H, the female connector half is formed as a socket for receiving the male connector half. To disengage the two halves of the connector one half is pulled axially from the other, to remove the male connector half from the socket of the female connector half. When pulling the connector halves 710, 711 apart, the female half elastically deflects so that sides of the female connector half ride over the lateral projections of the male connector half to release the lateral projections 714 from the apertures 715 in the female half.

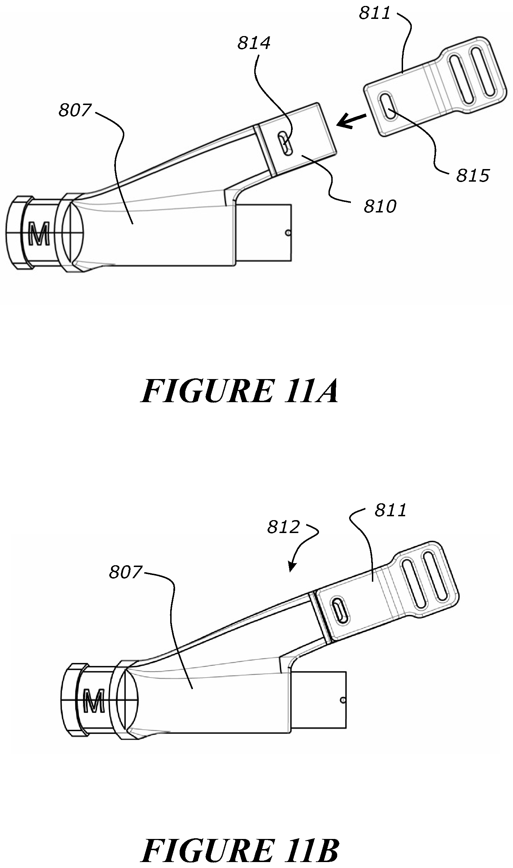

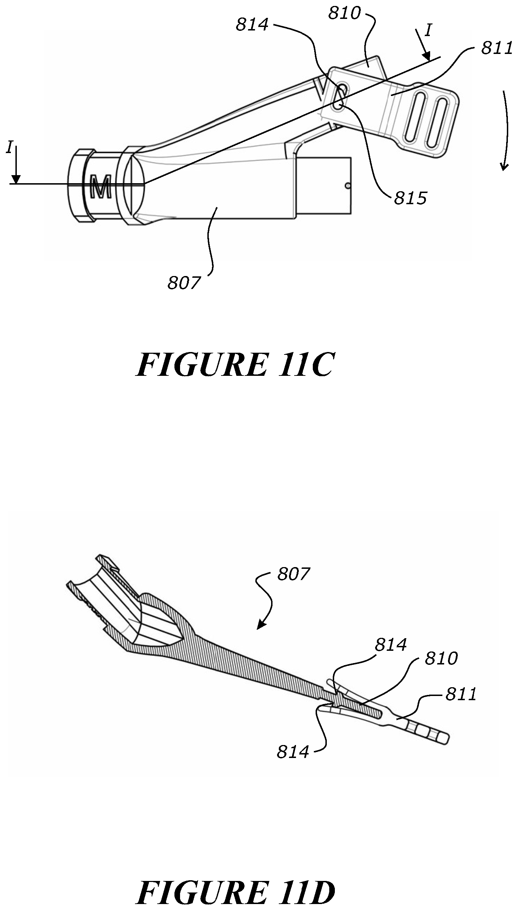

In the embodiment of Figures 10A to 10D, the female connector half 811 comprises two spaced apart tines or prongs 811A, 811B. The tines may form sides of the female part. The tines extend from a base 811C of the female connector half, such that a free end of each tine distal from the base may deflect laterally relative to the base. The way in which the connector halves 810, 811 are connected together and disconnected is illustrated in Figures 11A to 11D. As shown in Figure 11A, to connect the two halves 810, 811 together, the halves are pushed axially together represented by the arrow in Figure 11A, so that the male part is received between the tines of the female part. An axial direction is with respect to a direction that a headgear strap is to extend from the connector 812. When the halves are connected, the lateral projections 814 engage the apertures 815 in the tines, as shown in Figure 11B. Pulling the halves axially apart can take a significant amount of force, to allow the tines to deflect and spread apart to ride over the projections 814. Under normal use, any axial force applied to the connectors, for example from tightening the headgear, is less than the axial force required to axially separate the connector halves. However, to disengage the female part 811 from the male part 810, the female part may be rotated about an axis lateral to the connector parts 810, 811 or lateral to the axial direction, e.g. lateral to a headgear strap extending from the connector 812, as represented by the arrow in Figure 11C. The apertures 815 are shaped so that relative rotation between the male and female parts causes the projection to release from the aperture and deflect a said tine over the projection. For example, where the aperture is a slot and the corresponding projection is elongated to fit the slot, relative rotation between the parts causes the tines to spread as the projection interferes with an area of the tine around the slot. In Figure 11C and 11D, the female part is rotated so that the male and female parts are only partially engaged, with the tines deflected outwards to ride over the lateral projections, to disengage the female part from the male part. A force required to rotate the female part relative to the male part is reduced compared to a force required to separate the parts axially, because the protrusions 814 and corresponding apertures 815 are elongated with a major axis aligned perpendicular to the length direction of the head strap. With the major axis of the apertures and projections perpendicular to the strap an area over which an axial force may act is increased, creating a more secure attachment. The tines configuration of the female connector therefore achieves a connector that is secure in an axial direction on which forces in the headgear strap are aligned, yet allows for a relatively easy or reduced disconnection force by relative rotation between the male and female parts 810, 811. Relative rotation of the male and female parts does not occur in normal operation, other than when rotated by a person wishing to disconnect the headgear from the interface, and thus the described arrangement prevents or reduces accidental detachment. This configuration therefore allows for easily release of the cannula from the headgear which may assist in reducing the difficulty in removing the cannula from a user's face. Further, the female half 811 may be disengaged from the male half by a user singlehandedly, by simply twisting the female part relative to the male part. In another embodiment, the apertures and projections may be circular, with another feature such as complementary ramps or cam surfaces on the male and female parts arranged so that relative rotation between the parts cases the tines to spread apart to disengage each projection from the corresponding aperture.

To create an effective seal between a mask 300 and the cannula 700, 800 and the user's face, it may be desirable to have a section of the cannula side member or conduit that extends across the mask seal positioned on softer parts of the user's face. Correspondingly, it may be desirable to avoid hard parts of the patient's face. This may allow the user's face to deform around the cannula, to increase the chance of achieving an effective sealing of the mask seal over the cannula and with the user's face. Positioning the cannula on soft parts of the user's face may also help to improve patient comfort by not applying pressure on bony/hard parts of the user's face such as cheekbones. In general, it may also be comfortable to have other cannula components, such as head straps, lying on soft parts of the face.

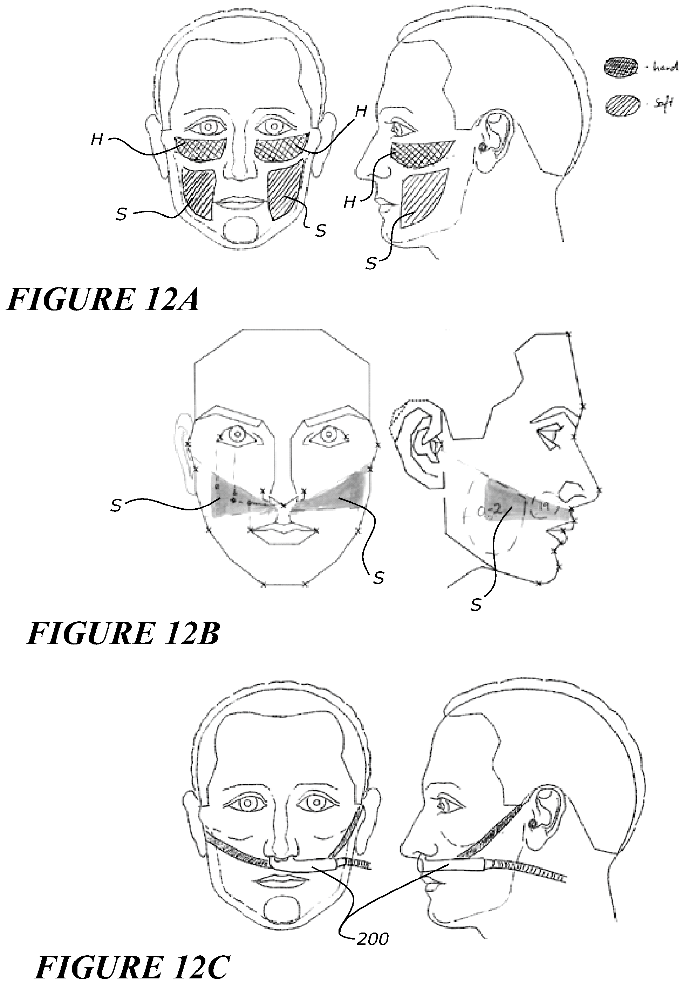

Figure 12A illustrates relatively soft areas S compared to relatively hard areas H of a user's face. Figure 12B further highlights the soft areas S of the user's face, which may be described as an area bounded by a line extending from the bottom of the users nose to a centre area of the user's ear, and a line running from a user's upper lip (bottom of the philtrum) approximately horizontally with the user in a standing position, e.g. above the user's lower jawbone. As shown in Figure 12C, preferably the cannula is positioned in the softer area of the user's face.

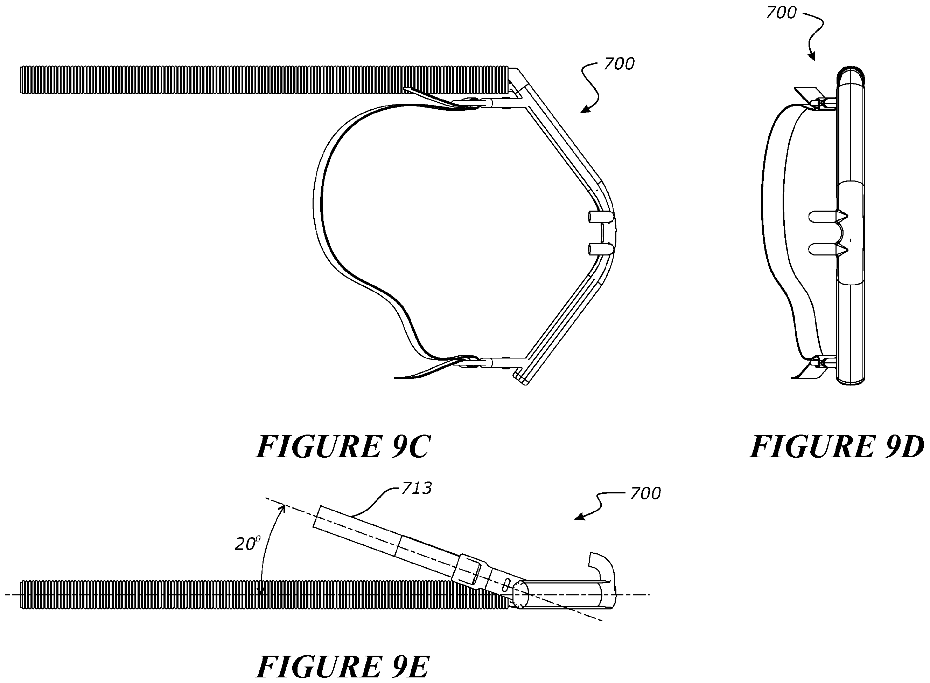

In order to position the cannula side members in a softer area of the user's face, in some embodiments the cannula is arranged so that the headgear strap extends from the side members at an angle to the side members when the cannula is viewed from a side of the cannula. For example, the angle may be 10 to 30degrees, or 15 to 25 degrees, or about 20degrees. In figures 9E and 10D the headgear strap 713 is shown to extend from the side members of the cannula at an angle of about 20 degrees. To position the headgear strap at the desired angle, the headgear connector is preferably oriented at the desired angle. For example, in Figure 9E, the female connector half 711 is integrally formed with the side member at an angle to the side member to orientate the strap correctly to the cannula. In Figure 10D, the male connector half 810 is integrally formed with the conduit connector 807 at an angle to the side member with the conduit connector attached to the side member to orientate the strap correctly to the cannula 800. In the embodiments of Figure 9A to 9H and 10A to 10D, the angle of the strap is positioned to locate the cannula approximately horizontally across the user's face (when the user is in a standing position) and with the headgear strap extending above the user's ears. As the head strap is directed over the patient's ears, the medical practitioner can apply a jaw thrust to the patient without obstruction. As shown in Figure 10D, the conduit connector 807 is arranged so that the conduit 112 extends from the cannula in line with the side member 703. Also, as shown in Figure 10C, in plan view, the angle of the conduit connector 807 and the headgear connector 812 relative to the side member 703, 704 are the same (or are similar) such that the conduit extends in line with the headgear strap in plan view. The arrangement of the conduit connector relative to the side member and headgear connector forces the inspiratory tube to lie alongside the patient's face when he or she is lying on his or her back. This configuration controls where the weight of the inspiratory tube lies and reduces the chance of the weight of the inspiratory tube kinking the collapsible portion of the cannula.

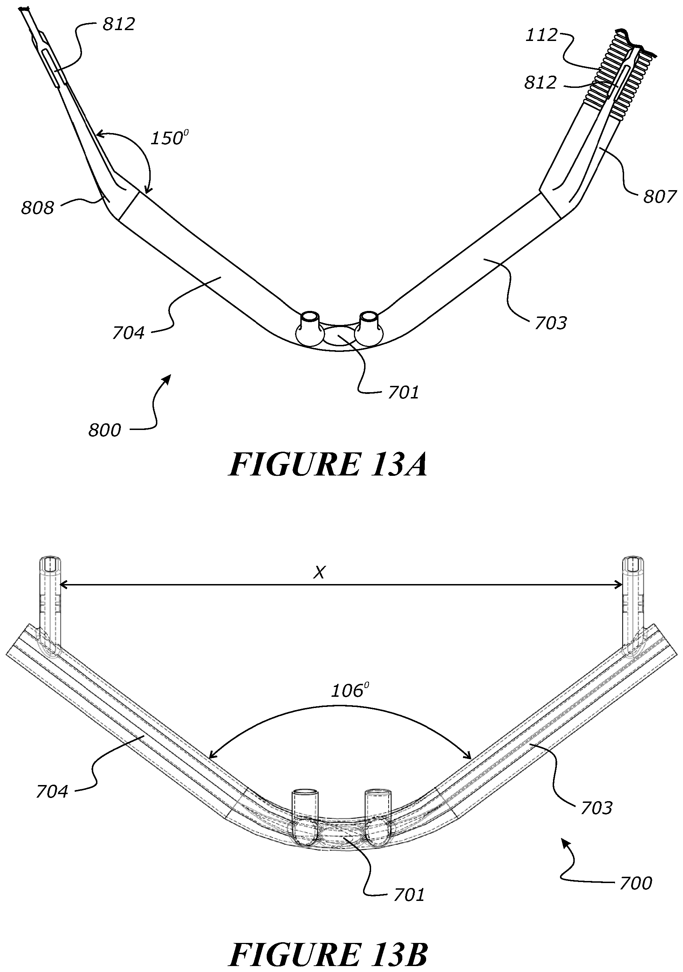

The cannulas 700, 800 described above are again illustrated in Figures 13A and 13B, with some indicated geometries (in figure 13B the conduit connector 707 and plug 708 are omitted). In the Figures, and including in Figures 13A and 13B, the cannula is illustrated in an unbent or un-deflected configuration, (e.g. a neutral or relaxed state). In some embodiments, in plan view, there is an obtuse angle between the side members. In some embodiments the angle between the side members is in the range of 100 to 130 degrees, or about 100 to 120 degrees, or about 100 to 110 degrees, or about 105 degrees. In the illustrated embodiments the angle is 106degrees. Such an angle allows the cannula to contour around the user's face without the manifold or side members kinking. In particular, this angle may be equal to or greater than the angle that is required to conform the cannula to a typical adult's face. In this case the cannula will lie on the face or may be slightly bent or deformed inwards to conform to the user's face as the headstrap is tightened. Bending the cannula inward (i.e. around the user's face) is much less likely to kink the flexible cannula than bending the cannula outward (i.e. away from the user's face, such as when a smaller angle is used). A large angle is also particularly useful to fit patients who receive this therapy who are likely to be high BMI and thus have larger head circumferences.

In some embodiments, in a plan view of the cannula, a distance between distal ends of the side arms, and/or between the pair of headgear connectors 712, 812 (distance X in Figure 13B), is about 100mm to 150mm, or about 110mm to 140mm, or about 110mm to 130mm or about 120mm. This distance is a sufficient width for a non-invasive ventilation mask to overlay the cannula with the edges of the seal falling within X (a non-invasive ventilation mask may typically have a width of approximately 100mm). In some embodiments, the relatively rigid connector 807 is positioned as close as possible to the prongs 702, while still allowing for the collapsible portion to be long enough for a mask to fit over (dimension X). Such an arrangement may improve comfort, as having the connector 807 as close as possible to the prongs (i.e. to the centre of the user's face) positions the connector away from the side of the patient's head so that with the patient lying on his or her side the connector may not be directly under the patient's face between the patient's head and pillow.

In some embodiments, in plan view there may be an obtuse angle between the headgear connector and the side arm. For example, as shown in Figure 13A, the cannula 800 comprises an angle of about 150degrees between the headgear connector 812 and the side member 704, 703. In some embodiments this angle may be in the range of 130 degrees to 170 degrees, or 140 degrees to 160 degrees, or 145 degrees to 155 degrees.

The above described geometries and arrangements may provide a number of benefits. Having the cannula horizontal across the face under the nose means the collapsible portion of the cannula intersects the facemask seal at a perpendicular angle and so the area over which the seal acts is as small as possible, reducing the required force to cause the collapsible portion to collapse. Further, a horizontal section encourages the cannula to lie in the soft sections of the face as the soft area just next to the nose is relatively small. The described arrangements may reduce the risk of the cannula and/or conduit 112 angling up towards the user's ears and lying across the user's hard cheekbone. The obtuse angle between the inspiratory tube and the cannula conduit described above removes sharp (sudden) corners in the gases flow path which can lead to turbulence and increase resistance to flow. The arrangement also aligns the conduit connector 807 close to the user's face to reduce leverage from the weight of conduit 112 which could cause kinking of the cannula. In some embodiments, the conduit connector 807 could be angled inwards towards the user's face for even closer positioning of the conduit to the face. For example, in Figure 10C the conduit connector is arranged approximately parallel to a sagittal plane of the user, whereas in some embodiments the connector 807 could be angled in inwards by 15 degrees relative to the sagittal plane. As described above, the headstrap connector 810 and the conduit connector 807 may be substantially in the same plane (vertically above) to reduce leverage from the weight of conduit which could cause kinking of the cannula. In some embodiments, the headgear connector 810 may be centred over the inspiratory tube connector 807 also to reduce leverage from weight of conduit which could cause kinking.

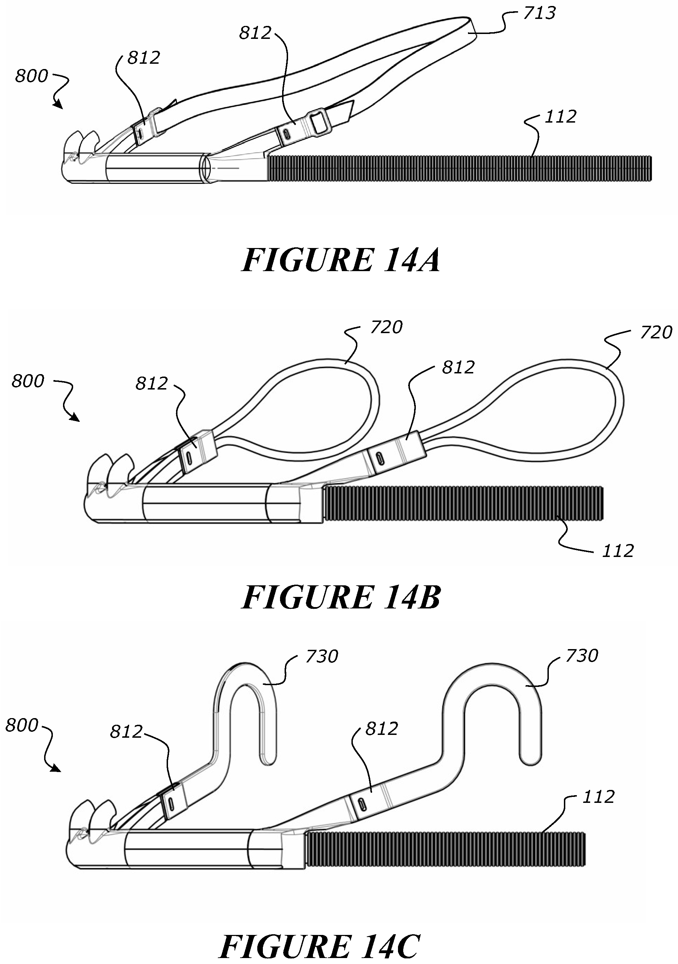

Figures 14A to 14C illustrate alternative headgear. In Figure 14A, the headgear comprises a strap 713. In Figure 14B, the headgear comprises a pair of elastic loops 720, each loop configured to loop around an ear of the user. In Figure 14C, the headgear comprises a pair of arms 730 similar in function the arms of a pair of spectacles. The arms 730 preferably extend down past the back of the ears to ensure secure retention of the cannula to the user's face. The described headgear may all have identical connectors and so be interchangeable as the user or patient desires. A headgear 720, 730 that does not go around the back of the patient's head may be particularly advantageous if the clinician does not wish to move the patient's head to apply or remove the cannula, or to prevent the patient' s hairnet being removed by the headstrap or the patient's hair becoming tangled in the headstrap. All headgear may be adjustable for different patient sizes (e.g. the elastic loops 720 may be pulled through the connector 812 to tighten).

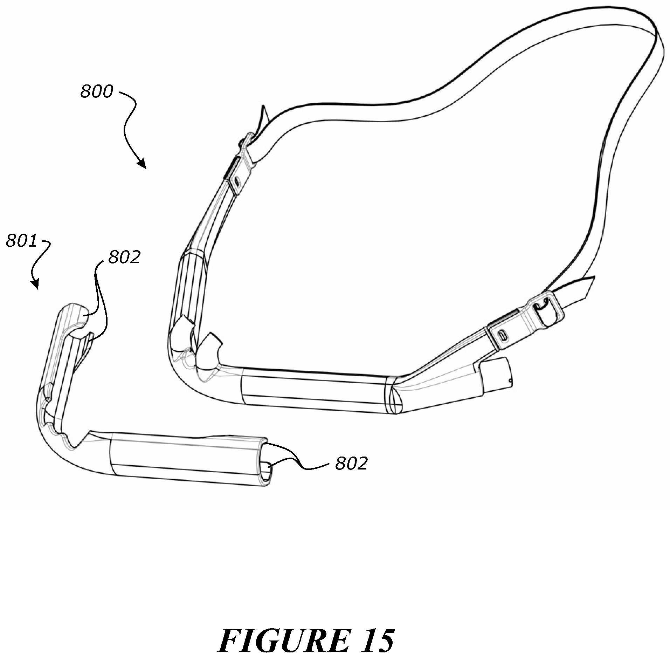

In some embodiments the cannula 700, 800 may be configured to be used without collapsing, by providing a shield or support member (e.g. a frame) to fit over and/or cover a side member, or both side members and the manifold. For example, as illustrated in Figure 15, a removable shield 801 may be provided to fit over the side members and manifold. The shield 801 may be formed from a relatively rigid material to support the cannula against collapse, for example against collapsing as a result of an external force applied to the cannula. The shield may comprise one or more (e.g. two) pairs of jaws 802 that are configured to grab around a portion of the cannula side arm or manifold or the plug or conduit connector to hold the shield to the cannula. In the illustrated embodiment the shield comprises a pair of jaws at each end of the shield, each pair of jaws configured to grip around a portion of a corresponding side member. The cannula may comprise a cannula body formed of a relatively flexible material, the cannula body comprising the manifold and at least one nasal prong or outlet, and a side member extending from each side of the manifold, as described earlier. The shield or frame is formed of a relatively rigid material (compared to the cannula body material). The shield attaches to the cannula body, to support the cannula body against collapse of the side arms.

In the above described cannula, in preferred embodiments, the cannula is 'slim' to reduce the size of the interface on the patient's face. Also, the relatively rigid headgear connector 712, 812 is slim to reduce bulk between the patient and a pillow supporting the patient's head when the patient is lying on his or her side to improve patient comfort.

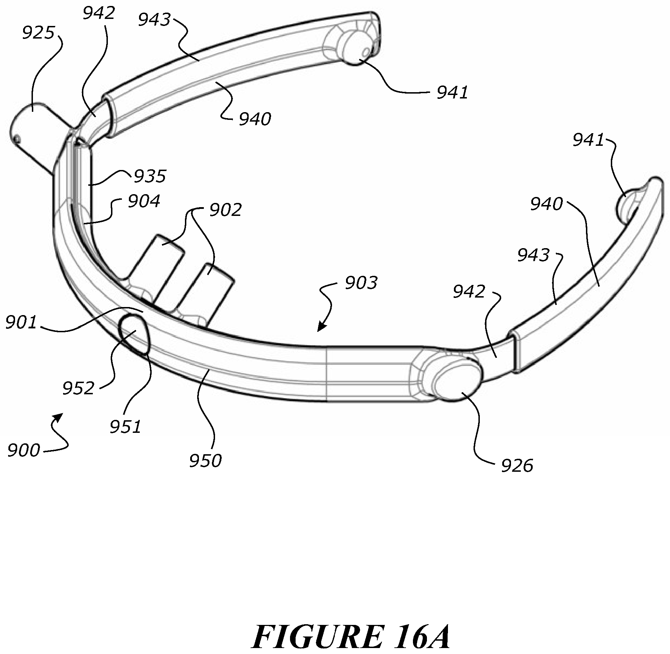

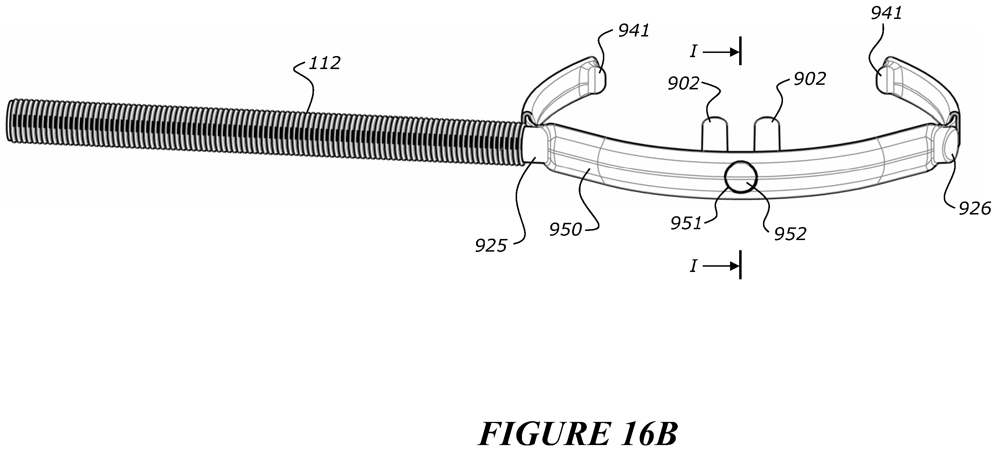

Figures 16A to 16E illustrate a further cannula embodiment 900 comprising a cannula body 935 formed of a relatively flexible material and a frame 950 formed of a relatively rigid material. The cannula body 935 comprises a manifold 901 and at least one nasal prong or outlet 902, and a side arm or member 903, 904 extending from each side of the manifold, as described earlier. A left side member 903 extends from a left side of the manifold and a right side member 904 extends from a right side of the manifold. Each side member comprises a lumen to provide a conduit for a flow of gases from an inlet of the cannula to the manifold. In some embodiments the conduit of each side arm comprises a collapsible portion as described in earlier embodiments. The frame 950 is attached to the cannula body 935 and may prevent collapse of the cannula body and conduit. In some embodiments, the frame 950 supports the cannula body 935 but allows for the body 935 to collapse when a force is applied to a front surface of the frame 950, to occlude the lumen. The frame 950 may be adapted to deform elastically so that a force applied to the front of the cannula frame 950 bends the frame and collapses a side member 903, 904 and lumen of the cannula body. Once the force is removed from the frame the frame 950 and cannula body 935 return to an un-collapsed configuration. Alternatively, in some embodiments, the frame 950 can act as a strut or support for the cannula body 935 to prevent collapsing of the cannula body. The frame can be used with the body in procedures where collapsing of the cannula is undesirable. The frame may be removably attachable to the body.

The cannula body 935 may comprise a gases inlet portion 924 to a lumen of the cannula body. The gases inlet portion 924 may be located at or towards an end of a side arm 903, 904. As illustrated, in some embodiments the cannula comprises a gases inlet portion 924 at each side arm 903, 904. The frame 950 may comprise an inspiratory tube connector 925 to attach an inspiratory tube 112 to the cannula. In some embodiments, the inspiratory tube connector 925 receives the gases inlet portion 924 of the cannula body. When gases are supplied to the cannula 900, a pressure of the gases forces the gases inlet portion 924 (e.g. inflates the inlet portion) against an inside of the inspiratory tube connector 925. An outer surface of the gases inlet portion 924 contacts an inner surface of the inspiratory tube connector 925 to create a seal to substantially prevent gases leaking.

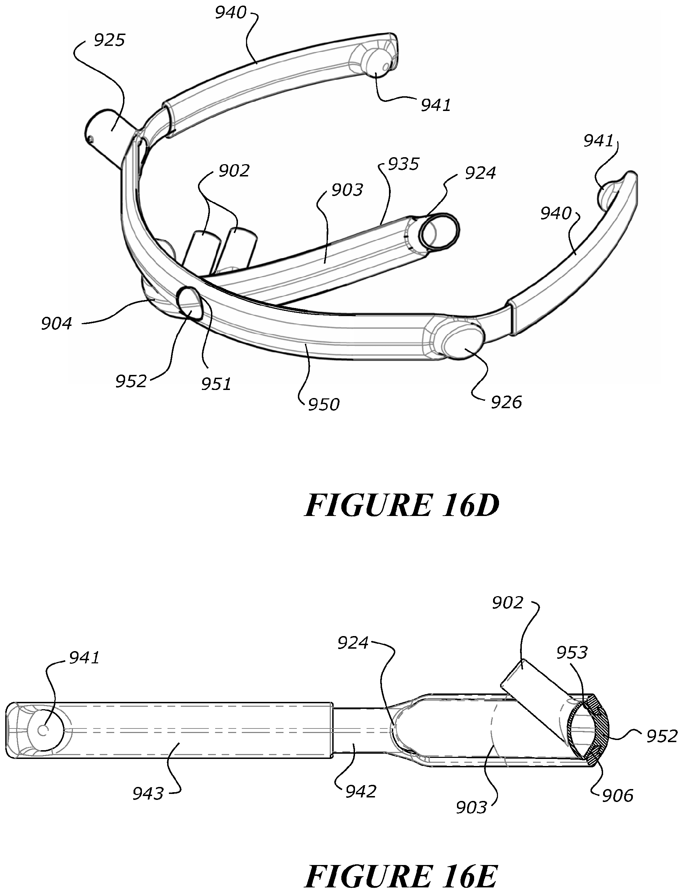

The cannula body 935 and frame 950 are movably attached together. For example, in some embodiments, the frame 950 may be pivotally (rotationally) attached to the cannula body 935, so that the frame may be rotated relative to the cannula body. In the illustrated embodiment, the cannula body may comprise a post 952 and the frame may comprise an aperture 951 or recess for receiving the post, the frame 950 rotating on the aperture or recess 951 about the post 952. The post may be formed of a relatively rigid material compared to the material generally forming the cannula body. The post may be overmoulded into the softer or resilient material of the cannula body. In an alternative configuration the cannula body 935 may comprise an aperture or recess to receive a post of the frame 950. In some embodiments, the cannula body comprises a gases inlet portion 924 at each side arm 903, 904 (e.g. towards or at an end of each side arm). The frame comprises an inspiratory tube connector and a blanked hollow projection or recess 926 (e.g. a blanked tubular projection). The inspiratory tube connector 925 is adapted to receive a said gases inlet portion 924 of the cannula body 935. When gases are supplied to the cannula 900, a pressure of the gases within the cannula forces the gases inlet portion 924 (e.g. inflates the inlet portion) against an inside of the tube connector 925. An outer surface of the gases inlet portion contacts an inner surface of the tube connector to create a seal to substantially prevent gases leaking from between the tube connector 925 and the cannula body 935. Similarly, the hollow projection 926 is adapted to receive a said gases inlet portion 924 of the cannula body 935. When gases are supplied to the cannula, a pressure of the gases within the cannula forces the gases inlet portion 924 (e.g. inflates the inlet portion) against an inside of the hollow projection 926. An outer surface of the gases inlet portion 924 contacts an inner surface of the hollow projection 926 to create a seal to substantially prevent gases leaking. Rotation of the cannula body 935 relative to the frame 950 selectively configures the cannula 900 between a left hand conduit inlet and a right hand conduit inlet. Figure 16D illustrates the frame rotated relative to the cannula body partway between the left and right hand configurations. Alternatively or additionally, the frame 950 may be removably attached to the body 935 and attachable to the body in two orientations, a first orientation providing a left hand inlet and a second orientation providing a right hand inlet. The cannula is configured as a left hand inlet cannula when the inlet portion 924 at the left hand side member 903 of the cannula body is received in the inspiratory tube connector 925 of the frame and the inlet portion 924 at the right hand side member 904 of the cannula body is received in the hollow projection 926 of the frame 950. The cannula is configured as a right hand inlet cannula when the inlet portion 924 at the right hand side member 904 of the cannula body is received in the inspiratory tube connector 925 of the frame and the inlet portion 924 at the left hand side member 903 of the cannula body is received in the hollow projection 926 of the frame 950 (as illustrated in Figure 16A).

In an alternative embodiment, the cannula body may include a pair of gases inlet portions 924, each located at or towards a distal end of each side arm, and the frame comprising a pair of inspiratory tube connectors 925 located at opposing ends or located at opposed sides of the frame to correspond with and receive one of the pair of gases entries 924 so that the cannula is configured for use as a dual entry cannula. A pair of inspiratory tubes may be attached to the pair of tube connectors 925 to supply gases to the cannula.

As illustrated in Figure 16E, the frame 950 may comprise a concave rear side 953 (the side that faces towards a user's face in use to receive the cannula body 935. A front 906 of the cannula body (facing away from the user's face in use may be complementarily convex to fit within the frame.

As illustrated by example in Figures 16A to 16E, in some embodiments a patient interface comprises a headgear, the headgear comprising a pair of ear plugs 941. Each ear plug 941 is adapted to fit within a user's ear, to retain the patient interface 900 in position on the user's face. The headgear may comprise a pair of arms 940, each ear plug provided on a respective said arm. One or both arms may be length adjustable. For example, one or both arms may comprise a telescopic configuration. One or both arms may comprise two or more parts that are arranged in a nested, telescoping configuration. In the illustrated embodiment, each arm comprises a first portion 942 slidingly received in a second portion 943, relative movement between the first and second portions achieving a variable length. This allows the interface to be easily sized for different patients. The arms may include a telescoping configuration with a ratchet assembly. The ratchet assembly may include a locking mechanism adapted to lock the telescoping arms one or more predetermined positions. For example the locking mechanism may comprise a moveable latch movably secured to one part 942, 943 of an arm to movably engage a series of grooves or apertures in another part 943, 942 of the arm. As shown, in some embodiments the patient interface is a nasal cannula. One of the first and second portions 942, 943 of each arm 940 may be integrally formed with a side of the cannula. In the illustrated embodiment, the first portion 942 of each arm 940 is integrally formed with the frame 950 that is attached to the cannula body 935. One of the first and second portions 942, 943 of the arms 940 may be more rigid than the other one of the first and second portions of the arms. For example, the first portion 942 received in the second portion 943 may be more rigid than the second portion 943. Preferably the ear plugs are formed of a soft material for comfort and grip in the ears of the user. For example the ear plugs may be formed of a silicone or other suitable plastics material or a foam material. In an alternative embodiment the arms 940 comprises first and second portions 942, 943 may be without ear plugs 941 to be received above and rest on top of the user's ears.

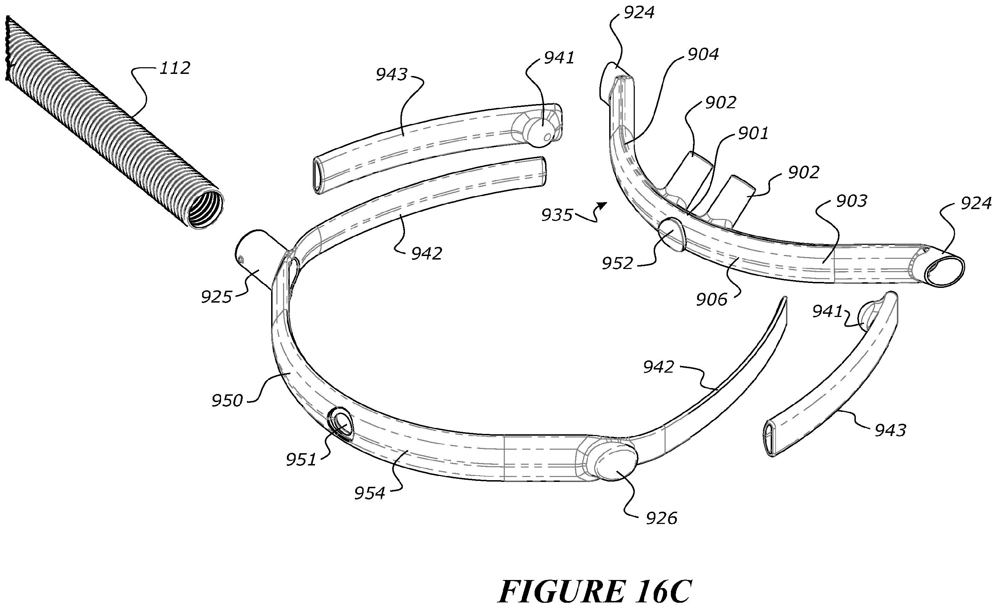

The cannula body 935 and frame 950 are generally curved to match the shape of a human face. A human face is substantially curved when moving from the nose to along the cheeks. The curved shape of the cannula body and the frame follow the general shape of the human face. The curved shape allows for a lower profile on the face and a better fit on the patients face. The cannula of Figures 16A to 16E may comprise the geometry features described above with reference to Figures 13A and 13B. The cannula body and frame have a curved shape. The cannula body and frame both have a convexly curved front surface (954 and 906 in Figure 16C). The convex curvature of the front surface of the cannula body and frame is a curvature from top to bottom of the body and frame (e.g. relative to a person wearing the cannula) as shown in the cross section of Figure 16E.

The cannula of Figures 16A to 16E is configured to rest or be positioned horizontal across the patient's face (e.g. with respect to the patient in a standing position). Such an arrangement ensures that when a face mask is applied over the top of the cannula the seal of the face mask is approximately perpendicular to the collapsible portion of the cannula, for a range of mask sizes and including larger masks. Also, the disclosed arrangement is useful in situations where a clinician does not want to lift the patient's head to apply or remove the cannula. The configuration of Figures 16A to 16E may be particularly easy to apply when the patient is lying down as no attachment is required behind the patient's ears and so the cannula may be applied straight onto the patient's face with the clinician standing directly over the patient.

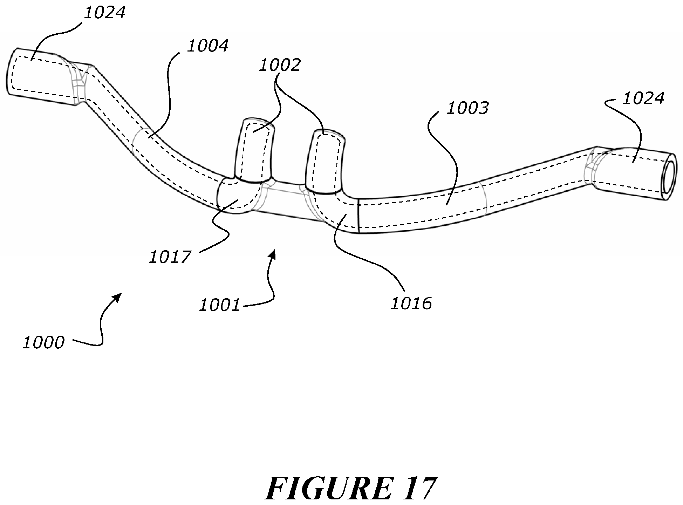

Figure 17 illustrates a further embodiment of a cannula 1000 with a left hand gases inlet 1024 and a right hand gases inlet 1024. The cannula comprises a manifold 1001 and a pair of nasal prongs 1002 or outlets extending from the manifold, and a side member 1003, 1004 extending from each side of the manifold 1001. The term 'manifold', as used in this specification and claims is intended to broadly mean, unless the context suggests otherwise, a member comprising at least two separate lumens, or a member comprising a single lumen with at least two inlets or at least two outlets. In the illustrated embodiment, the manifold 1001 comprises two separate manifold lumens 1016, 1017, each manifold lumen in fluid communication with a respective prong or outlet 1002, such that the prongs or outlets are pneumatically separate. The side members 1003, 1004 each comprise a lumen to provide a conduit for a flow of gases from an inlet 1024 to a corresponding manifold lumen 1016, 1017 and associated nasal prong or outlet 1002. A flow of gases is provided to the cannula via the two inlets 1024. In some embodiments the inlets 1024 may be formed from a rigid material which may provide an improved connection with a gases supply tube or conduit. A rigid element may help to achieve a sealed connection with a connector of a gases supply conduit. The rigid connector may be moulded or overmoulded or co-moulded to the cannula body.

Each side member 1003, 1004 is configured to be collapsible, and is independently collapsible of the other. In normal use, if one member 1003, 1004 was to be collapsed or its lumen inadvertently obstructed, the other side member 1003, 1004 would continue to provide a flow of gases to the user via the associated nasal prong or outlet 1002. In some embodiments, as illustrated, the cannula 1000 is preferably formed in a single integrally formed body of flexible material. In some embodiments, in addition to the single integrally formed cannula body, the cannula may comprise a rigid frame or shield, for example frame 801 as described above with reference to the embodiment of Figure 15, or frame 950 described with reference to Figures 16A to 16E. An interface that delivers flow from two sides may allow the size of the inspiration conduits 112 to be smaller than a single delivery conduit while still being able to deliver the same flow rate. This would be advantageous in reducing the size of the interface on the face and allowing the clinician greater access to the patient's face and airway.

In an alternative embodiment, the manifold includes a gases pathway that allows fluid communication between the lumens of the left and right side members 1003, 1004. The gases pathway in the manifold also allows fluid or gases communication between the lumen of the left side member 1003 and the right hand prong 1002 and the lumen of the right side member 1004 and the left hand prong 1002. In such an alternative embodiment, gases can be received by both prongs from either of the left and right inlets 1024 in case one prong is unexpectedly occluded. Such an arrangement may be advantageous because the inspiratory demand can be met and a sufficient flow rate be provided to an apnoeic patient to ensure there is enough O2 delivered and flushing of CO2 occurs.

In some embodiments the cannula 1000 is formed in a curved configuration to conform to the facial features of a user and may comprise geometry features as described above with reference to Figures 13A and 13B. The cannula 1000 may include appropriate headgear connectors, for example as described with reference to Figures 11A to 11D. The headgear connectors may be attached to the cannula at a location on the side members or may be connected to the inlet sections 1024. A force from headgear may pull the cannula against the user's face such that flexible body of the cannula deforms to conform to the face of the patient. With the cannula conforming to the patient's face the cannula achieves a low profile on the patient's face.

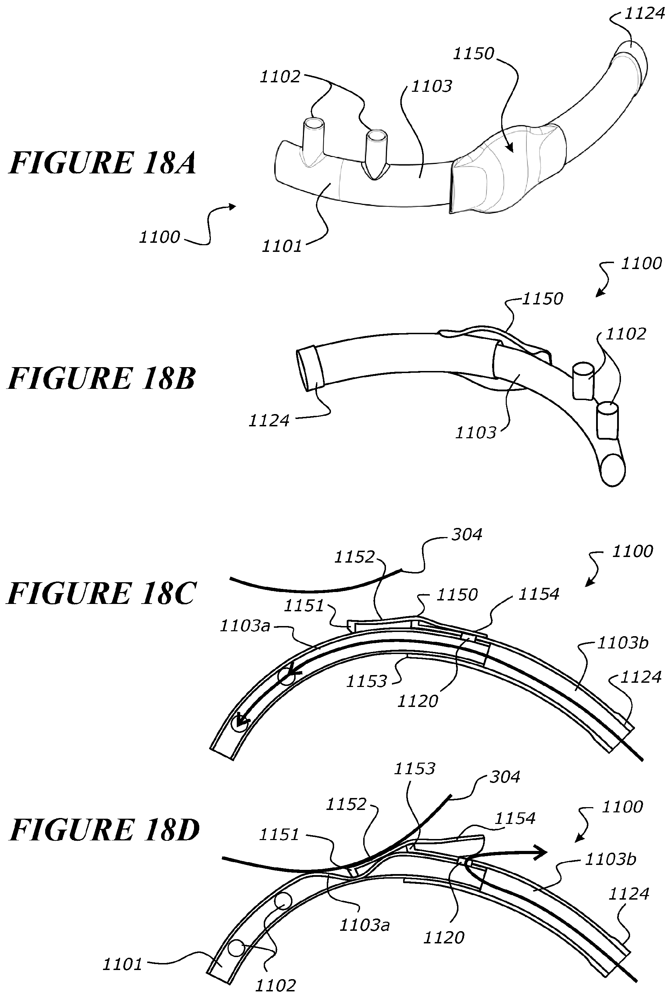

Figures 18A to 18D provide a further example of a nasal cannula 1100 comprising a collapsible conduit portion. The cannula comprises a side member 1103 that forms or comprises a collapsible conduit portion. The nasal cannula 1100 comprises a manifold portion 1101 from which nasal prongs 1102 extend. A side arm or member 1103 extends from one side of the manifold portion 1101. In some embodiments a side member 1103 extends from each side of the manifold portion as described in earlier embodiments. A collapsible conduit portion 1103 may be integrally formed in or with a side member of the cannula. In some embodiments, a side member 1103 is a conduit for transporting a flow of gases from a patient conduit (e.g. conduit 112 in Figure 1) to the manifold 1101, e.g. the cannula comprises a conduit 1103 extending from at least one side of the manifold 1101. Substantially a full length of the conduit 1103 may be configured to collapse, or a portion of the length of the conduit 1103 may be configured to collapse. In the illustrated embodiment, the cannula 1100 comprises a conduit 1103 with a collapsible portion 1103a and a non-collapsible portion 1103b. The collapsible portion 1103a may be formed from a relatively soft flexible material, such as a silicone material. The non-collapsible portion 1103b may be formed from a relatively hard or rigid material, compared to the material of the collapsible portion. In some embodiments the collapsible portion 1103a is located between the manifold 1101 and the non-collapsible portion 1103b of the conduit. In some embodiments the non-collapsible portion 1103a comprises an inlet 1124 to receive a flow of gases to the cannula 1100.

In some embodiments the cannula 1100 further comprises a mechanism to collapse the collapsible portion of the conduit. In some embodiments the mechanism is a rigid component (rigid relative to the collapsible conduit portion) attached to an outside of the cannula to move from a first configuration in which the collapsible portion is in the open configuration to a second configuration in which the component presses against an outside of the collapsible portion to pinch or flatten the collapsible portion into the closed configuration. In the illustrated embodiment the component is a lever 1150 that is actuated by an externally applied force, for example a force provided by the seal 304 of a face mask pressing against the lever 1150 as the face mask 304 is applied to a user's face over the top of the cannula 1100. In some embodiments the lever 1150 is pivotally supported by or attached to the non-collapsible portion 1103b of the conduit 1103. In use a user may press the lever (e.g. by pressing a face mask seal against the lever) to pivot the lever 1150 to press the lever against the collapsible portion 1103a to collapse the collapsible portion 1103a and occlude or partially occlude the lumen of the collapsible portion 1103a. The lever 1150 is pivotable between a first configuration as shown in Figure 18C in which the collapsible portion is open, and a second configuration as shown in Figure 18D in which the collapsible portion is closed. In the second configuration the lever presses against an outside of the collapsible portion 1103a of the conduit to pinch or flatten the conduit. In some embodiments, the lever may be pivotally attached to the manifold portion 1150, should the manifold have sufficient rigidity to pivotally support the lever to pivot between the first and second configurations. When a force is removed from the lever 1150, gases flow through the collapsible portion and force the lever to return to the first configuration. The lever may comprise a projection such as a rim 1151 that contacts and pinches the conduit in the closed configuration. The projection or rim 1151 is preferably wider than the collapsible portion so that the rim applies across the full width of the conduit.

In some embodiments, the lever 1150 comprises a first arm 1152 extending from a first side of a pivot 1153 and a second arm 1154 extending from an opposite second side of the pivot 1153. When in the first configuration (figure 18C) the lever 1150 is pivoted about the pivot 1153 so that the first arm does not pinch or flatten the conduit and the second arm 1154 covers or closes or obscures a vent aperture 1120 in the conduit 1103. In the second configuration (figure 18D) the lever 1150 is pivoted so that the first arm 1152 pinches or flattens the conduit 1103 and the second arm 1154 lifts away from the vent aperture 1120 to allow gases in the conduit upstream of the collapsible portion 1103a to vent to atmosphere. In such an embodiment the lever 1150 operates in a seesaw fashion to, in the first configuration, occlude the collapsible portion 1103a and vent the conduit upstream, and in the second configuration, to allow the collapsible portion 1103b to open and close the vent aperture 1120.

In some embodiments the cannula 1100 is formed in a curved configuration to conform to the facial features of a user. The cannula 1100 is illustrated with a single side member 1103 however in some embodiments may comprise a left hand side member and a right hand side member as described in earlier embodiments, and may comprise geometry features as described above with reference to Figures 13A and 13B. Further, the cannula 1100 may include appropriate headgear connectors, for example as described with reference to Figures 11A to 11D. The headgear connectors may be attached to the cannula at a location on the side members. A force from headgear may pull the cannula against the user's face such that flexible body of the cannula deforms to conform to the face of the patient. With the cannula conforming to the patient's face the cannula achieves a low profile on the patient's face.

In some embodiments, a conduit, e.g. inspiratory conduit 112 may comprise a collapsible portion and a lever 1150 as described above.

In some embodiments, the patient interface or a conduit may comprise a collapsible portion and a rigid shield or member attached to the outside of the collapsible portion. The member is rigid relative to the conduit portion and therefore is adapted to distribute an external force applied to the member over a predetermined collapsible area of the collapsible portion. The rigid member assists to ensure the collapsible portion is pinched off adequately to substantially occlude the conduit and avoid creasing or folding of the conduit that might otherwise provide a leak path through the collapsed portion of the conduit.

Aspects of the present invention are described above with reference to nasal cannulas. However, aspects of the present invention may be applied in other interfaces, such as for example an oral interface. An example oral interface 1200 is illustrated in Figure 19, general features of which are described in US patent 9,155,855. The interface 1200 comprises a vestibular shield 1221, an outer flap 1225, and a connector 1235 that connects the outer flap to the vestibular shield. In use the vestibular shield 1221 is received in the user's mouth and sits inside the user's lips, and the outer flap 1225 sits outside the user's mouth about the outside of the user's lips. A seal is formed by pressure caused by the outer flap 1225 on the outside of the user's lips and an opposing force of the vestibular shield 1221 on the inside of the user's lips. The interface 1200 provides a flow of gases to the user through the connector and via outlets 1223, 1224 from the connector. The outlets 1223, 1224 may be received in outlets 1232, 1233 of the shield 1221. In the illustrated embodiment a manifold 1201 is provided to attach to the connector. A side member or conduit comprising a lumen extends from each side of the manifold, a left side member 1203 and a right side member 1204 (with respect to a user). An inspiratory conduit (e.g. conduit 112) is connected in use to at least one of the side members 1203, 1204 to provide a flow of gases via the side member or members and the manifold 1201 to the connector 1235 via an inlet 1234 of the connector and to the user's airway via the connector outlets 1223, 1224. In some embodiments the interface may comprises a body comprising the manifold 1201 and outlet 1202 from the manifold (e.g. to be connected with connector inlet 1234) and the side members 1203, 1204. In some embodiments, the body may also be integrally formed with the outer flap 1225 or connector 1235 or both. In some embodiments, the outer flap 1225, connector 1235 and shield 1221 may be integrally formed, and may be integrally formed together with the manifold 1201 and side members 1203, 1204. In some embodiments, an oral interface may be without a manifold and may comprise an elbow connector to configure the interface as a single inspiratory conduit embodiment. The side members 1203, 1204 each comprise a collapsible conduit portion, as described previously with reference to cannula embodiments. In some embodiments the side members 1203, 1204 wrap around and/or are closely adjacent to the outer flap 1225. In some embodiments, the side members or conduits are integrally formed with the outer flap and/or may be positioned between a patient face side of the outer flap that contacts a user's face or lips and an outer (opposite) side of the outer flap. The oral interface 1200 may further comprise any one or more features of cannula embodiments described above in relation to collapsible conduit portions and/or configurability, e.g. as a dual or single inlet interface.

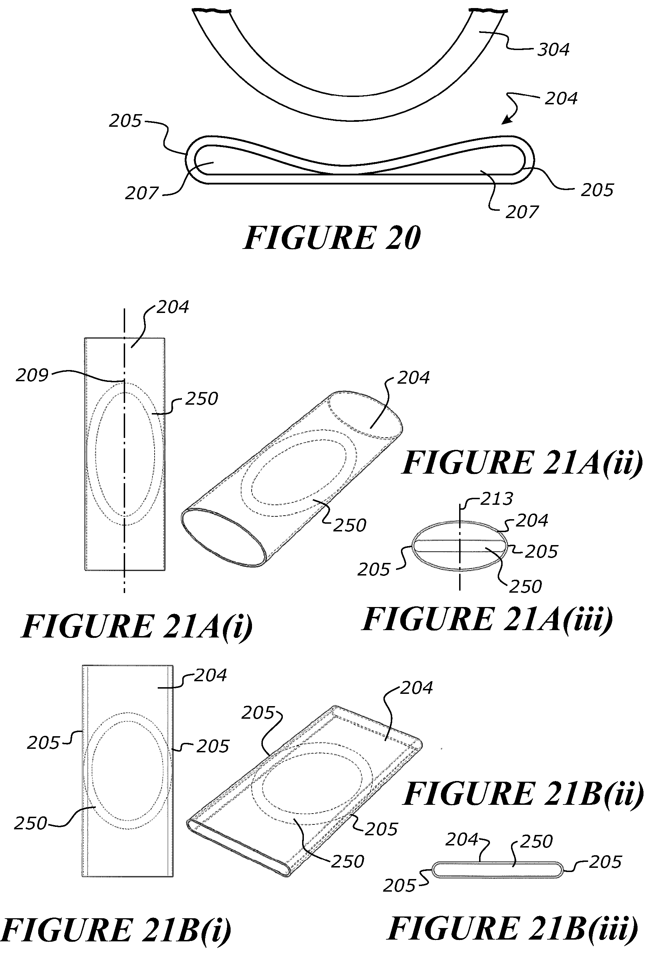

With reference to Figure 20, when a conduit portion 204 is collapsed to a flattened or collapsed configuration, the flattened conduit has a folded edge 205 at each side of the collapsed portion. In some embodiments, when in the collapsed configuration, the inside cross section of the conduit may not completely pinch shut adjacent one or both folded edges 205 of the cross section, presenting a gap or leak path 207 adjacent the folded edge of the conduit 204. In some embodiments, an insert may be provided to the inside of the collapsible portion of the conduit 204 to reduce or prevent a leak path 207 at a folded edge 205 of a conduit when in a collapsed configuration. The leakage gases flow through the collapsed portion of the conduit 204 when in a collapsed condition. The leakage gases flow may be about 15L/min or less; or about 10 L/min or less, or about 10L/min. The leakage gases flow may be about 5L/min to about 10L/min. The leakage gases flow may be measured by a sensor for example located in the patient interface, inspiratory conduit or any upstream flow generator.

For example, as shown in Figures 21A(i) to 21B(iii), a flexible insert 250 may be provided to the inside of the collapsible section 204. The flexible insert 250 extends across the lumen of the conduit 204 and contacts opposite sides of the inside of the conduit, the sides corresponding with the folded edges 205 of the conduit when in the collapsed configuration. The insert 250 is preferably biased to contact against opposite sides of the inside of the conduit corresponding with the folded edges 205 of the conduit when in the collapsed configuration. The height of the insert 250 is less than the height of the lumen of the conduit so that the insert 250 does not completely block the lumen when the conduit is in the open configuration, as shown in Figures 21A(i) to 21A(iii). In Figure 21A(iii) the insert 250 is shown as being located approximately centrally. However, in some embodiments the insert may be located within the lumen of the conduit at a location other than centrally, e.g. against a side of the conduit 204 that contacts a patients face in use.

When the conduit is collapsed as shown in Figures 21B(i) to 21B(iii), for example by the seal 304 of a mask acting on the outer surface of the conduit 204, the inside cross section of the conduit increases in a direction across the patient's face and decreases in a direction lateral to the user's face, i.e. the height dimension of the conduit. As the cross section of the conduit increases across the user's face a bias of the insert 250 causes the insert to expand in the direction across the user's face, to maintain contact with opposite sides 205 of the conduit 204. The insert maintains contact with the inside of the conduit even when in the collapsed state. In the collapsed configuration, as shown in Figure 21B(iii), the cross section of the conduit 204 is collapsed so that a shape of the inside of the conduit corresponds with the insert 250 at least in an end view. The insert 250 preferably has a rounded end profile located at the side wall or folded edge 205 of the conduit so that as the conduit 204 collapses onto the insert 250 the inside of the conduit wraps around or conforms to the insert 250 so that the insert 250 fills the lumen adjacent the folded edge 205 of the conduit. In the collapsed configuration the conduit 204 is collapsed onto or around the insert 250 so that the insert substantially blocks the conduit, as shown in Figure 21B(iii).

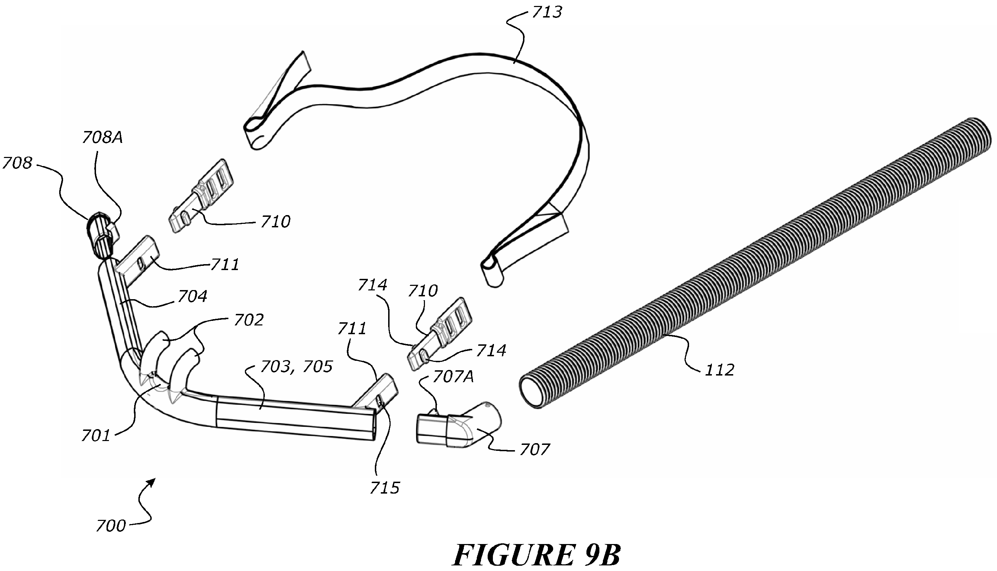

In some embodiments, and as shown in Figures 21A(i) to 21B(iii) the insert 250 is an annular member or ring or 'o-ring', for example an elastomeric o-ring. The o-ring is located with a central axis 213 (e.g. an axis through the centre of the outside diameter of the o-ring) lateral to the longitudinal axis 209 of the conduit. The o-ring preferably has an outside diameter (a lateral dimension of the insert) that is larger than the inside dimension of the collapsed conduit 204 in a direction across the user's face so that the o-ring maintains a bias against the folded edges 205 of the conduit when in the collapsed configuration. For example as shown in Figure 21B(i) a circular o-ring insert 250 is shown slightly elastically deformed into an oval shape being squeezed between the folded edges of the collapsed conduit 204. The insert 250 is elastically deflected to fit within the conduit 204. Preferably the cross section of the o-ring (i.e. the outside diameter minus the inside diameter of the o-ring) is equal to or greater than the height of a gap that would form adjacent the folded edge of the conduit in a collapsed configuration without the insert present (e.g. gap 207 on Figure 9). In this way the o-ring fills any gap that might form adjacent the folded edge 205. Preferably the conduit provides bias against the bias of the insert so that when a force is removed from the conduit in the collapsed configuration the conduit returns to the open or un-collapsed configuration against the bias of the insert acting between the opposite sides of the conduit. The insert may 'float' in or being located in the conduit without being attached to the walls of the conduit, e.g. an o-ring insert may be placed or inserted into the conduit. Alternatively, the insert may be attached to opposite sides 205 of the conduit. The insert 250 may be attached to opposite sides of the conduit and biased to return the conduit from the collapsed configuration to the un-collapsed or open configuration. The insert 250 may be circular or otherwise shaped, e.g. oval, or may be polygonal, such as hexagonal or octagonal. 'Circular' should be interpreted broadly to mean substantially circular including a polygon having more than 8 sides.

Preferably the insert 250 is sized so that the mask seal 304 completely covers over the insert in a longitudinal direction 209 of the conduit.

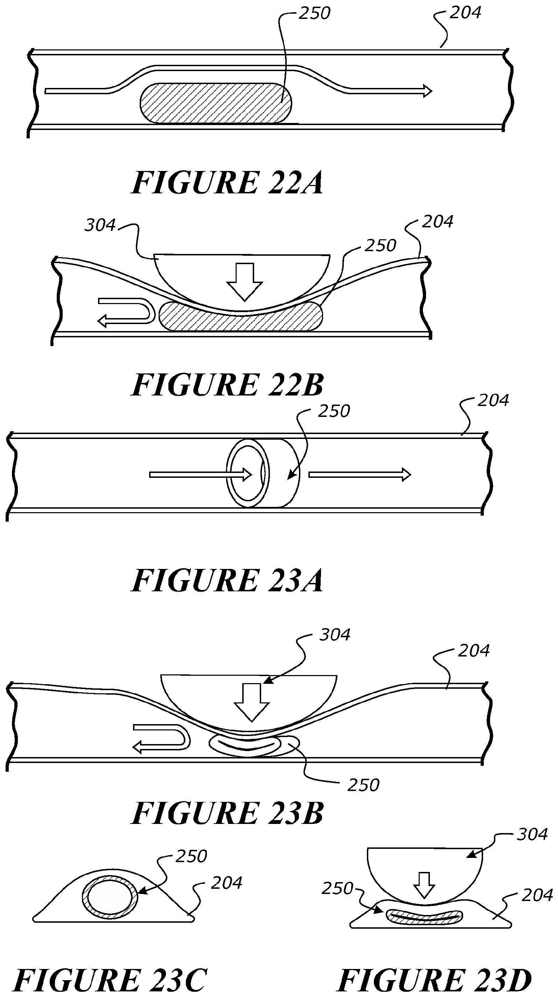

Figures 22A and 22B illustrate another flexible insert 250. In Figures 22A and B the insert 250 is a very soft material such as a foam material, memory foam material, a fluid filled member or bag and/or comprises a gel material. The height of the insert 250 is less than the height of the inside of the conduit 204 so that gases can flow over the insert 250 in the open configuration. In the collapsed configuration the conduit 204 is collapsed onto the insert 250, and the insert is deformed to spread laterally across the conduit between the folded edges 205 of the conduit, and also to spread longitudinally along the conduit. The material, e.g. a gel, is so soft that it flows to fill any gaps between walls of the conduit in the collapsed state. In some embodiments the insert 250 may comprise a bag formed from a thin resilient material such as a silicone rubber or other rubber material and filled with a fluid or gel or paste or jelly. The insert 250 may have a modulus of elasticity or comprise a material with a modulus of elasticity significantly less than the modulus of elasticity of the material forming the conduit 204.

In the o-ring embodiment of Figures 21A(i) to 21B(iii), the mask seal preferably covers over the whole of the o-ring in a longitudinal direction of the conduit. In the embodiment of Figures 22A and 22B, the mask seal may cover over a portion of the insert in the longitudinal direction so that the insert 'squashes' longitudinally along the conduit beyond the mask seal.

Figures 23A to 23D illustrate a further example of a flexible insert 250. In Figures 23A to 23D, the flexible insert 250 comprises an annular member with a central axis aligned with a longitudinal axis of the conduit. The annular member 250 is arranged around an inner surface of the conduit. The annular member or ring 250 may have a modulus of elasticity or comprise a resilient material with modulus of elasticity significantly less than the modulus of elasticity of the conduit. In the open configuration shown in Figure 23A the gases flow passes through the ring, and in the collapsed configuration shown in Figure 23B, the ring is compressed to a flat configuration together with the conduit to block the flow path of the conduit 204. As the ring preferably has a modulus of elasticity significantly less than the conduit the ring flows or otherwise deforms to fill any gaps that would otherwise be present between sides of the collapsed conduit. With reference to the end views presented in Figures 23C and 23D, in some embodiments the conduit may have a flat side to contact a user's face.

In the insert 250 embodiments described, the conduit 204 may comprise internal features to locate and/or retain the insert in a position within the conduit. For example the conduit may comprise ribs to locate the insert, or a depression or recess in the wall of the tube to receive the insert.

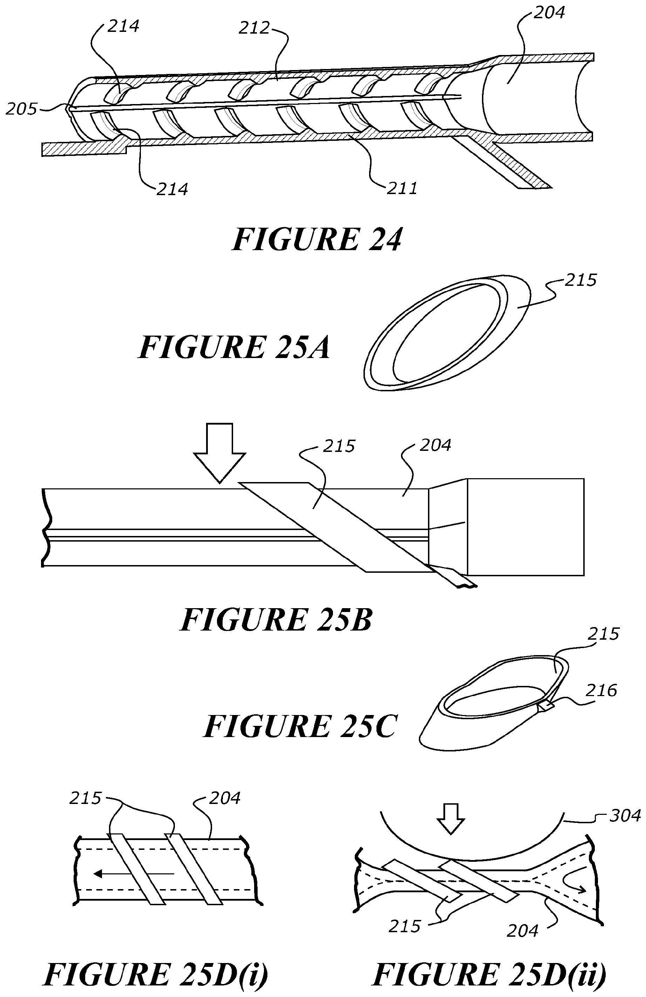

With reference to Figure 24, in some embodiments the conduit comprises protrusions on the inner wall of the conduit. The conduit comprises a first side 211 for positioning against a user's face and a second side 212 opposite the first side that faces away from the user's face. The first and second sides are joined by first and second fold points 205. In a partially closed or closed configuration the second side is moved towards or against the first side with the collapsible conduit 204 folding at the first and second fold points. Each of the first and second sides have a projection or rib 214 extending between the fold points. The projection or rib on the first side is longitudinally offset from the projection or rib on the second side with respect to the longitudinal axis of the conduit. In the collapsed configuration, with the first side and second side moved together, the projections 214 are arranged adjacent one another. The adjacent projections or ribs may laterally abut or contact one another, to assist with sealing or blocking flow through the conduit. The adjacently arranged projections may present a tortuous flow path to create a flow restriction. Each rib 214 may extend linearly across the conduit, or may comprise a curved shape, or may form a chevron shape or other suitable shape.

In some embodiments, the conduit 204 comprises a member to pinch or kink the collapsible portion of the conduit to block flow through the conduit. For example, as shown in Figure 25B, in some embodiments the conduit 204 comprises or is provided with a ring or band 215 extending around the conduit. The band, shown in Figure 25A, may extend around an inner diameter or surface of the conduit, or an outer surface, or may be embedded in a wall of the conduit. The ring 215 is arranged around the conduit at an angle to the longitudinal axis of the conduit 204. When a force is applied to the ring 215, the ring is tilted or rotates about an axis or axes lateral to the longitudinal axis of the conduit and acts to pinch or kink the conduit into a collapsed configuration. In Figure 14B the ring is shown at an angle of approximately 45 degrees to the longitudinal axis of the conduit. The ring is angled from a second side of the conduit to an opposite first side of the conduit, wherein the second side is the side that faces away from a user's face in use. In Figure 14C, the ring has protrusions 216 at opposite sides of the ring to fill gaps at folded edges of the conduit when the conduit is in the collapsed configuration.

As shown in Figures 25D(i) and 25D(ii), in some embodiments the conduit comprises two rings 215 as described above located side by side, each ring extending around the conduit 204. As described above, when an external force is applied to the rings or conduit adjacent the rings, the rings tilt or pivot to pinch or kink the conduit closed. With two rings, as the conduit is moved to the collapsed configuration one ring lies over the other ring, creating a pinch region between the two rings to pinch or kink the conduit in the collapsed configuration.

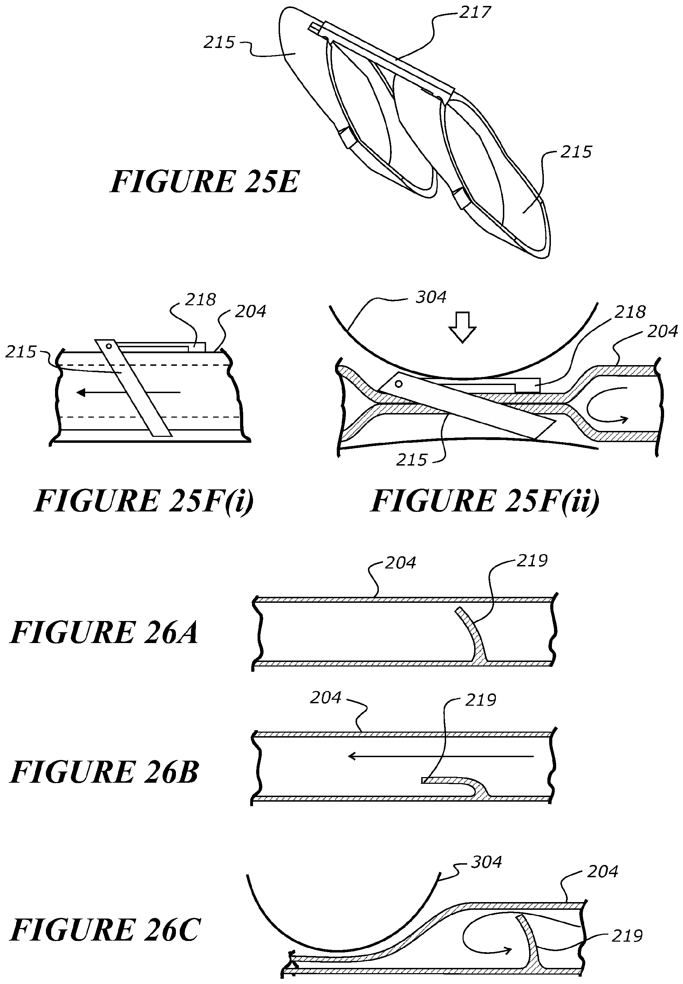

With reference to Figure 25E, in some embodiments the two rings 215 are connected together by a connecting member 217. Each ring is connected to the connected member by a pivot or hinge to allow the two rings to tilt or rotate relative to the longitudinal axis of the conduit as described above. The connecting member 217 helps to ensure the two rings 215 are spaced apart at a set distance so that a pinch region of the conduit 204 between the rings is located correctly.

With reference to Figures 25F(i) and 25F(ii), in some embodiments the conduit comprises a ring 214 and a lever 218 or member pivotally attached to the ring. The ring 215 extends around the conduit and with the conduit in the open configuration the lever is pivoted from the ring to lie along an outer surface of the conduit 204. When an external force is provided to the conduit or to the lever or the ring, the ring tilts or rotates relative to the longitudinal axis as described above, and also pivots relative to the lever. As the conduit collapses, the lever comes to lie over the ring and the conduit is pinched or kinked between the ring and the lever 218, as shown in Figure 25F(ii).

In some embodiments the conduit 204 comprises an internal flap 219 arranged to move between a closed position in which the flap extends across the inside of the conduit 204 to block flow along the conduit, and an open position in which the flap bends or otherwise deflects away from the closed position to allow flow along the conduit (Figure 26B). Figure 26A shows a no flow condition, where the flap is in an undeformed or undeflected position. Flow along the conduit acts on a front side (with respect to the flow direction) of the flap 219 and causes the flap to bend away from the closed or undeflected position to the open position. When the conduit is collapsed to a closed configuration, as shown in Figure 26C, the flap 219 returns to the closed or undeflected position since the flow along the conduit is blocked or is reduced. Flow along the conduit is blocked at the collapsed portion of the conduit and recirculates to act on a back side of the flap 219 causing the flap to move to and/or be held in the closed position and block the conduit. Thus the flap acts as a secondary valve or secondary mechanism to the collapsed conduit to block flow along the conduit.

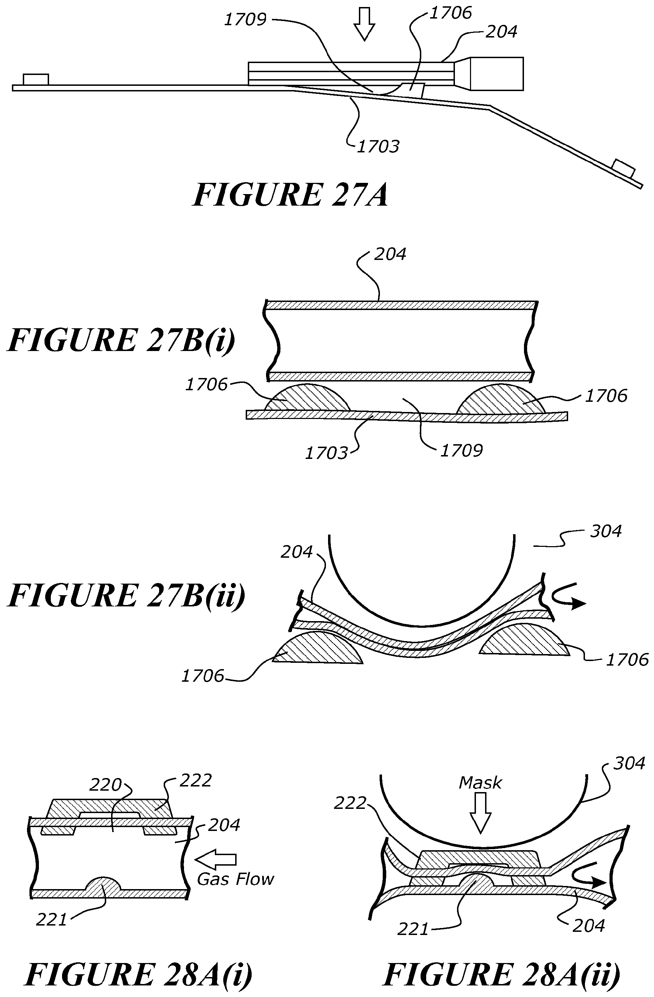

In some embodiments, a patient interface or a head strap or headgear for a patient interface is provided with a profile over which a conduit lies. The profile is adapted to cause the conduit 204 to kink or bend when a force is applied to the conduit. By causing a kinking or bending of the conduit the profile assists to collapse the conduit and create a seal against gases flow along the conduit. For example, in Figure 27A a head strap or side arm 1703 of a patient interface comprises a projection 1706. A collapsible conduit portion 204 is placed against the strap or side arm 1703 and the projection 1706, such that a gap 1709 is present between the conduit 204 and the side arm or strap 1703. When a force is applied to the conduit in the direction of the arrow, the conduit bends into the gap. The conduit may kink in a location between the position the force is applied and the projection. The projection helps to accentuate kinking by supporting the conduit with a gap between the conduit and the patient interface or head strap.

In some embodiments the profile comprises two projections 1706, as shown in Figures 27B(i) and 27B(ii). Figure 27B(i) shows the conduit 204 in an open configuration, with the conduit supported by the two spaced apart projections 1706 and with a gap or space 1709 between the conduit and the patient interface 1703 or headgear or other supporting component associated with the projections. Figure 27B(ii) shows a mask seal 304 applied to the conduit 204 to move the conduit to the collapsed configuration. The conduit bends into the space between the two projections. The two projections accentuate or increase the bending of the conduit to assist in collapsing the conduit to block flow through the conduit.

In some embodiments the conduit 204 may have a profile on a side of the conduit to bear against a user's face or against a patient conduit or head strap or headgear. For example, the conduit may comprise a projection or two spaced apart projections to create a gap between the conduit and the patient interface or strap into which the conduit can bend, as described above.

In some embodiments the conduit comprises complementary internal profiles to assist with blocking flow when the conduit is in a collapsed configuration. For example, as shown in Figures 28A(i) and 28A(ii), the conduit 204 may comprise a recess 220 in one side of the internal surface of the conduit and a complementary projection 221 in an opposite side of the internal surface of the conduit. In the collapsed configuration the projection is received in the recess. The complementary profiles create a tortuous path to increase the resistance to flow through the collapsed portion of the conduit. The recess may be created by two spaced apart protrusions. In the illustrated embodiment, the conduit comprises a female part 222 inserted into a side of the conduit to receive a corresponding projection 221 in an opposite side of the conduit. As shown in Figure 28A(ii), the mask seal 304 may act against the female part 222 to collapse the conduit 204.

In some embodiments, for example as shown in Figures 6A to 8B, the collapsible conduit is formed (e.g. moulded) in the open configuration, such that the resiliency of the material forming the conduit biases the conduit to the open configuration. An external force must be applied in order to collapse the conduit from the open configuration to the closed or collapsed configuration. Preferably a small force is required to collapse the conduit such that the leak through the conduit is not physiologically relevant. This force should be commensurate with the force required to create a seal between the mask and (n adult face, preferably the face of a healthy adult with no facial hair. Preferably, this force would be no more than the force typically applied by an experienced anaesthetist or trained specialist. The conduit should seal such that leak is not physiologically relevant when a mask is pressed to the face with a force equivalent to between 5cmH₂O and 200cmH₂O of pressure within the cuff of the mask. Preferably the required seal would be achieved, with mask cuff pressure of between 25cmH₂O and 85cmH₂O. Ideally the conduit would seal under a mask cuff pressure of between 30cmH₂O and 35cmH₂O.

In some embodiments, a collapsible portion of a conduit is formed in a collapsed configuration. For example, a conduit may be moulded in a collapsed configuration. The conduit may be pressurised by an air flow to expand from the collapsed configuration to an open or non-collapsed configuration. For example, a conduit may be moulded to have a cross section as shown in Figure 6B. In order to move to an open configuration the inside of the conduit is pressurised by a flow of gases. To block the flow of gases an external force may be provided to an outer surface of the conduit to return the conduit to the collapsed configuration. In such an embodiment, the resiliency of the material forming the conduit biases the conduit to the collapsed configuration.

In some embodiments, a conduit portion formed in a collapsed configuration may comprise internal features or components to separate sides of the conduit when in the collapsed configuration and without a force applied to the conduit. Thus such a conduit may have an intermediate position, intermediate the open and collapsed configurations, the internal feature or features or component or components holding the conduit in the intermediate position. The conduit is moved from the intermediate position to the open configuration by a flow of gases pressurising the conduit, and from the intermediate position to the collapsed configuration by an external force overcoming the pressure of the flow of gases within the conduit. The internal feature or features may be a rib or other projection located at one or both sides of a collapsible portion of the conduit, i.e. a rib at an upstream side or a rib at an upstream side and a rib at a downstream side of the collapsible portion. The rib may be longitudinal or lateral to the flow direction. In some embodiments, the conduit may comprise a component at an upstream side, or a component at an upstream side and a downstream side, to hold the collapsible portion in an intermediate or slightly open (almost fully collapsed) position. The component or components may be formed of a material more rigid than material of the wall of the conduit. The component or components may be annual or may be a lateral strut or other component for separating the sides of the conduit adjacent the collapsible portion. In some embodiments the feature(s) or component(s) adjacent the collapsible portion may hold the conduit in a fully open position adjacent the collapsible section, such that the collapsible portion is held in an intermediate position as described above.

A further embodiment of a nasal cannula with a collapsible profile is disclosed with reference to Figures 29A to 32, and Figures 34A to 34B. In use, the cannula will collapse under the force of a resuscitation mask. Possible headgear attachments to provide a means of support for the cannula are also disclosed. Aspects of the nasal cannula and conduit with collapsible profile may be combined with or substitute features of other embodiments of nasal cannula as described above.

As previously recognised in connection with the invention, patients may lose respiratory function during anaesthesia, or sedation, or more generally during certain medical procedures. Prior to a medical procedure a patient may be pre-oxygenated by a medical professional to provide a reservoir of oxygen saturation. This pre-oxygenation and CO2 flushing/washout may be carried out with a high flow therapy via a nasal cannula or other patient interface.

During fitting or removal of the nasal cannula or patient interface from the conscious or sedated patient it may be necessary for the medical professional to manipulate the position of the patient's head in order to apply or remove a head strap, or the like. As a result, this may cause some disturbance to the patient's position and/or comfort, provide awkward or uncomfortable mechanical loading on the medical professional, disturb the patient's hairnet and increase the time of the medical procedure.

As recognised above, intubation under general anaesthetic can take a significant amount of time depending on the situation. In the event that manual ventilation of an apnoeic, non-intubated, patient is urgently required (such as due to unsuccessful intubation of the patient) it is desirable to have a nasal cannula which does not have to be removed from the patient's nose and mouth prior to use of a non-invasive interface, such as a face mask. The cannula embodied herein will collapse due to the force from application of a non-invasive mask and will form a seal over and around the cannula.

Additionally, during procedures, it is desirable to avoid movement of the patient's head and disruption of a patient's hairnet and to improve handling and positioning of a patient interface. For these reasons, a form of headgear is important in order to support the collapsing nasal interface described.

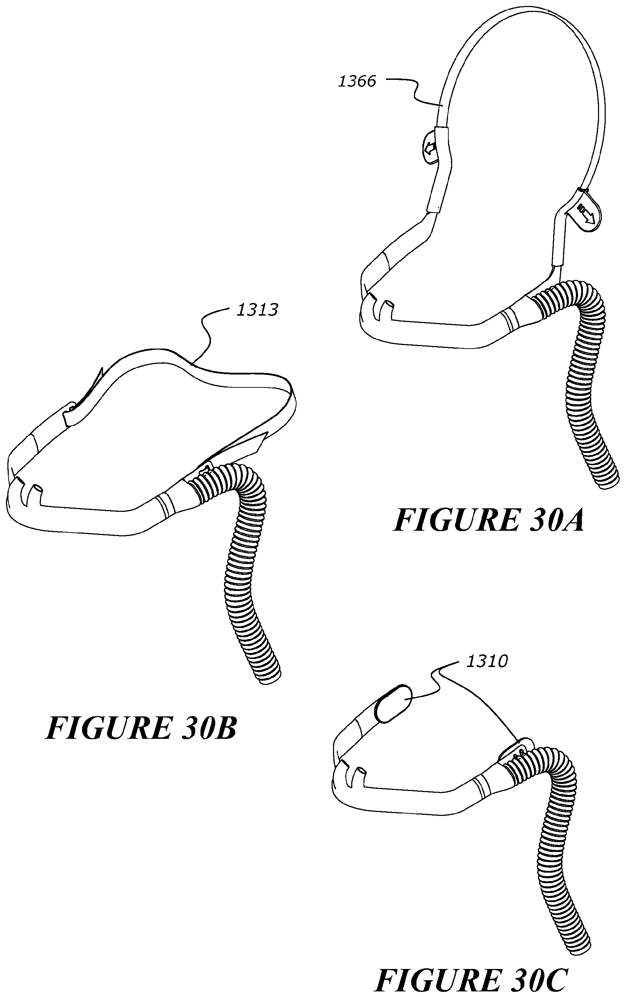

As generally shown by Figures 29G - 29R2, and 34A - 34D, a nasal cannula 1300 has two sides of supporting arms for contacting a user's face, an air-delivering side 1303 and a non-air delivering side 1304. The air-delivering side 1303 comprises a collapsible portion 1305 along part of its extent whereas the non-air delivering side may be a solid arm in the form of a strap or part thereof (optionally with a constant cross-section). The cross-sections (illustrated by Figures 29A to 29F) of both sides 1303 and 1304 are an important consideration so that a resuscitation mask can form a seal with a user's face over the cannula while it remains in place. The nasal cannula may be attached to a headgear/retention system which supports the cannula on the patient's face. Example retention options are illustrated by Figures 30A to 30C, namely a headgear 1366 with a strap portion extending over the ears, a back-of-head strap 1313 or adhesive type pads 1310, respectively.

The interface 1300 may comprise a base portion 1400 to support the nasal prong(s) 1302. The base portion 1400 may be substantially the same, or near the same width as a base of the nasal prong(s) 1302 or outlet (optionally at the largest width of the base portion 1400 and/or the base of the nasal prong(s) 1302. The width of the base portion 1400 and/or nasal prongs 1302 is taken to be the distance from the surface that is adapted to be arranged adjacent the patient's face in use, to the distal surface that is adapted to face away from the patient's face in use. One benefit of this configuration is that in use the base of the nasal prong(s) 1302 is/are located on or near a patient's face. This provides for a low profile interface or cannula 1300 in a region under a patients nose which provides a clinician more space in the area of the patient's mouth and under a patient's nose. This is important because it allows for the use of instruments such as laryngoscope, or endoscope in a fuller range of movement without collision with part of a nasal cannula under the patient's nose.

The base portion 1400 may comprise a hollowed section 1411 located on the rear side of the base portion 1400.

The base portion 1400 may comprise a manifold 1201. The manifold 1201 may be configured to provide a flow of gases to the nasal prong(s) 1302 from the air delivery side member. The manifold 1201 may be, or comprise features of the manifold as described in more detail in elsewhere in specification. In some embodiments the manifold 1201 may comprise one or more lumens which provide a gases flow path from the manifold 1201 to the or each nasal prong 1302. The one or more lumens may be angled from the manifold 1201 towards the or each nasal prong 1302.

As is shown in Figures 29O and 29P the lower portion 1401 of a front face of the base portion 1400 may be rounded or bevelled (optionally from the front face towards the bottom of the base portion). In some embodiments the base portion may comprise a wedge or taper (optionally from the front face towards the bottom of the base portion).

In some embodiments the profile of a front face of the base portion may be the same as a profile of the front face of the gases delivery side member 1303 and/or the collapsible portion 1305, and/or the intermediate section 1407.

The interface 1300 may comprise a dipped portion 1402 (i.e. being a substantially downwardly dipped portion) between a pair of nasal prongs 1302.

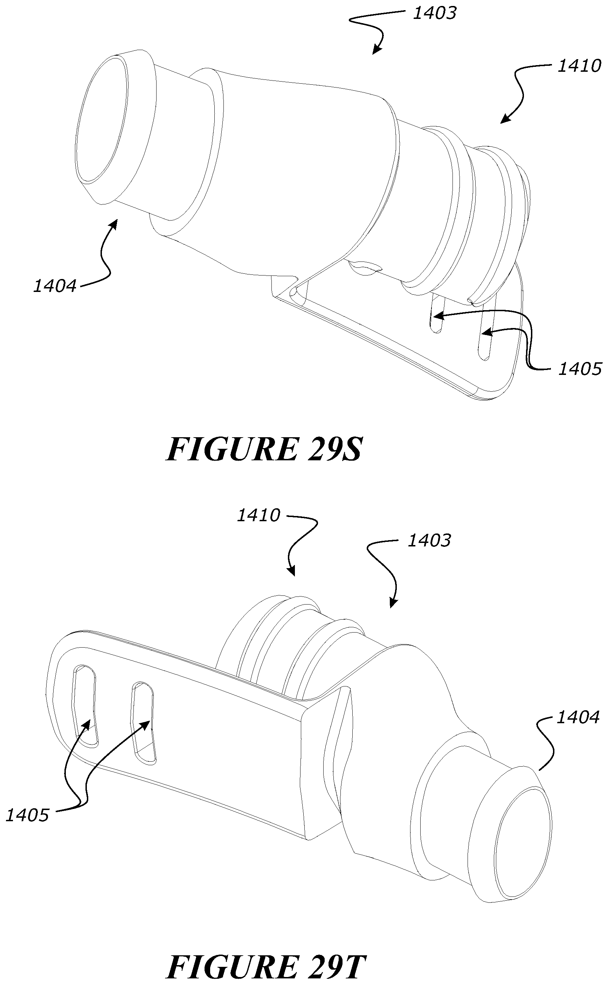

Figures 29I-29P show a gases delivery side member 1303 connected to, or connectable to an inspiratory tube connector 1403 (as shown in Figures 29S and 29T). In some embodiments the connection between the inspiratory tube connector 1403 and gases delivery side member 1303 is a sealed or substantially sealed pneumatic connection. The gases delivery side member 1303 may comprise an opening which is configured to be connectable with the inspiratory tube connector 1403. The inspiratory tube connector 1403 may be integrally formed with the gases delivery side member 1303. The gases delivery side member 1303 and inspiratory tube connector 1403 may be connected by overmoulding. The inspiratory connector 1403 may comprise a connector for connection with the gases delivery side member 1303. The connector for connection with the gases delivery side member 1303 may be a barb portion or a barb-shaped portion 1404 for connection with the gases delivery side member 1303, where the barb is to inserted in an opening 1406 (as for example shown in Figure 29Q) of the air delivery side member 1303 which may comprise an inwardly extending portion to retain the barb portion 1404.

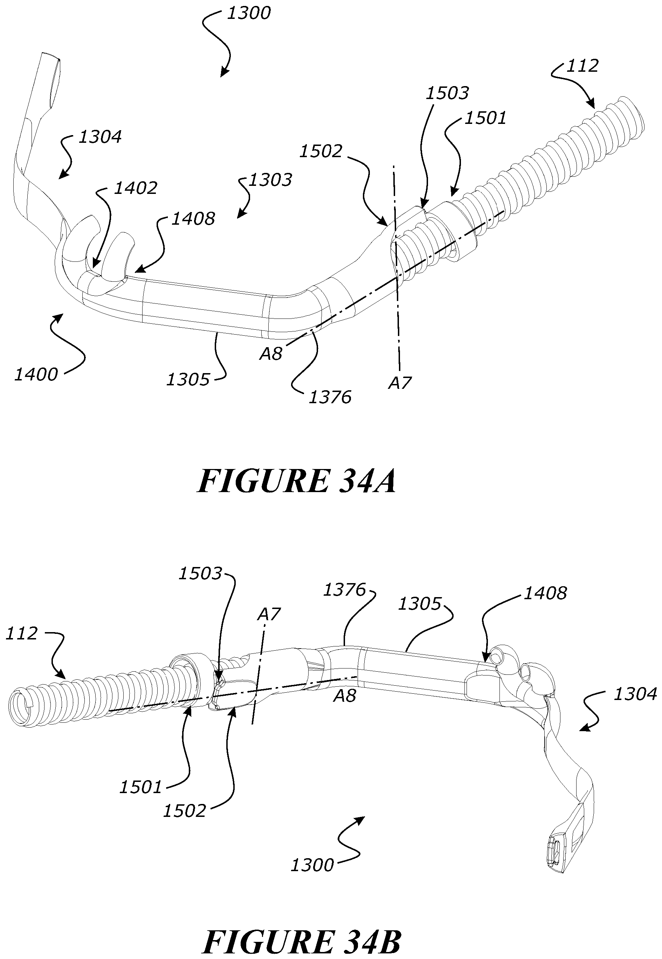

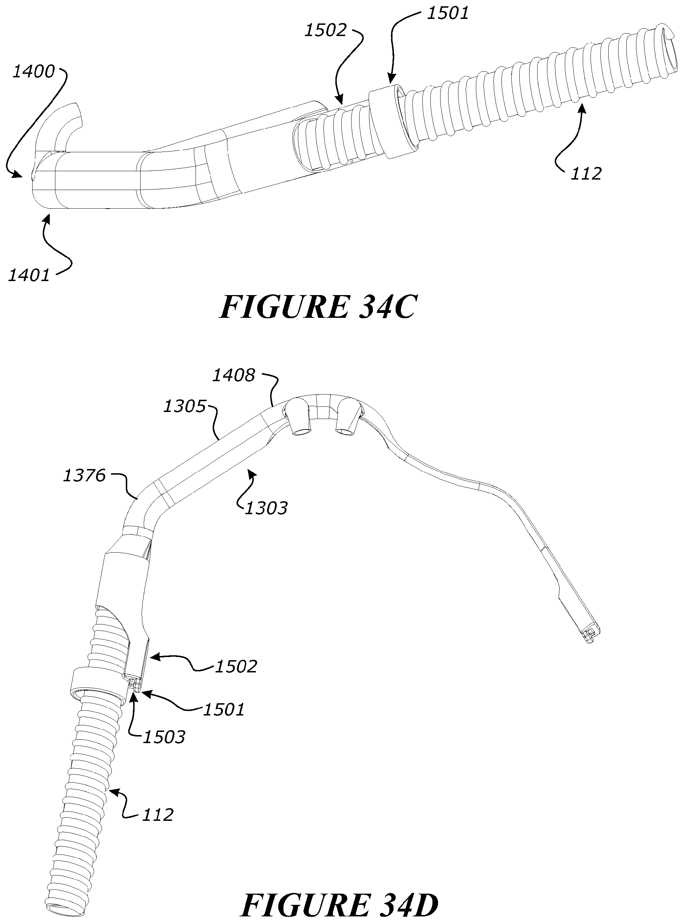

Figures 34A to 34D show a gas delivery side member 1303 which is integrally formed with an inspiratory tube connector 1403. In this case the inspiratory tube connector 1403 is integrally formed with or as part of the gases delivery side member 1303.

The inspiratory tube connector 1403 may be rigid or of a substantially non-deformable construction. The rigidity or non-deformability may provide for some degree of structural support for the air delivery member.

The inspiratory tube connector 1403 may comprise a connection for connection with an inspiratory conduit. In some embodiments the connection between the inspiratory tube connector 1403 and inspiratory conduit is a sealed or substantially sealed pneumatic connection. The connection for connection with an inspiratory conduit a barbed or a barb-shaped portion and/or a threaded connection 1410 which is configured for connection with an inspiratory conduit. The inspiratory conduit may be configured to screw onto the barbed and/or threaded connection 1410. The inspiratory conduit may comprise corrugations or other features which engage with the threaded connection 1410.

As shown in Figures 29S and 29T the inspiratory tube connector may comprise a connection feature for connection of the inspiratory tube connector to a head strap (for example strap 1313) and/or associated headgear (for example headgear 1366). The connection feature for connection of the inspiratory tube connector to a head strap may be integral with the inspiratory tube connector. The connection feature may be one or a pair, or a plurality of slots 1405. The connection feature for connection of the inspiratory tube connector 1403 may be provided on an extension portion of the inspiratory tube connector 1403 so as to allow for the tube connection to be protected from engagement with a patients face and/or the connection between the inspiratory tube connector 1403 and a head strap (for example strap 1313) and/or associated headgear (for example headgear 1366). The slots 1405 may extend to an edge of the extension portion.

The gases delivery side member may comprise a lumen configured to provide for a flow of gases from an inlet of the patient interface 1390 (optionally from an inspiratory conduit) to the at least one nasal prong 1302 or an outlet of said patient interface. At least one elbow portion, or flexible portion may be provided at one or both ends of the gases delivery side member 1303 and/or the collapsible portion 1305. Optionally as part of said air delivery side member 1303. At least one elbow portion or flexible portion, may be located substantially at or toward one or both of a downstream end of said gases delivery member 1303 or an upstream end of said gases delivery member 1303.

A lumen may be provided through the gases delivery side member, and the at least one elbow or flexible portion to allow for a flow of gases from an inlet of the patient interface to the at least one nasal prong or said outlet

The at least one elbow portion, or flexible portion may be or comprise one or more of: an angled section, a portion which has different material properties, a portion which has is relatively more flexible, or a hinging or pivoting portion, or a concertina, bellows or spring, or may have a specific geometric construction which leads to a different stiffness in a particular direction (for example a T or U shape).

The at least one elbow portion, or flexible portion comprises a first elbow or a first flexible portion 1376. The first elbow or first flexible portion 1376 may be located at an end of the gases delivery side member 1303 and/or the collapsible portion 1305 near or adjacent an inspiratory tube or the inspiratory tube connector 1403. The first elbow or first flexible portion 1376 may be located between the gases delivery side member 1303 and/or the collapsible portion 1305, and an inspiratory tube or the inspiratory tube connector 1403 as is shown in Figures 29I-29N.

The interface may also comprise an intermediate section 1407 located between the first elbow or the first flexible portion and an inspiratory tube or the inspiratory tube connector 1403. The intermediate section 1407 may be the part of the air delivery side member 1303 which provides for the connection to the inspiratory tube connector 1403.

The intermediate section 1407 may be located at an angle of about -25 degrees to about 45 degrees from a centreline of the patient interface (for example as indicated by θ₁ in Figures 29M and 29N) - with a positive angle being in a direction from the centreline towards the gas delivery side member 1303, and a negative angle being in a direction from the centreline toward the non-air delivering side arm. The intermediate section may be located at an angle of about 10 degrees from a centreline of the patient interface.

The intermediate section 1407 may be located at an angle from a front face or a rear face (for example as indicated by θ_{1A} in Figures 29M and 29N). The intermediate section 1407 may be located at an angle of about 0 degrees to about 90 degrees from a front face or a rear face, or a longitudinal axis of the gases delivery side member 1303 and/or the collapsible portion 1305. The intermediate section 1407 may be located at an angle of about 20 degrees to about 60 degrees from a front face, or a rear face, or a longitudinal axis of the gases delivery side member 1303 and/or the collapsible portion 1305. The intermediate section is located at an angle of about 25 degrees to about 35 degrees from a front face, or a rear face, or a longitudinal axis of the gases delivery side member 1303 and/or the collapsible portion 1305.

The intermediate section 1407 may be located at an angle from a lower face, or an upper face, or a longitudinal axis of the gases delivery side member 1303 and/or the collapsible portion 1305 (for example as indicated by θ₃ in Figures 29O). The intermediate section 1407 may be located at an angle of about 0 degrees to about 30 degrees from a lower face, or an upper face, or a longitudinal axis of the gases delivery side member 1303 and/or the collapsible portion 1305. The intermediate section may be located at an angle of about 5 degrees to about 25 degrees from a lower face, or an upper face, or a longitudinal axis of the gases delivery side member 1303 and/or the collapsible portion 1305, optionally the intermediate section is located at an angle of about 15 degrees from a lower face, or an upper face, or a longitudinal axis of the gases delivery side member 1303 and/or the collapsible portion 1305. The angle between the intermediate section and the lower face, or an upper face, or a longitudinal axis of the gases delivery side member and/or the collapsible portion may be an angle of elevation. This allows for the connection to the headstrap or headgear provided as part of the inspiratory tube connector 1403 to be located in an upwardly extending plane so as to direct the headstrap or headgear above a patient's ears.

The intermediate section 1407 provides for a flow path from the inspiratory tube or inspiratory tube connector 1403 to the gases delivery side member 1303 and/or the collapsible portion 1305.

The intermediate section may be about 0mm to about 30mm in length, or about 12mm to about 25mm in length.

The first elbow or first flexible portion 1376 may provide for or comprise a pivot portion and/or a hinging portion, to allow for relative movement about at least one axis (for example axis A1, or axis A2, or axis A3).

The at least one axis may comprise a first axis for example the axis A1 as shown in Figures 29I and 29L. The first axis may be oriented parallel with, or along a height of the gases delivery side member 1303 and/or the collapsible portion 1305. The first axis may be oriented substantially parallel to a patient's face in use. The first axis may be oriented substantially parallel to a portion of the gases delivery side member 1303 configured to contact a user's face. The first axis may be oriented substantially parallel to a front face and/or a rear face of the gases delivery side member and/or the collapsible portion 1305. The first axis may be located through (optionally so as to bisect) said the first elbow or first flexible portion 1376.

The at least one axis may comprise a second axis (for example axis A2 or axis A3), the second axis being oriented parallel with, or along a length of the gases delivery side member and/or the collapsible portion and/or an intermediate portion.

A cross-sectional wall thickness of the at least one elbow or flexible portion may be increased or varied compared to a wall thickness of the air delivery side member, optionally the wall thickness may be in the range of about 0.2mm to about 1mm, or about 0.05mm to about 2mm.

The gases delivery side member 1303 and/or the collapsible portion 1305 may be a substantially straight section. This allows for the collapsible portion to have a greater stiffness along its length so as to avoid kinking.

The gases delivery side member 1303 and/or the collapsible portion 1305 may be of a substantially constant cross-section.

The gases delivery side member 1303 may be about 50mm to about 100mm in length, or between about 20mm to about 70mm in length, or between about 25mm to about 35mm in length.

The collapsible portion 1305 may be at least about 5mm, about 1mm to about 30mm in length, or about 5mm to about 15mm in length, or about 10mm in length. The collapsible portion 1305 may be the length of a mask of other occluding or collapsing feature so that the mask of other occluding or collapsing feature may come into contact with the collapsing portion 1305 to cause the collapsing portion 1305 to collapse.

The at least one elbow portion, or flexible portion comprises a second elbow, or second flexible portion 1408. The second elbow, or second flexible portion 1408 may be located substantially in a region of the gases delivery side member 1303 proximate where said second flexible portion is attachable to the at least one nasal prong 1302 or outlet, and/or said base portion 1400. In some embodiments the second elbow, or second flexible portion 1408 may be located between the or a pair of nasal prongs 1302.

The second elbow or second flexible portion 1408 may provide for or comprise a pivot portion and/or a hinging portion. The pivot portion and/or a hinging portion may allow for relative movement about at least one axis (for example axis A4, or axis A2, or axis A6).

The at least one axis may comprise a first axis (for example A4), the first axis may be oriented parallel with, or along a height of the gases delivery side member 1303 and/or the collapsible portion 1305. The first axis may be oriented substantially parallel to a patient's face in use. The first axis may be oriented substantially parallel to a portion of the gases delivery side member 1303 configured to contact a user's face. The first axis may be oriented substantially parallel to a front face and/or a rear face of the gases delivery side member 1303 and/or the collapsible portion 1305. The first axis may be located through (optionally so as to bisect) said the second elbow or second flexible portion 1408.

The at least one axis may comprise a second axis (for example axis A2 or axis A6), the second axis being oriented parallel with, or along a length of the gases delivery side member 1303 and/or the collapsible portion 1305 and/or the base portion 1400.

The gases delivery side member 1303 and/or the collapsible conduit may be located at an angle from a centreline of the patient interface (for example as indicated by θ₂ in Figures 29M and 29N). The gases delivery side member 1303 and/or the collapsible conduit may be located at an angle of about 30 degrees to about 80 degrees from a centreline of the patient interface. The gases delivery side member 1303 and/or the collapsible conduit may be located at an angle of about 55 to about 60 degrees from a centreline of the patient interface.

The gases delivery side member 1303 and/or the collapsible conduit may be located at an angle from a centreline of the base portion (for example as indicated by θ_{2A} in Figures 29M and 29N). The gases delivery side member 1303 and/or the collapsible conduit may be located about 10 degrees to about 60 degrees from the base portion, or about 35 degrees to about 60 degrees from the base portion. The gases delivery side member 1303 and/or the collapsible conduit may be located about 30 degrees to about 35 degrees from the base portion.

The gases delivery side member 1303 and/or the collapsible portion 1305 may be relatively stiffer or less flexible or more resilient to a flex, along a length of the gases delivery side member 1303 and/or the collapsible portion 1305 than along a length of the at least one elbow or flexible portion (optionally one or more of the first elbow, or first flexible portion 1376 and/or the second elbow or second flexible portion 1408).

The gases delivery side member 1303 and/or the collapsible portion 1305 is relatively stiffer or less flexible or more resilient to a flex, than the at least one elbow or flexible portion about a gases delivery side member and/or the collapsible portion axis (for example axis A5). The gases delivery side member and/or the collapsible portion axis (for example axis A5) may be oriented parallel with, or along a height of the gases delivery side member 1303 and/or the collapsible portion 1305. The gases delivery side member and/or the collapsible portion axis may be oriented substantially parallel to a portion of the gases delivery side member 1303 and/or the collapsible portion 1305 configured to contact a user's face. The gases delivery side member 1303 and/or the collapsible portion axis may be oriented substantially parallel to a front face and/or a rear face of the gases delivery side member and/or the collapsible portion. The gases delivery side member and/or the collapsible portion axis may be located through (optionally so as to bisect) the gases delivery side member 1303 and/or the collapsible portion 1305. The gases delivery side member and/or the collapsible portion axis may be located through a centre (optionally a lengthwise centre), of the gases delivery side member 1303 and/or the collapsible portion 1305.

The gases delivery side member 1303 and/or the collapsible portion 1305 may be relatively stiffer or less flexible or more resilient to a flex, along a height of the gases delivery side member 1303 and/or the collapsible portion 1305 than along a height of the at least one elbow or flexible portion (optionally one or more of the first elbow, or first flexible portion 1376 and/or the second elbow or second flexible portion 1408).

The gases delivery side member 1303 and/or the collapsible portion 1305 may be relatively stiffer or less flexible or more resilient to a flex, than the at least one elbow or flexible portion about at least one axis. (for example axis A1, and/or axis A2 and/or axis A3).

The gases delivery side member and/or the collapsible portion may be relatively stiffer or less flexible or more resilient to a flex, than one or more of the first elbow, or first flexible portion about at least one axis.

The at least one axis may comprise a first axis (for example axis A1). The first axis may be oriented parallel with, or along a height of the gases delivery side member 1303 and/or the collapsible portion 1305. The first axis may be oriented substantially parallel to a patient's face in use. The first axis may be oriented substantially parallel to a portion of the gases delivery side member 1303 and/or the collapsible portion 1305 configured to contact a user's face. The first axis may be oriented substantially parallel to a front face and/or a rear face of the gases delivery side member 1303 and/or the collapsible portion 1305. The first axis may be located through (optionally so as to bisect) said the first elbow or first flexible portion 1376.

The at least one axis may comprise a second axis (for example axis A2 or axis A3), the second axis being oriented parallel with, or along a length of the gases delivery side member 1303 and/or the collapsible portion 1305 and/or an intermediate portion 1407.

The gases delivery side member 1303 and/or the collapsible portion 1305 may be relatively stiffer or less flexible or more resilient to a flex, than one or more of the second elbow, or second flexible portion 1408 about at least one axis (for example axis A4 and/or axis A2 and/or axis A6).

The at least one axis may comprise a first axis (for example A4). The first axis may be oriented parallel with, or along a height of the gases delivery side member 1303 and/or the collapsible portion 1305. The first axis may be oriented substantially parallel to a patient's face in use. The first axis may be oriented substantially parallel to a portion of the gases delivery side member 1303 and/or the collapsible portion 1305 configured to contact a user's face. The first axis may be oriented substantially parallel to a front face and/or a rear face of the gases delivery side member 1303 and/or the collapsible portion 1305. The first axis may be located through (optionally so as to bisect) said the second elbow or second flexible portion 1408.

The at least one axis may comprise a second axis (for example axis A2 or axis A6). The second axis may be oriented parallel with, or along a length of the gases delivery side member 1303 and/or the collapsible portion 1305 and/or the base portion 1400.

The collapsible portion may be relatively stiffer or less flexible or more resilient to a flex, than the at least one elbow (optionally one or more of the first elbow, or first flexible portion and/or the second elbow or second flexible portion).

In another embodiment the collapsible portion 1305 is provided or constructed to be of a sufficient construction or relative stiffness such that the collapsible portion 1305 is substantially maintained in an operative or uncollapsed configuration, and such an operative or uncollapsed configuration allowing for the supply of gases through the collapsible portion. The collapsible portion 1305 configured to maintain or resist unintended collapsing due to bending of the collapsible portion in use. Optionally, undesired or unintentional kinking or bending of the collapsible portion is substantially avoided, yet the collapsible portion is configured to facilitate relative ease of collapse upon imposition of a or the patient interface, or other collapsing feature.

In further embodiments the collapsible portion may be of a predetermined geometry such that the collapsible portion 1305 is substantially maintained in an operative or uncollapsed configuration

The collapsible portion 1305 may be relatively stiffer or less flexible or more resilient to a flex, than the at least one elbow (optionally one or more of the first elbow, or first flexible portion and/or the second elbow or second flexible portion) about an axis or direction along one or more of the height, or length of collapsible portion.

The relative stiffness relationships and/or the elbow sections allow for the isolation or attenuation of the collapsible portion from forces applied (for example force applied the air delivery arm) to the patient interface. For example, when a force is applied to bend the cannula about axis A1 (for example when the air-delivering side 1303 and non-air delivering side 1304 are moved apart from each other to widen the interface) the first elbow, or first flexible portion 1376 and/or the second elbow or second flexible portion 1408 may bend before the collapsible portion so as to prevent the collapsible conduit from kinking.

The gases delivery side member 1303 and non-air delivering side member 1304 may be located at different angles with respect to the at least one nasal prong 1302, and/or the base portion 1400.

In some embodiments the non-air delivering side member 1304 may comprise a connection feature for connection of the non-air delivering side member to a head strap and/or an associated headgear, optionally the connection feature is one or a pair, or a plurality of slots. The connection feature may be any one of the connection features as described above in relation to the inspiratory tube connector 1403.

As shown in Figures 34A to 34B the interface may comprise an engagement portion 1501. The engagement portion 1501 may be configured to engage the patient conduit 112.

In some embodiments the engagement portion 1501 is configured to engage a surface or portion of the patient conduit 112.

The engagement portion 1501 may be connected to the patient interface by a bridging section 1502.

The interface 1400 may comprise at least one nasal prong or an outlet to be received by a user's nare or mouth. In some embodiments the at least one nasal prong or an outlet extending from a manifold.

In some embodiments, the patient conduit 112 may be configured to supply a flow of gases to the at least one nasal prong;

In some embodiments the interface may comprises a gases delivery side member 1303 extending from a side of the manifold. In other embodiments the patient conduit is provided directly to a manifold.

In some embodiments, the interface may comprise a non-gases delivery side member extending from another side of the manifold.

In some embodiments the patient conduit 112 is connected to an end of the gases delivery side member 1303.

The engagement portion 1501 may be configured to engage with a surface of the patient conduit 112, at a location away, or distal from, the end of the gases delivery side member and/or where the patient conduit is connected to the gases delivery side member or the patient interface.

The patient conduit may be free from engagement with the patient interface in a location between the engagement portion 1501 and the end of the gases delivery side member and/or where the patient conduit is connected to the gases delivery side member or the patient interface.

The bridging portion 1502 may provide for, or comprises, a pivoting portion and/or a hinging portion. The bridging portion 1502 may allow for relative movement about at least one axis.

The pivoting portion and/or a hinging portion may allow the engagement portion 1501 to pivot or hinge with respect to the patient interface 1300.

The pivoting portion and/or a hinging portion may provide for a pivoting or hinging point located at or near where the bridging portion 1502 is attached to the interface.

The at least one axis (for example axis A7) may be located at or bisecting the end of the gases delivery side member 1303 and/or where the patient conduit 112 is connected to the gases delivery side member 1303 or the patient interface 1300.

The at least one axis (for example axis A7) may be oriented parallel with, or along a height of the gases delivery side member 1303 and/or the collapsible portion 1305. The at least one axis may be oriented substantially parallel to a patient's face in use. The at least one axis may be oriented substantially parallel to a portion of the gases delivery side member 1303 and/or the collapsible portion 1305 configured to contact a user's face. The at least one axis may be oriented substantially parallel to a front face and/or a rear face of the gases delivery side member 1303 and/or the collapsible portion.

The bridging section 1502 may be configured to deform before the gases delivery side member 1303 when a force is applied to the conduit 112 in a direction towards, or away from, a patient's face.

In some embodiments the elbow 1376, and/or gases delivery side member 1303 and/or the collapsible portion 1305 may be relatively stiffer or less flexible or more resilient to a flex, the bridging section 1502 about at least one axis (for example axis A7).

The bridging section 1502 may be configured to be deformable when the patient conduit 112 engages with the engagement portion 1501.

The bridging section 1502 may be configured to be deformable in a direction towards or away from a patient's face, in use, when the patient conduit engages with the engagement portion 1501.

Deformation of the bridging section 1502 may be configured to isolate the gas delivery side member 1303 from forces applied to the conduit 112.

The engagement portion 1501 may be configured to engage with an internally facing surface and/or a patient facing surface of the patient conduit 112.

The engagement portion 1501 may be configured to engage with an externally facing surface or a surface facing away from a patient of the patient conduit 112.

The engagement portion 1501 may be configured to be freely moveable relative to the patient conduit112. The engagement portion 1501 may be configured to be freely moveable along a length of the patient conduit 112.

The engagement portion 1501 may be configured to be connected, or connectable to the patient conduit 112. In some embodiments, the connection between the engagement portion 1501 and patient conduit 112 may prevent relative movement between the engagement portion 1501 and patient conduit 112.

The engagement portion 1501 may comprise one or more connection features configured to allow for connection between the engagement portion and the patient conduit.

The bridging section 1502 may comprise a relatively flexible section. In some embodiments, the relatively flexible section may comprise a material with shape memory.

The bridging section 1502 may be relatively more flexible torsionally about an axis along the bridging section 1502 (for example A8). In some embodiments, the axis (for example A8) extends along a length of the bridging section 1502. In some embodiments, the axis (for example A8) extends in a direction along an axis of the patient conduit 112.

As described above, the gases delivery side member 1303 may comprises a collapsible portion 1305.

The engagement portion 1501 may engages the patient conduit 112 at a location away from the gas delivery side member 1303 and/or a location away from where the patient conduit 112 is connected to the gases delivery side member 1303 or the patient interface 112.

As shown in Figures 34A-34D, the engagement portion 1303 may comprise a loop, wherein the loop extends around at least part, or the entirety of the patient conduit. In some embodiments, the loop is a connection features as described above.

The patient conduit 112 may be configured to be located within the loop.

A diameter of the loop may be larger than a diameter of the patient conduit 112.

The patient conduit 112 may be configured to be moveable within the loop, to allow relative movement between the patient conduit 112 and the loop.

A diameter of the loop may be the same, or smaller than, a diameter of the patient conduit 112.

The patient conduit 112 may be configured to be retained within the loop, to prevent relative movement between the patient conduit 112 and the loop.

The engagement portion 1501 may comprises a C-shaped portion.

The engagement portion 1501 and bridging section 1502 are integrally formed, and/or formed a single component.

The engagement portion 1501 and bridging section 1502 are integrally formed with the patient interface 1300 and/or the gases delivery side member 1303.

The patient conduit 112 may be configured to be directly connected to the end of the gases delivery side member 1303.

The patient conduit 112 may be configured to be connected to the end of the gases delivery side member 1303 by one or more connector (for example having features of the inspiratory connector 1403 as described above).

The patient conduit 112 may be integrally formed with the gases delivery side member 1303.

The gases delivery side member 1303 may be connected to the patient interface 1300 by one or more threaded connection. The gases delivery side member 1303 may be internally threaded.

The interface 1300 may comprise a connection feature 1503. The connection feature 1503 may be for connection of the gases delivery side member 1303 to a head strap and/or an associated headgear. In some embodiments the connection feature is one or a pair, or a plurality of slots or a clip.

The bridging section 1502 and/or the engagement portion 1501 may comprise the connection feature 1503.

The connection feature 1503 may be integrally formed with the bridging section 1502 and/or the engagement portion 1501.

The connection feature 1503 may be connectable and disconnectable from the bridging section 1502 and/or the engagement portion 1501.

The connection feature 1503 may be connected to the head strap and/or the associated headgear at a location rearward to the engagement portion and/or the bridging portion and/or further from the at least one nasal prongs.

In some embodiments the connection feature 1503 may be located distal or away from where the bridging portion 1501 is connected or attached to the interface 1300.

The connection feature 1503 being located on the bridging portion may provide for isolation of strap or tube forces from the interface. The flexibility of the bridging portion may act to attenuate the interface and the gases delivery side member from strap and tube forces.

The connection feature 1503 provides for a pivotable connection with the head strap and/or the associated headgear. In some embodiments, the pivotable connection is pivotable in a direction towards or away from a patient's face in use.

The configurations of the air-delivering side 1303 and non-air delivering side 1304 from the nasal cannula of Figures 29G, 29H, and 34A respectively may be separately combined with any compatible aspect of earlier embodiments. For example, the cross section shape (Figures 29A and 29B) of the non-air delivering side may be combined with a collapsible air delivering side configured according to any of the earlier embodiments such as Figures 6A to 8D and/or 16A to 19. Likewise, the cross section shapes (Figures 29C to 29F) of the air delivering side may be incorporated as the collapsible section according to any of the same earlier embodiments.

A cross section view of the non-air delivering side 1304 is best illustrated by Figures 29A and 29B which particularly shows an elongated asymmetric lens shape. An inner or skin side 1368 of the cross section, which is in direct contact with the patient's face, has a greater depth and thus a more pronounced outward curve than the mask side 1369 that faces, to contact in use, the mask surface 304.

The elongated, asymmetric lens shape is embodied by both the skin side 1368 and mask-facing or outer side 1369 of the cross section having curved profiles with a central, flat region 1370 and 1371 respectively. The skin side 1368, which is in direct contact with the patient, is thicker and bulges further than the outer side 1369 from the relatively sharp edges 1372 where the skin side 1368 and outer side 1369 meet; e.g. a bulging thickness of 2mm compared to 0.5 mm from the edges. The greater depth or pronounced outward curve of the skin side 1368 compared to outer side 1369 is relative to an imaginary plane extending between the meeting edges 1372 as shown in Figure 29A.

The difference in thickness is required because skin 1367 deforms more than a mask 304 so the thicker, skin side 1368 can press into the face and thus aid in the formation of a seal. The dimensions shown by Figure 29A are by way of example only and may vary by 50% or more depending on the size/scale (e.g. adult/child) of cannula.

Deformation of the skin 1367 is shown by Figure 29B as a mask 304 surface is pressed against outer side 1369.

Alternatively, the outer side 1369 of the cross section may be substantially flat from one edge 1372 to the other. The points where the skin 1368 and outer 1369 sides meet may alternatively be more tapered to a point or a rounded point.

Another embodiment of the non-air delivering side 1304 may involve a very thin cross section. However, this embodiment is not expected to be preferable as a thin cross section has a lack of structural rigidity which means the non-air delivering side of the cannula could be prone to rip or tear. Nevertheless, some construction materials may provide adequate performance in the context of the invention.

In further forms, the non-air delivering side of the cannula may be excluded altogether. This can allow a design with reversible prongs to be used on either side of the patient's face. Further detail regarding this design is provided in connection with description of the headgear to follow.

Figures 29C to 29F illustrate cross sections of the air or gases-delivering side 1303. Particularly, Figure 29C shows an obround shaped constant wall thickness of a substantive part of the air delivery side 1303, as seen at the open end visible from Figure 29G; whereas Figures 29D to 29F show alternative cross section details of a collapsible portion indicated at area 1305 of Figure 29G.

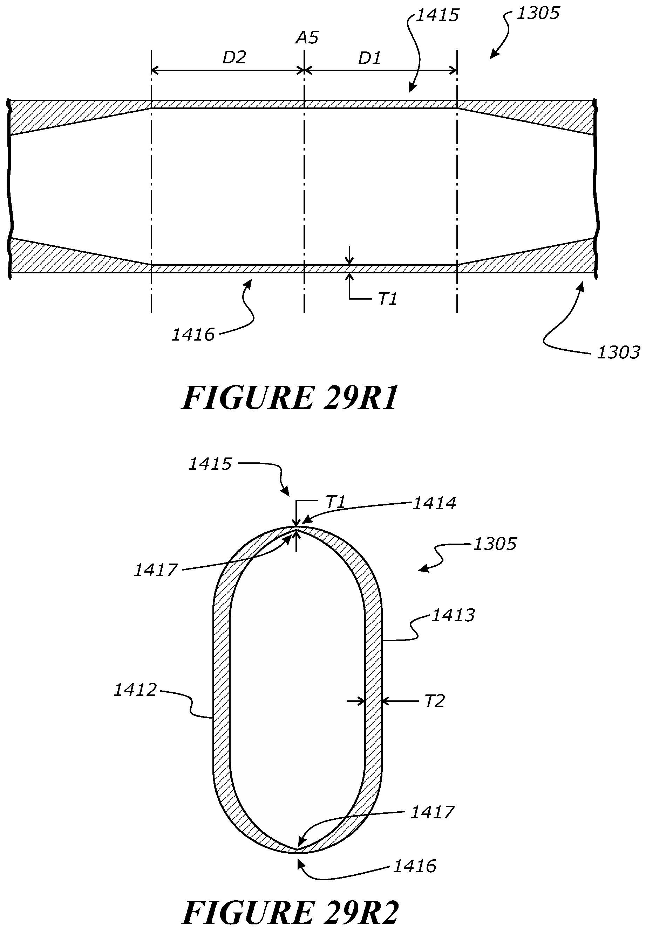

The gases delivery side member 1303 may have a wall thickness of between about 0.2mm to about 1mm, or about 0.05mm to about 2mm, or about 1mm. A cross-section of the interface taken through the gases delivery side member 1303 shows the cross-section of the gases delivery side member which may be of an obround. The wall thickness of the gases delivery side member 1303 as shown in Figures 29R shows a constant wall thickness. A cross-sectional profile of a substantive length of the gases delivery side member 1303, excluding the collapsible portion 1305, may have a constant wall thickness.

The collapsible portion 1305 may be located substantially in a central portion of the gases delivery side member 1303.

One embodiment of the collapsible region as shown in Figure 29D involves the respective, opposing (skin contacting and mask contacting) sides of the cross section progressively narrowing until they meet at an offset region 1373. This offset region 1373 has the thinnest wall thickness of the cross section. The shape allows collapsing to occur anywhere along each (upper or lower) offset region 1373 as opposed to at a single point which may require specific cannula alignment.

Another embodiment as shown in Figures 29E, 29F and 29R2 involves the respective, opposing sides of the cross section progressively narrowing until they meet. The place at which the opposing sides meet is the place of thinnest wall thickness 1374. This shape also collapses somewhere in the region of this place of thinnest thickness as force F, rather than necessarily at a point (indicated by Figure 29F where the collapsible portion 1305 is shown being squashed) from the mask is applied, either entirely vertical or having a horizontal component. Both cross sectional embodiments of the collapsible region include a portion with constant thickness 1375 that spans a length of both opposing sides and, in either case, the thinnest regions promote collapsing.

Opposing walls of the collapsible portion may, in use, contact the user's face and a mask respectively. The opposing walls (optionally comprising a front wall 1412 to contact a mask and a rear wall 1413 to contact the user's face) progressively narrow or reduce to meet at, or until said opposing walls until they meet at the thinned region 1414. The thinned region 1414 optionally being an offset region. The thinned region 1414 may have thinnest wall thickness (for example T1) of the cross-sectional profile (for example as shown in Figure 29R2). The thinned region 1414 may be the thinnest wall thickness region (for example T1) of the cross sectional profile.

The opposing walls (optionally comprising a front wall 1412 to contact a mask and a rear wall 1413 to contact the user's face) may meet at an angle to each other in the thinned region 1414 on an inner surface of the collapsible portion 1305. The wall thickness at the location where the opposing walls meet (for example the thinned region 1414, and thickness T1) may be about 0.1mm, or about 0.05mm to about 2mm.

The external surface of the collapsible portion 1305 may be substantially round or rounded or is a continuous surface in the area of the thinned region 1414 as shown by Figure 29R2.

The opposing walls (optionally comprising a front wall 1412 to contact a mask and a rear wall 1413 to contact the user's face) may have a substantive portion with constant thickness and optionally the thinned region 1414 promotes collapsing (from the front face to the rear face - for example in the same manner as shown in Figure 29F).

The thickness of the opposing walls (for example T2) may be about 1mm. The wall thickness of the opposing walls (for example T2) is about 0.2mm to about 1mm, or about 0.05mm to about 2mm.

A cross-sectional profile (for example that shown in Figures 29D, 29E, 29F and 29R2 of the collapsible portion may comprise at least one substantially thinned region 1414.

The thinned region 1414 may be located at one or both of an upper portion 1415 or a lower portion 1416 of the collapsible portion 1305. The thinned region 1414 may be provided at alternative or substantially opposing locations about an internal perimeter or inner surface of said collapsible portion 1305.

The collapsible portion 1305 may comprise an angled section 1417 (optionally on an inner surface of the collapsible portion 1305) in the thinned region 1414.

The inner surfaces of the collapsible portion 1305 converge to said thinned region 1414.

The inner surfaces of the collapsible portion 1305 may meet at said thinned region 1414 to provide for a pre-determined bending or hinging point.

The external surface of the collapsible portion may be substantially round or rounded or is a substantially continuous surface in the area of the thinned region (for example as shown in Figures 29D, 29E, 29F and 29R2.)

The wall thickness of the collapsible portion in the thinned region is about 0.1mm, or about 0.05mm to about 2mm.

The collapsible portion 1305 extends along a portion of the length of the air or gases-delivering side 1303.

The gases delivery side member 1303 transitions from a gases delivery side member wall thickness that may be substantially greater than said the wall thickness of the thinned region 1414 of said collapsible portion 1305.

The gases delivery side member wall thickness remains of a constant wall thickness or is of a substantially greater wall thickness than the wall thickness of the gases delivery side member 1303 at the thinned region 1414 of the collapsible portion 1305.

The wall thickness of the gases delivery side member 1303 along a length of said lumen may remains substantially constant or may be of a substantially greater wall thickness than the wall thickness of the gases delivery side member at the thinned region of the collapsible portion 1305.

The wall thickness of the gases delivery side member 1303 may vary along a length of the lumen, the wall thickness reducing or being reduced or substantially less in the region of the thinned region 1414 relative to the wall thickness of the gases delivery side member 1303 along the length of the lumen.

The wall thickness of gases delivery side member 1303 outside of the thinned region 1414 may transition via a step or a taper to the wall thickness at the thinned region 1414.

The thinned region 1414 may extend along the entire length of the collapsible portion 1305, and optionally where the thinned region comprises an internal angled section or portion may provide for a lengthwise valley or edge along the collapsible portion to provide for a hinging or pivoting or folding type location so as to aid in collapsing the collapsing portion 1305.

The collapsible region 1305 comprises thinned regions 1414 (being upper region 1415 or lower region 1416), e.g. being about 3 to about 10mm, about 10mm or about 5 mm length portions along the length of the air-delivering side 1303 of the cannula (for example as distances D1 and D2 from axis A5 as shown in Figure 29R1). Preferably, there is an elbow 1376 in the region of the cannula near where it attaches to an inspiratory tube connector. This distances the connector from the collapsible section. Additionally, the elbow 1376 projects forward of the face to avoid kinking of the cannula. The elbow gives rigidity and keeps this part of the cannula in an unobstructed state.

Figures 29G and 29H illustrate perspective and plan views respectively with important features identified, such as the air delivery side arm 1303, the elbow 1376, nasal prongs 1302 and a lip contacting region 1377 being contoured to accommodate an upper lip of a user.

It is apparent that both the non-air delivering 1304 and air delivering 1303 sides of the cannula are contoured to be placed across a patient's face and, particularly, the region 1377 of the cannula in the vicinity of the nasal prongs is contoured to the upper lip. This contouring means that a medical professional's vision won't be occluded when looking down along a patient's face for any reason, such as looking down a laryngoscope while placing an endotracheal tube.

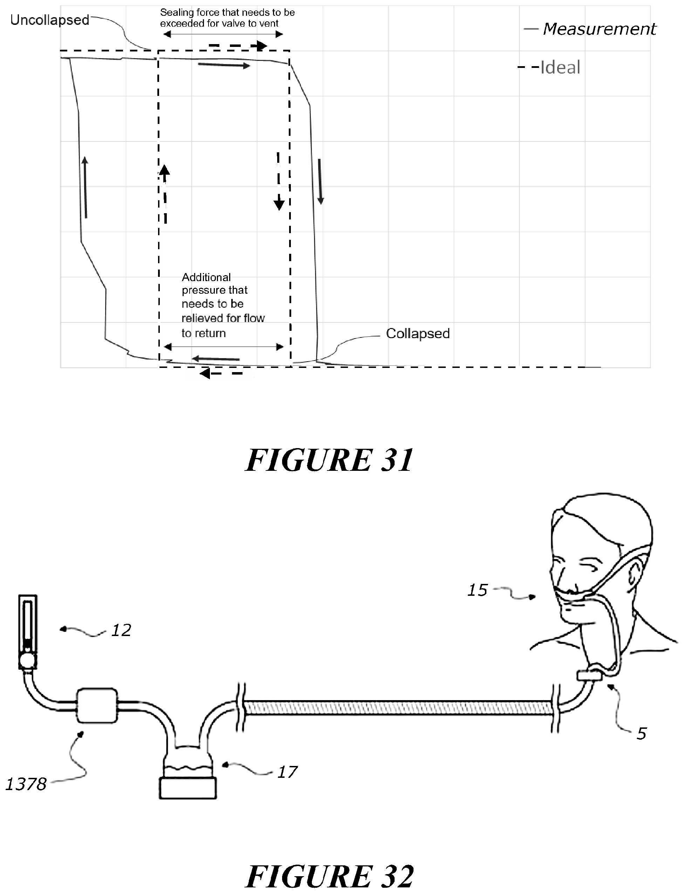

For different sizes of cannula, the resistance to flow will preferably be the same. Particularly the same system, which may comprise an inspiratory tube connector and flow-compensated pressure relief valve (FCPRV) can be used for all sizes of cannula e.g. small, medium, large*.* Alternatively, the resistance to flow of the FCPRV 1378 connected downstream from a flow source 12 illustrated by Figure 32 may be altered which will result in a specific valve for each cannula size. In general the FCPRV dynamically adjusts its relief pressure proportionally with the flow rate through the system

Alteration of the resistance to flow can take place by introducing a restriction or by varying the thickness of a section of the wall of the cannula. A thicker wall increases resistance. For example, the wall thickness may be varied in the elbow region 1376, i.e. the region immediately preceding the collapsible region 1305 or in the nasal prong(s) 1302. Ideally, the resistance to flow of the smallest nasal cannula will remain unchanged and the larger cannula sizes will be altered to match the resistance to flow of this smallest size.

Additionally, the nasal prongs 1302 of the cannula may be more curved to go back further in the nose.

In a preferred form of manufacture, the moulding tool used imparts the exterior surface (the surfaces of the cannula in contact with the patient and also with the external environment) of the cannula material a slightly roughened finish. This roughness allows the exterior surface of the cannula not to stick with the mask. The moulding tool may make the interior surface of the material smooth. A smoothness gives the material some stickiness and a small amount of hysteresis which contributes to holding together the cannula when collapsed. Alternatively, the interior surface could be roughened to avoid excessive sticking and hysteresis.

As illustrated by Figures 30A and 30B, the collapsible nasal cannula may be supported on the patient by a suitable retention mechanism, e.g. a rigid headband 1366, an elastic strap 1313. Preferably there are slots at the end of each side arm of the cannula which a strap can be moved through and adjusted. The strap may be a single strap or a variation thereof (e.g. a bifurcated strap).

In the form illustrated by Figure 30C, adhesive pads 1310 may be employed, which form a strong connection as they stick/attach to the face of a patient in the upper cheek bone area. These pads may be configured to disconnect and re-attach to the cannula, e.g. clip into the same slots as the strap and can be easily unclipped during use so the pads can remain on the face whilst the cannula is removed.

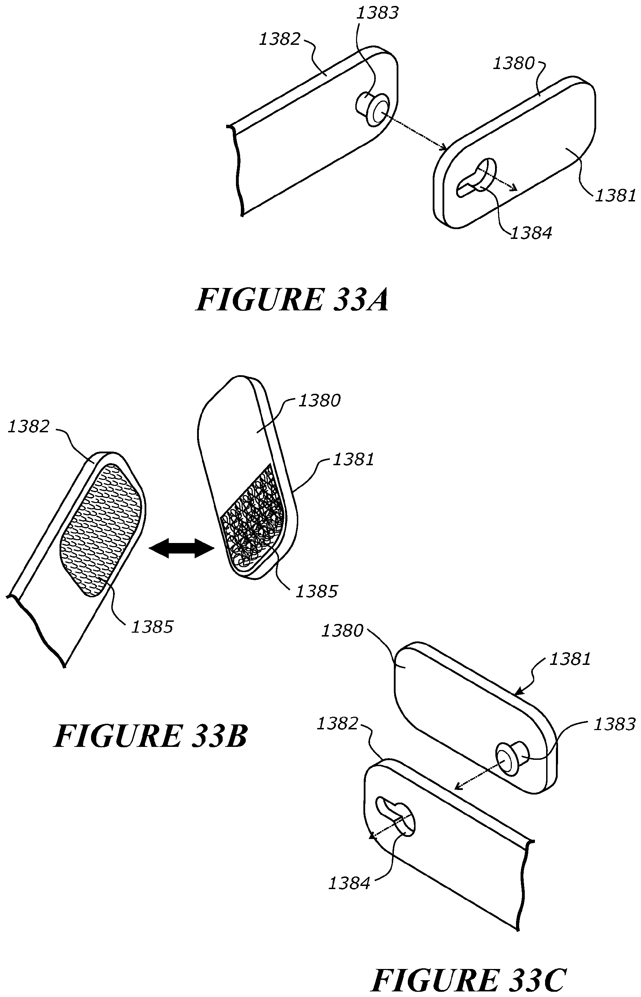

Various forms of adhesive pad, compatible with at least the headgear arrangement of Figure 30C, are illustrated by Figures 33A to 33I. In all forms the adhesive pad is generally labelled 1380, with an adhesive side thereof labelled 1381, i.e. the side attached to a user's face. The patient interface component side is generally denoted 1382, i.e. the side associated with the cannula or other patient interface to be attached to the user's face by the adhesive pad 1380. Each embodiment has a particular attachment means between pad 1380 and component 1382.

For example, Figure 33A shows a pin 1383 and hole/slot 1384 configuration where an enlarged end of the pin is retained by a slot. Such a configuration allows a swivel connection for the patient interface 1382 while attached to the face. Figure 33C shows the same connection means but reversed, where the pin extends from the non-adhesive side of the pad 1380. Alternatively, a dome configuration may be implemented.

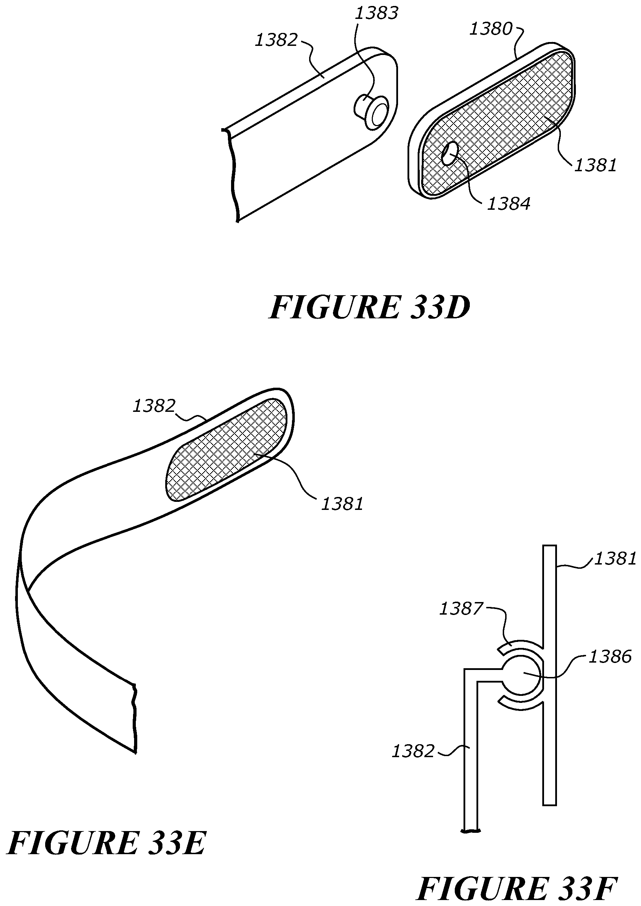

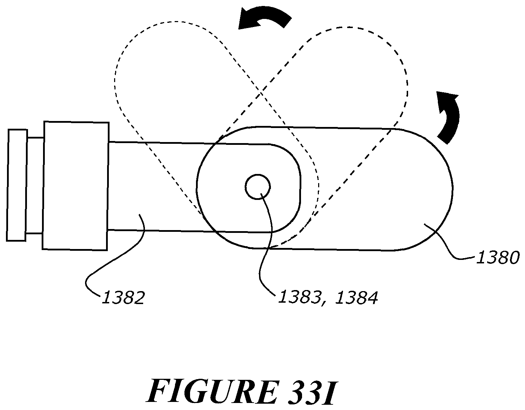

Figure 33B shows a hook and loop connection 1385 while Figure 33D shows a pad 1380 featuring a hole 1384 that is intended to deform and receive a barbed pin 1383 by virtue of its material of construction, e.g. a relatively soft material to stretch over and around the barb. This configuration, and that of Figures 33A and 33C may provide a swivel solution as generally shown by Figure 33I.

Figure 33E shows a configuration where the patient interface 1382 may be attached directly to the user's face by an adhesive surface 1381. The distal end of the interface may be removable by a perforation of suitable plug/socket type joint.

Figure 33F shows a ball 1386 and socket 1387 configuration where the socket 1387 is mounted on the pad 1380 to receive a ball 1386 extending from the patient interface side 1382. However, the configuration could be reversed where the socket is located on the interface side.

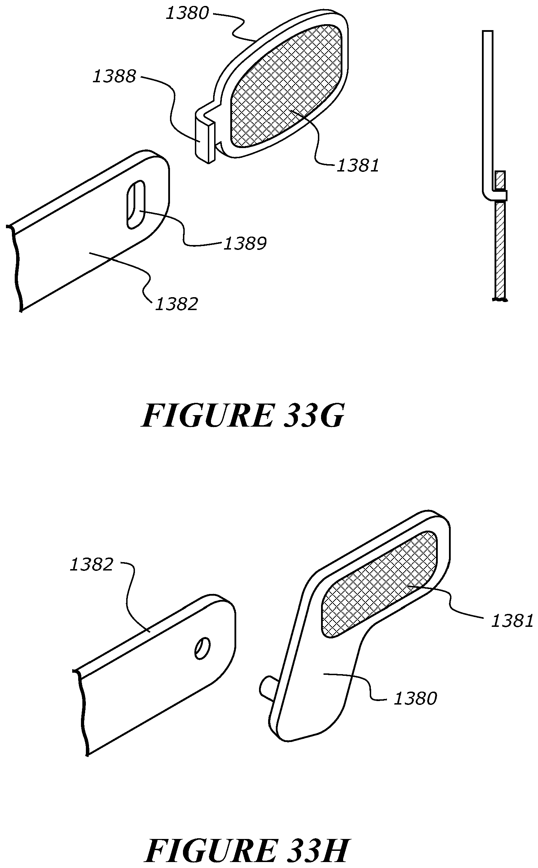

Figure 33G shows an adhesive pad 1380 with an extending hook 1388 or clip end, to be received by a slot or mating feature 1389 located at the component side 1382. Again, the configuration could be reversed where the male part 1388 extends from the component side 1382.

Figure 33H shows a non-uniform shape for the adhesive pad 1380 to contrast the relatively symmetric configuration of other embodiments. However, any suitable non uniform pad shape could be implemented, e.g. to conform to facial contours and geometry as needed.

The illustrated adhesive pads 1380 may be made of Hydrocolloid, hydrogel or other suitable adhesive material commonly used as on skin or as a wound dressing. Adhesive formulation used may allow the pad to be re-adhered to the patient's face.

Alternatively, the non-air delivering side 1304 of the cannula can be removed so just the air delivering, collapsible side remains. An adhesive may then be placed along the entire, one-sided cannula to secure on the patient. This one-sided format may allow the cannula to be placed on either side of the patient if reversible prongs are implemented.

A hysteresis effect has been observed during the interaction of a collapsible nasal cannula with a face mask. This effect is due to the use of the collapsible cannula in combination with a FCPRV as suggested in the system of Figure 32. The hysteresis effect is beneficial as it means an intentional action has to be made by someone, such as a medical professional, to collapse the cannula and also, to uncollapse the cannula. For example, a medical professional will decide to push a resuscitation mask down on the cannula with enough force (to cause collapse) and will then decide when to pull it off again (reversing the collapse).

The function of a system involving a FCPRV during collapsing and uncollapsing of the cannula, which results in hysteresis is illustrated by Figure 31. It is noteworthy that the operating parameters described herein may vary in practice and for are example purposes.

In terms of the hysteresis as shown by Figure 31, the top line represents a constant flow. Preferably, when the mask is applied over the cannula with a pressure exceeding a certain level the pressure valve vents. Flow in the cannula then drops to zero and the cannula fully collapses. When removing the mask, the certain level needs to be relieved on the cannula and then flow returns and rises back to the initial value.

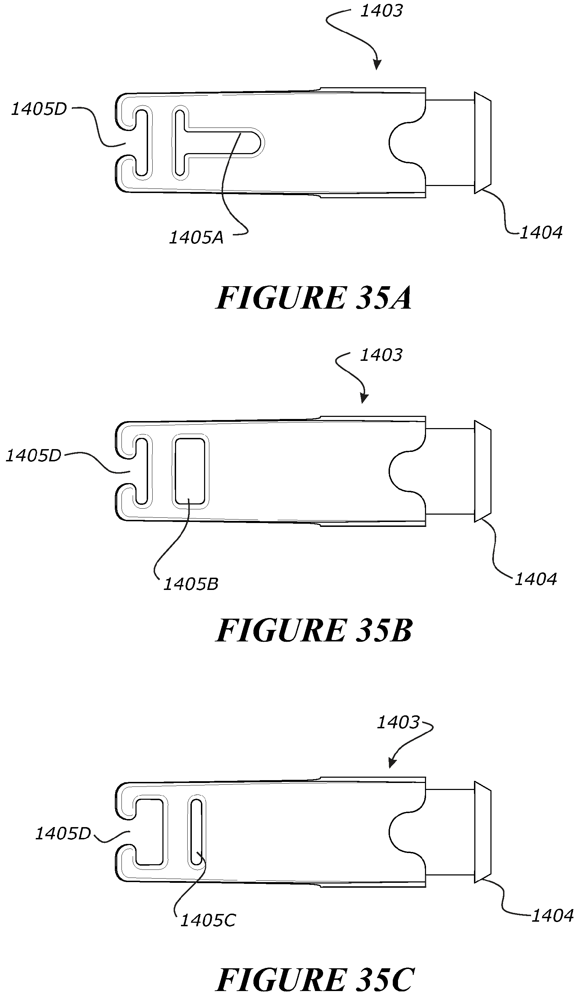

Figures 35A, 35B, and 35C show various example embodiments of a connector, such as the inspiratory tube connector 1403 described herein for the connection of a head strap and/or headgear to a patient interface. Such a connector is also shown in Figures 29S and 29T, where the inspiratory tube connector may comprise a connection feature for connection of the inspiratory tube connector to a head strap (for example strap 1313) and/or associated headgear (for example headgear 1366).

The connection feature for connection of the inspiratory tube connector to a head strap may be integral with the inspiratory tube connector. The connection feature may comprise one or a pair, or a plurality of slots 1405.

The connection feature may be provided on an extension portion of the inspiratory tube connector 1403 so as to allow for the inspiratory conduit connection 1410 (which may be threaded) to be protected from engagement with a patient's face and/or the connection between the inspiratory tube connector 1403 and a head strap (for example strap 1313) and/or associated headgear (for example headgear 1366). The slots 1405 may extend to an edge of the extension portion.

In an example embodiment shown in Figure 35A, the one or more slots 1405 may comprise a T slot 1405A. Whilst a T-shaped slot is shown in Figure 35A, it will be understood that any similar form of perpendicularly arranged slot could be provided, for example an L shape, U shape, or C shape.

Alternatively or in addition, Figures 35B and 35C show wider or narrower slots 1405B and 1405C, respectively. Any of these example embodiments may be combined on a connector, and may provide for ease of use and installation of a headgear strap. For example, such slot configurations may allow connection of a headgear strap with reduced dexterity required.

In the example embodiments of Figures 35A, 35B, and 35C, an additional slot or recess 1405D is shown, comprising an open ended slot or opened T section suitable for receiving a headgear strap. It will be understood however that these embodiments could be provided as a single slot, could be combined with closed slots, or provided with more than two slots as required. Such a feature may provide for improved ease of use and installation of a headgear strap, for example by allowing said strap to be threaded through the open section of 1405D, rather than through a closed slot (which may require increased dexterity).

Figures 35B and 35C illustrate a wide slot 1405B and a narrow slot 1405C, and may additionally comprise a corresponding narrow recess 1405D, shown in Figure 35B, or corresponding wide recess 1405D, shown in Figure 35C. These varied sizes of recess or slot may provide additional ease of installation benefits, and/or improved security of connection to a headgear strap as outlined above.

In example embodiments (not shown), the slots described above in relation to Figures 35A, 35B and 35C may comprise retention features on an edge of the slots. In example embodiments the retention features may comprise a jagged edge, teeth, a tapered profile, or any other form of grip feature to facilitate retention of a strap when inserted into the slot and brought into a use position. Such a feature may advantageously provide for additional security of connection of a headgear strap and the connector.

It will be understood that an additional connector on an opposite end of the headgear or patient interface, for example a non-air delivery side as described herein, may comprise similar connectors, connection features, and slots/recesses to those described above.

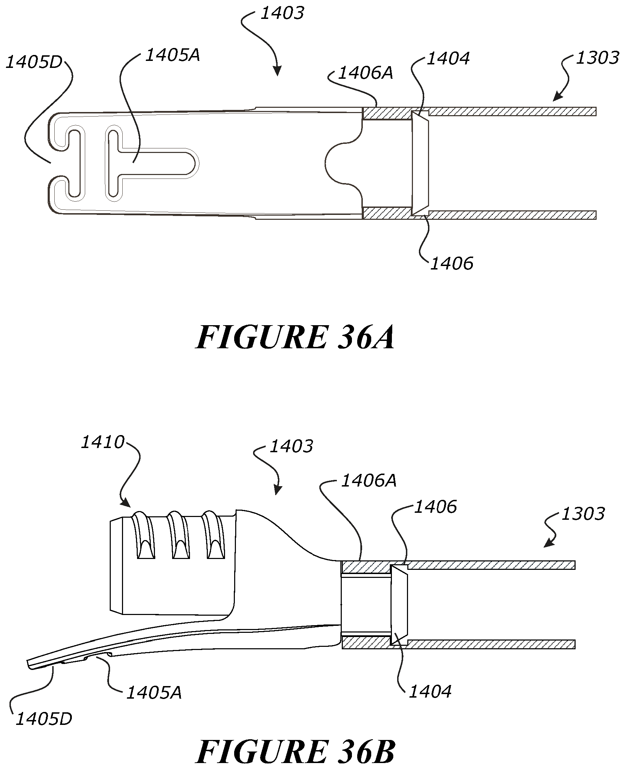

Figures 36A and 36B illustrate an example embodiment of a connector in connection with a gases delivery side member 1303 as described herein. As outlined above in relation to figures 29I-29P, which show a gases delivery side member 1303 connected to, or connectable to an inspiratory tube connector 1403 (as shown in Figures 29S and 29T).

In some embodiments the connection between the inspiratory tube connector 1403 and gases delivery side member 1303 is a sealed or substantially sealed pneumatic connection.

The gases delivery side member 1303 may comprise an opening which is configured to be connectable or releasably connectable with the inspiratory tube connector 1403. The inspiratory tube connector 1403 may be integrally formed with the gases delivery side member 1303. The gases delivery side member 1303 and inspiratory tube connector 1403 may be connected by overmoulding. The inspiratory connector 1403 may comprise a connector for connection with the gases delivery side member 1303. The connector for connection with the gases delivery side member 1303 may be a barb portion or a barb-shaped portion 1404 for connection with the gases delivery side member 1303, where the barb is to inserted in an opening 1406 (as for example shown in Figure 29Q) of the air delivery side member 1303, which may comprise an inwardly extending portion to retain the barb portion 1404.

In an example embodiment as shown in Figures 36A and 36B, the air delivery side member 1303 may comprise an inwardly extending portion or thinned sidewall 1406 configured to retain a barb portion 1404 of the connector 1403. In an example embodiment, an additional thickened region or sidewall 1406A may be provided to provide additional retention to the connector barb portion 1404. Such an inwardly extending portion or thinned sidewall 1406 may advantageously provide for improved comfort to a patient, and/or provide additional security of connection.

In an example embodiment, the barbed portion 1404 in combination with the inwardly extending portion 1406 may provide for additional flexibility and allow additional movement of the connection, which may improve patient comfort. In an example embodiment, these features may provide for an inward narrowing movement of the sidewalls of the air delivery side member 1303 upon exertion of a separation force between the air delivery side member 1303 and the connector 1403. This may advantageously provide for additional resistance to disconnection, and/or may provide for a resistance to pinching of the air delivery side member 1303.

Turning to Figure 36B, the inspiratory conduit connection 1410 may be positioned at an angle or flared with respect to the portion of the inspiratory tube connector 1403 comprising the connection feature(s) 4105. An example configuration is illustrated in Figure 36B. Such a configuration may facilitate the separation of the inspiratory conduit connection 1410 and a patient's face, allowing an inspiratory conduit, such as a gases supply tube, to be connected, whilst also maintaining a gap between the inspiratory conduit and a patient's face when in use.

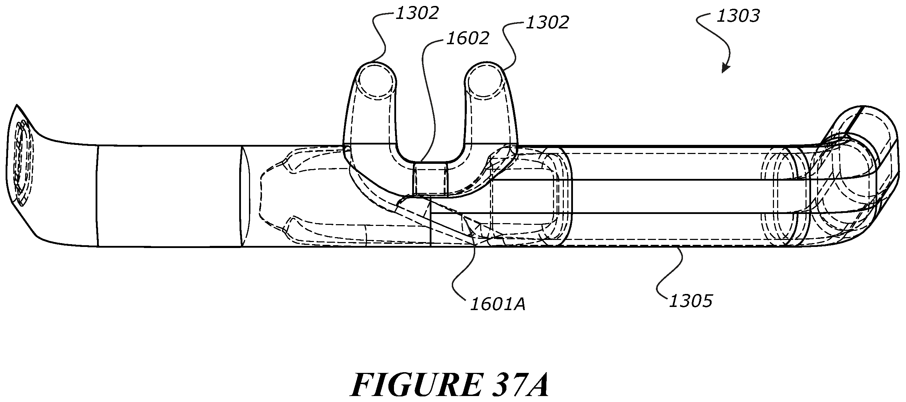

Figure 37A illustrates a frontal view of a patient interface as described herein showing hidden detail lines. In an example embodiment, a ramp portion 1601A may be provided in the region of the nasal prongs 1302, for example adjacent to the prong region of the patient interface.

In an example embodiment a septum dip or gap may be formed between a prong or prongs. In an example embodiment, the septum dip or gap may be positioned between two nasal prongs, and at a height below an upper or top wall of the collapsible portion 1305. Such a dip or gap, reduced in height, is shown as 1602 in Figure 37A and may advantageously direct a portion of gas flow into an upstream nasal prong.

In an example embodiment, the ramp portion 1601A may work in conjunction with the septum dip 1602 and aid in the substantial distribution of gases flowing to the prongs. The septum dip 1602 may split the gas flow. The ramp 1601A may aid in flow distribution by providing a certain resistance to flow in one prong that causes the gas to preferentially flow in the other prong.

In an example embodiment, the ramp portion 1601A, in combination with dipped region 1602, may advantageously even out or balance or distribute a flow of gas from the collapsible region 1305 to the nasal prongs 1302. Gas flow may be directed by the dipped region 1602 being below the upper wall level of the collapsible region, and by the ramp portion 1601A beginning a transition in the nasal prong region, and directed towards the downstream nasal prong. In this way, the distribution of gas provided to each nasal prong may be adjusted as required, for example to balance or even out the flow of gas through each nasal prong.

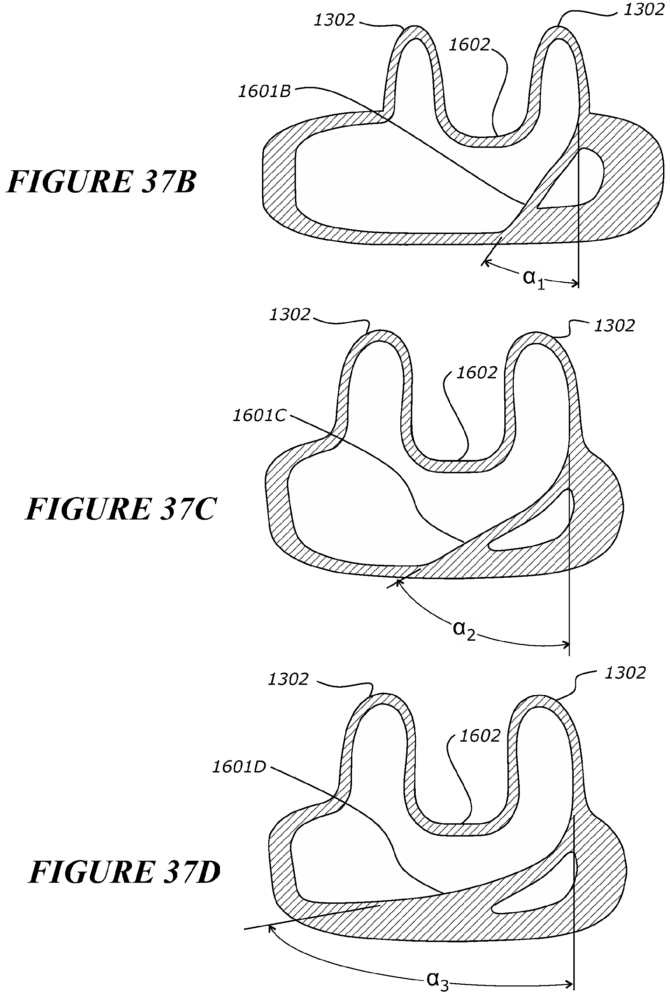

Figure 37B illustrates an alternative patient interface, for example for a smaller patient. In this example embodiment the ramp portion 1601B comprises a steeper transition angle. This may advantageously reduce the resistance to flow.

It will be understood that the ramp portion 1601A and 1601B could be curved or shaped to advantageously provide a target resistance to flow (e.g. by increasing or decreasing flow resistance), and improved balance of gas supplied via nasal prongs. Said ramp portions 1601A and 1601B may be varied in geometry depending on the orientation and size of the nasal prongs and the collapsible portion volume.

Figure 37C illustrates an alternative patient interface with a mid length ramp 1601C, comprising a ramp angle between that of Figures 37A and 37B. Such a ramp geometry may be provided for a medium size patient interface for example, falling between the larger size interface of Figure 37A, and smaller size interface of Figure 37B. Put another way, in an example embodiment, the ramp shape and/or angle may be varied, for example in relation to the patient interface size. This may facilitate achievement of a target resistance to flow despite varied change in patient interface size, which may advantageously allow interfaces to be exchanged as required.

Figure 37D illustrates an alternative patient interface with a relatively longer length ramp 1601D, comprising a ramp angle of less than that of Figures 37B and 37C. Such a ramp geometry may be provided for a larger size patient interface for example, larger than the interface of Figure 37B or Figure 37C.

Figures 37B, 37C and 37D show ramp angles α1, α2, and α3 of increasing angle as the size of the patient interface increases as outlined above. It will be understood that angles α1-3 may comprise any angle between substantially 0 and 90 degrees, and may be about 35 degrees for angle α1, corresponding to a smaller patient interface.

In an example embodiment, moving the ramp to a sharper incline (such as in Figure 37B) in a small sized patient interface may move the constriction at the downstream prong towards the outlet of the prong. This reduces the length of the constriction towards the outlet of the prong which reduces the resistance to flow in the downstream prong. Similarly, moving the ramp to a shallower incline (such as in Figures 37A, 37D or 37C) may provide for a change in resistance to flow as required for a change in patient interface size to a medium or larger interface.

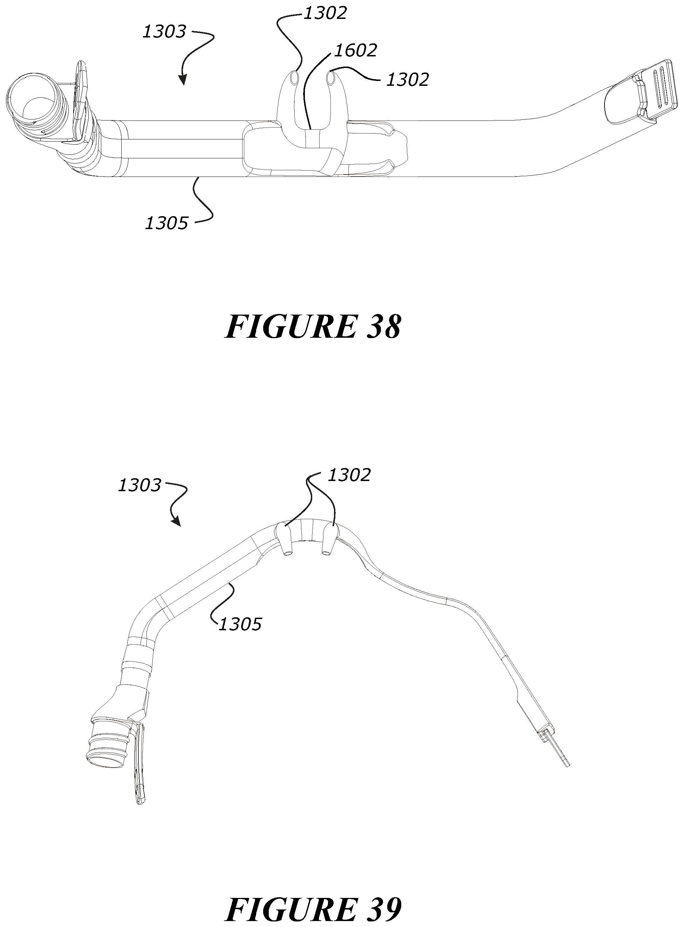

Figures 38 and 39 illustrate a rear and top-down view of an alternative patient interface, for example designed for a smaller patient. The nasal prongs are shown to be more oval in shape at their base, which may advantageously allow the maintaining of a manifold portion width between varying patient interface size, for example to achieve a target resistance to flow. In an example embodiment, an oval base with a wider section in a direction perpendicular to a flow of gas may provide an increased width of the manifold portion of the patient interface, providing a target flow resistance.

In an example embodiment, a wall of the patient interface at a manifold portion may be provided in a thicker or thinner profile to change a manifold width and therefore change or achieve a target resistance to flow. In an example embodiment, a smaller sized patient interface may comprise a thinner wall section in the manifold region, leading to a wider manifold portion, and therefore reduced resistance to flow.

Advantageously, an oval shape with a narrowed section in a direction parallel to the flow of gas may provide for a spacing or gap or a dip between nasal prongs, thereby improving patient comfort.

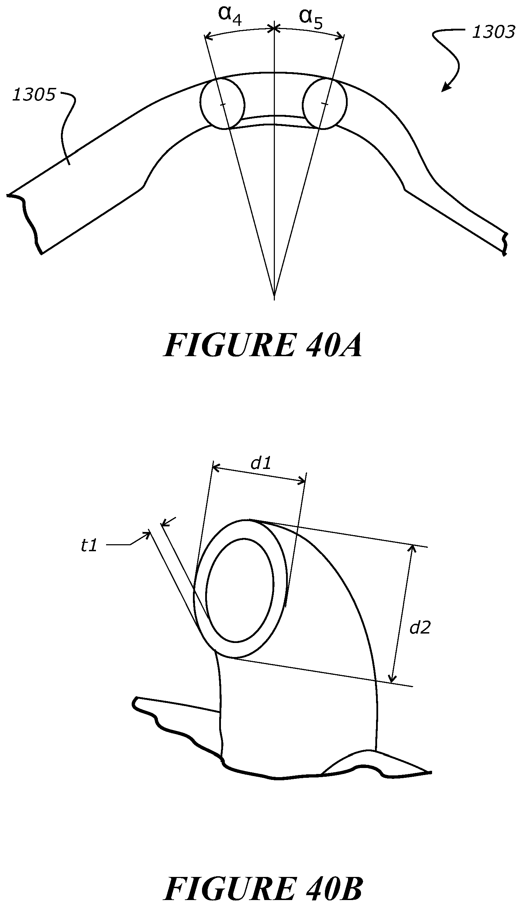

Figure 40A shows a top down sectional view illustrating an example smaller sized patient interface showing the base of nasal prongs 1302 as a substantially oval shape, as described above. Advantageously the oval shape may maintain a manifold portion width as described above. Additionally, Figure 40A illustrates an angle of separation comprising angles α4 and α5 of the prongs, which may be substantially equal or unequal as required to size the patient interface to a patient.

Figure 40B illustrates an example nasal prong 1302. Dimensions t1, d1 and d2 of an outlet of prong 1302 in Figure 40B illustrate that said outlet of said prong may be substantially rounded or substantially oval (in this case oval), and may have varied wall thickness as required. Advantageously this may provide for improved patient comfort, and allow for a gas leakage path, to avoid sealing a patients nare with said prong. It will be understood that d1 may be smaller than d2 to produce a substantially oval nasal prong outlet as illustrated in Figure 40B. It would be appreciated that the shape of each prong may change from its base to its outlet, e.g. from an oval shape to a round shape.

In an example embodiment, the prong outlet may be s-shaped or elongate. The prong outlet may have the edges of the gases outlet cut-out or may be shaped cut-outs to confirm to a surface that has a substantially reverse S-shape, the S-shape being aligned substantially vertically. Alternatively, the prong out may be substantially elliptical or elongated in a substantially vertical direction, or a lower edge of the surface cuts extend across the rear of the prongs to create the cut-out, the surface being a reverse S-shape to obtain the cut-out shape.

Prong outlet shapes may be those as described in US patent no. 8,997,747, the contents of which are hereby incorporated by reference. As described in that specification, one or each of the nasal prongs includes a gases exit cut-out section on the rear side of a nasal prong. The gases exit cut-out or cut-out section gives each of the prongs the appearance of a scoop. The front side of the nasal prong (the side further away from the patient) extends further upwards and inwards from the interface, and forms a guide wall that guides humidified gases into the patient's nasal passage when a prong or pair of nasal prongs is/are in use. The gases exit cut-out in the prong has a sectional area greater than the cross-sectional area of the prong at or close to the point of entry of gases into the prong from a manifold section - that is, the cross-sectional area of the prong is greater at the point where the gases exit the prong (and enter the users nare), in comparison to a point at or close to where the gases enter the prong from the manifold section.

The cut-out section can be formed in various shapes. In an embodiment the cut-out section can be oval in shape when viewed from the rear. That is, when viewed from the rear, the perimeter of the cut-out section describes an oval shape, the top of the oval may optionally be angled slightly inwards towards the other nasal prong. The cut-out section could also be triangular (with one point of the triangle oriented towards the base of the prong, and the other two corners at the topmost inner edges of the cut-out section). The cut-out could also be rectangular in shape.

The cut-out can extend from various positions along the nasal prong. For example, the cut-out section can extend from between halfway and two thirds of the way along the nasal prong, when measured from the top tip of the nasal prong. Alternatively, the cut-out section may extend from less than halfway along the nasal prong, when measured from the top tip of the nasal prong. As a further alternative, the cut-out may extend the entire length of the prong. In another embodiment, the cut-out section may extend from between halfway and two-thirds of the way along the nasal prong.

The cut out can be formed during the moulding process. The prongs may be moulded by injection moulding, casting or vacuum forming. The mould used to produce the desired prong shape can have the cut-out feature built into it.

In alternative embodiments, the cut-out section can be created by cutting across the rear of each of the prongs after these have been formed in an initial forming operation - e.g. after the interface has been moulded in an initial forming operation, the cut-out can be formed by removing material either by machining or by hand.

The reverse S shape can be aligned substantially vertically. After forming, the edges or perimeter of the cut-out section can conform to the surface of the reverse S-shaped surface.

Where, in the foregoing description reference has been made to integers or components having known equivalents thereof, those integers are herein incorporated as if individually set forth.

Although the present disclosure has been described in terms of certain embodiments, other embodiments apparent to those of ordinary skill in the art also are within the scope of this disclosure. Thus, various changes and modifications may be made without departing from the spirit and scope of the disclosure. For instance, various components may be repositioned as desired. Moreover, not all of the features, aspects and advantages are necessarily required to practice the present disclosure. Accordingly, the scope of the present disclosure is intended to be defined only by the claims that follow.

### REPRESENTATIVE FEATURES

1. A patient interface comprising:
   at least one nasal prong or an outlet of said patient interface to be received by a user's nare(s) or mouth;
   a gases delivery side member extending from a side of the at least one nasal prong or said outlet;
   wherein the gases delivery side member comprises:
      a lumen for a flow of gases from an inlet of the patient interface to the at least one nasal prong or said outlet;
      a collapsible portion;
      at least one elbow portion or flexible portion, located substantially at or toward one or both of a downstream end of said gases delivery member or an upstream end of said gases delivery member.
2. The patient interface of clause 1, wherein the collapsible portion allows for a leakage gases flow through the collapsed portion of the conduit when in a collapsed condition, optionally said leakage gases flow is about 15L/min or less; or about 10 L/min or less, or about 10L/min, or about 5L/min to about 10L/min, optionally the leakage gases flow may be measured by a sensor.
3. The patient interface of clause 1 or clause 2, wherein the patient interface comprises a base portion to support the at least one nasal prong, the base portion being substantially the same, or near the same width as a base of the nasal prongs, such that in use the base of the nasal prongs is located on or near a patient's face, optionally to provide for a low profile cannula in a region under a patients nose, optionally the base portion comprises a hollowed section located on the rear side of the base portion.
4. The patient interface of any one of clauses 1 to 3, wherein the base portion is a manifold, the manifold configured to provide a flow of gases to the at least one nasal prong or outlet from the air delivery side member.
5. The patient interface of any one of clauses 1 to 4, wherein the at least one elbow portion, or flexible portion comprises a first elbow or a first flexible portion, the first elbow or first flexible portion is located at an end of the gases delivery side member, and/or collapsible portion near or adjacent an inspiratory tube or the or an inspiratory tube connector, optionally the first elbow or first flexible portion is located between the gases delivery side member and/or the collapsible portion, and an inspiratory tube or the or an inspiratory tube connector.
6. The patient interface of any one of clauses 1 to 5, further comprising an intermediate section located between the first elbow or the first flexible portion and an inspiratory tube or an inspiratory tube connector.
7. The patient interface of clause 6, wherein the intermediate section is located at an angle of about -25 degrees to about 45 degrees from a centreline of the patient interface, optionally, the intermediate section is located at an angle of about 10 degrees from a centreline of the patient interface.
8. The patient interface of clause 6 or clause 7, wherein the intermediate section is located at an angle of about 0 degrees to about 90 degrees from a front face, or a rear face, or a longitudinal axis of the collapsible portion and/or the gases delivery side member, optionally the intermediate section is located at an angle of about 20 degrees to about 60 degrees from a front face, or a rear face, or a longitudinal axis of the collapsible portion and/or the gases delivery side member, optionally the intermediate section is located at an angle of about 25 degrees to about 35 degrees from a front face, or a rear face, or a longitudinal axis of the collapsible portion and/or the gases delivery side member.
9. The patient interface of any one of clauses 6 to 8, wherein the intermediate section is located at an angle of about 0 degrees to about 30 degrees from a lower face, or an upper face, or a longitudinal axis of the gases delivery side member and/or the collapsible portion, optionally the intermediate section is located at an angle of about 5 degrees to about 25 degrees from a lower face, or an upper face, or a longitudinal axis of the gases delivery side member and/or the collapsible portion, optionally the intermediate section is located at an angle of about 15 degrees from a lower face, or an upper face, or a longitudinal axis of the gases delivery side member and/or the collapsible portion, optionally the angle between the intermediate section and the lower face, or an upper face, or a longitudinal axis of the gases delivery side member and/or the collapsible portion is an angle of elevation.
10. The patient interface of any one of clauses 6 to 9, wherein the intermediate section provides for a flow path from the inspiratory tube or inspiratory tube connector to the gases delivery side member and/or the collapsible portion.
11. The patient interface of any one of clauses 5 to 10, wherein the first elbow or first flexible portion provides for or comprises a pivot portion and/or a hinging portion, optionally to allow for relative movement about at least one axis (for example axis A1, or axis A2, or axis A3).
12. The patient interface of clause 11, wherein said at least one axis comprises a first axis (for example axis A1), the first axis being oriented substantially parallel with, or along a height of the gases delivery side member and/or the collapsible portion, optionally said first axis being oriented substantially parallel to a patient's face in use, optionally said first axis being oriented substantially parallel to a portion of the gases delivery side member and/or the collapsible portion configured to contact a user's face, optionally said first axis being oriented substantially parallel to a front face and/or a rear face of the gases delivery side member and/or the collapsible portion, optionally, said first axis being located through (optionally so as to bisect) said the first elbow or first flexible portion.
13. The patient interface of clause 11 or clause 12, wherein said at least one axis comprises a second axis (for example axis A2 or axis A3), the second axis being oriented parallel with, or along a length of the gases delivery side member and/or the collapsible portion and/or an intermediate portion.
14. The patient interface of any one of clauses 1 to 13, wherein the gases delivery side member and/or the collapsible portion is a substantially straight section.
15. The patient interface of any one of clauses 1 to 14 wherein a cross-sectional profile(s) of the collapsible portion, is an obround shape.
16. The patient interface of any one of clauses 1 to 15, wherein opposing walls of the collapsible portion that, in use, contact the user's face and a mask respectively, progressively narrow or reduce to meet at, or until said opposing walls meet at, a thinned region, the thinned region being a thinnest wall thickness region of the cross-sectional profile.
17. The patient interface of any one of clauses 1 to 16, wherein a cross-sectional profile of the collapsible portion comprises at least one substantially thinned region.
18. The patient interface of any one of clauses 1 to 17, wherein a or the thinned region is located at one or both of an upper portion or a lower portion of the collapsible portion, optionally the thinned region may be provided at alternative or substantially opposing locations about an internal perimeter or inner surface of said collapsible portion.
19. The patient interface of any one of clauses 1 to 18, wherein an inner surface of the collapsible portion comprises an angled section in the thinned region.
20. The patient interface of any one of clauses 1 to 19, wherein inner surfaces of the collapsible portion converge to a or said thinned region.
21. The patient interface of any one of clauses 1 to 20, wherein inner surfaces of the collapsible portion meet at a or said thinned region to provide for a pre-determined bending or hinging point.
22. The patient interface of any one of clauses 1 to 21, wherein the external surface of the collapsible portion is substantially round or rounded or is a substantially continuous surface in the area of a or the thinned region.
23. The patient interface of any one of clauses 1 to 22, wherein the gases delivery side member transitions from a gases delivery side member wall thickness that is substantially greater than said the wall thickness of the or a thinned region of said collapsible portion.
24. The patient interface of any one of clauses 1 to 23, wherein the wall thickness of the gases delivery side member along a length of said lumen remains substantially constant or is of a substantially greater wall thickness than the or a wall thickness of the gases delivery side member at the or a thinned region of the collapsible portion.
25. The patient interface of any one of clauses 1 to 24, wherein the or a wall thickness of the gases delivery side member varies along a length of the lumen, the wall thickness reducing or being reduced or substantially less in the region of the thinned region relative to the wall thickness of the gases delivery side member along the length of the lumen.
26. The patient interface of any one of clauses 1 to 25, wherein the wall thickness of gases delivery side member outside of the or a thinned region transitions via a step or a taper to the wall thickness at the thinned region.
27. The patient interface of any one of clauses 1 to 26, wherein at least one elbow portion, or flexible portion comprises a second elbow, or second flexible portion, the second elbow, or second flexible portion located substantially in a region of the gases delivery side member proximate where said second flexible portion is attachable to the at least one nasal prong or outlet, and/or said base portion.
28. The patient interface of clause 27, wherein the second elbow or second flexible portion provides for, or comprises, a pivot portion and/or a hinging portion, optionally to allow for relative movement about at least one axis (for example axis A4, or axis A2, or axis A6).
29. The patient interface of clause 28, wherein said at least one axis comprises a first axis (for example A4), the first axis being oriented parallel with, or along a height of the gases delivery side member and/or the collapsible portion, optionally said first axis being oriented substantially parallel to a patient's face in use, optionally said first axis being oriented substantially parallel to a portion of the gases delivery side member and/or the collapsible portion configured to contact a user's face, optionally said first axis being oriented substantially parallel to a front face and/or a rear face of the gases delivery side member and/or the collapsible portion, optionally, said first axis being located through (optionally so as to bisect) said the second elbow or second flexible portion.
30. The patient interface of clause 28 or clause 29, wherein said at least one axis comprises a second axis (for example axis A2 or axis A6), the second axis being oriented parallel with, or along a length of the gases delivery side member and/or the collapsible portion and/or the base portion.
31. The patient interface of any one of clauses 1 to 30, wherein the gases delivery side member and/or the collapsible conduit is located about 30 degrees to about 80 degrees from a centreline of the patient interface, optionally the gases delivery side member and/or the collapsible conduit is located about 55 to about 60 degrees from a centreline of the patient interface.
32. The patient interface of any one of clauses 1 to 31, wherein the gases delivery side member and/or the collapsible conduit is located about 10 degrees to about 60 degrees from a centreline of the base portion, optionally the gases delivery side member and/or the collapsible conduit is located about 30 degrees to about 35 degrees from a centreline of the base portion.
33. The patient interface of any one of clauses 1 to 32, wherein the gases delivery side member and/or the collapsible portion is relatively stiffer or less flexible or more resilient to a flex, along a length of the gases delivery side member and/or the collapsible portion than along a length of the at least one elbow or flexible portion (optionally one or more of the first elbow, or first flexible portion and/or the second elbow or second flexible portion).
34. The patient interface of any one of clauses 1 to 33, wherein the gases delivery side member and/or the collapsible portion is relatively stiffer or less flexible or more resilient to a flex, along a height of the gases delivery side member and/or the collapsible portion than along a height of the at least one elbow or flexible portion (optionally one or more of the first elbow, or first flexible portion and/or the second elbow or second flexible portion).
35. The patient interface of any one of clauses 1 to 34, wherein the gases delivery side member and/or the collapsible portion is relatively stiffer or less flexible or more resilient to a flex, than the at least one elbow or flexible portion about at least one axis.
36. The patient interface of any one of clauses 1 to 35, wherein the gases delivery side member and/or the collapsible portion is relatively stiffer or less flexible or more resilient to a flex, than one or more of the first elbow, or first flexible portion (optionally one or more of the first elbow, or first flexible portion and/or the second elbow or second flexible portion) about at least one axis.
37. The patient interface of any one of clauses 1 to 36, wherein the gases delivery side member and/or the collapsible portion is relatively stiffer or less flexible or more resilient to a flex, than the at least one elbow or flexible portion about a gases delivery side member and/or the collapsible portion axis (for example axis A5),
   optionally, said gases delivery side member and/or the collapsible portion axis being oriented parallel with, or along a height of the gases delivery side member and/or the collapsible portion,
   optionally said gases delivery side member and/or the collapsible portion axis being oriented substantially parallel to a portion of the gases delivery side member and/or the collapsible portion configured to contact a user's face,
   optionally said gases delivery side member and/or the collapsible portion axis being oriented substantially parallel to a front face and/or a rear face of the gases delivery side member and/or the collapsible portion,
   optionally, the gases delivery side member and/or the collapsible portion axis being located through (optionally so as to bisect) the gases delivery side member and/or the collapsible portion,
   optionally, the gases delivery side member and/or the collapsible portion axis being located through a centre of the gases delivery side member and/or the collapsible portion.
38. The patient interface of clause 36 or 37, wherein said at least one axis comprises a first axis (for example A1), the first axis being oriented parallel with, or along a height of the gases delivery side member and/or the collapsible portion,
   optionally said first axis being oriented substantially parallel to a patient's face in use,
   optionally said first axis being oriented substantially parallel to a portion of the gases delivery side member and/or the collapsible portion configured to contact a user's face,
   optionally said first axis being oriented substantially parallel to a front face and/or a rear face of the gases delivery side member and/or the collapsible portion,
   optionally, said first axis being located through (optionally so as to bisect) said the first elbow or first flexible portion.
39. The patient interface of any one of clauses 36 to 38, wherein said at least one axis comprises a second axis (for example axis A2 and/or axis A3), the second axis being oriented parallel with, or along a length of the gases delivery side member and/or the collapsible portion and/or an intermediate portion.
40. The patient interface of any one of clauses 1 to 39, wherein the gases delivery side member and/or the collapsible portion is relatively stiffer or less flexible or more resilient to a flex, than one or more of the second elbow, or second flexible portion about at least one axis
41. The patient interface of clause 40, wherein said at least one axis comprises a first axis (for example A4), the first axis being oriented parallel with, or along a height of the gases delivery side member and/or the collapsible portion,
   optionally said first axis being oriented substantially parallel to a patient's face in use,
   optionally said first axis being oriented substantially parallel to a portion of the gases delivery side member and/or the collapsible portion configured to contact a user's face,
   optionally said first axis being oriented substantially parallel to a front face and/or a rear face of the gases delivery side member and/or the collapsible portion, optionally, said first axis being located through (optionally so as to bisect) said the second elbow or second flexible portion.
42. The patient interface of clause 40 or clause 41, wherein said at least one axis comprises a second axis (for example axis A2 and/or axis A6), the second axis being oriented parallel with, or along a length of the gases delivery side member and/or the collapsible portion and/or the base portion.
43. The patient interface of any one of clauses 1 to 42, wherein the at least one elbow (optionally one or more of the first elbow, or first flexible portion and/or the second elbow or second flexible portion) are configured to substantially deform before the gases delivery side member and/or the collapsible portion when a force is applied to the or an end (whether an upstream end or a downstream end or both of these ends) of the air delivery arm.
44. The patient interface of any one of clauses 1 to 43, wherein the at least one elbow (optionally one or more of the first elbow, or first flexible portion and/or the second elbow or second flexible portion) is/are configured to isolate or attenuate the gases delivery side member and/or the collapsible portion from forces applied (for example force applied the non-air delivery arm, of the gases delivery side member) to the patient interface,
   optionally to maintain the profile of the collapsible portion,
   optionally to prevent the collapsible conduit from kinking.
45. The patient interface of any one of clauses 1 to 44, wherein the patient interface further comprises a non-air delivering side member extending from a second side of at least one nasal prong or outlet, said gases delivery side member and non-air delivering side member are at different angles with respect to the at least one nasal prong or outlet.
46. A patient interface comprising:
   a manifold and at least one nasal prong or an outlet extending from the manifold to be received by a user's nare or mouth;
   a gases delivery side member extending from a side of the manifold;
   a non-gases delivery side member extending from another side of the manifold;
   wherein at least a part of the non-gases delivery side member comprises an outer wall for, in use, contact with a mask, and an inner wall for, in use, contact with a user's face, where a cross-sectional profile of the at least a part of the non-gases delivery side member comprises the inner wall having a more pronounced outward curve than the outer wall.
47. The patient interface of clause 46, wherein the cross-sectional profile of the at least part of the non-air delivery side is an elongated asymmetric lens shape.
48. The patient interface of any one of clauses 46 or 47, wherein the inner wall has a curved profile with a central, flat region.
49. The patient interface of any one of clauses 46, 47 or 48, wherein the outer wall has a curved profile with a central, flat region.
50. The patient interface of any one of clauses 46 to 49, wherein the outer wall has a flat profile relative to the outward curve of the inner wall.
51. The patient interface of any one of clauses 46 to 50, wherein, relative to a plane taken through meeting edges of the inner and outer walls, the outward curve of the inner wall extends from 1 to 3mm and the outward curve of the outer wall extends 0 to 0.9mm.
52. The patient interface of any one of clauses 46 to 51, wherein edges of the inner and outer walls meet at a point, a taper or a rounded area.
53. The patient interface of any one of clauses 46 to 52, wherein the gases delivery side member is or forms part of a conduit for a flow of gases from an inlet of the patient interface to the manifold.
54. The patient interface of clause 53, wherein the gases delivery side member comprises a collapsible portion adapted to be pinched or flattened to a closed configuration to occlude or substantially occlude the conduit.
55. The patient interface of clause 54, wherein a cross -sectional profile of the collapsible portion includes a thinned region.
56. The patient interface of any one of clauses 46 to 55, wherein the gases delivery side member has an obround shaped cross-sectional profile.
57. The patient interface of any one of clauses 46 to 56, wherein both the non-air delivery side member and air delivery side member are contoured for placement across a user's upper lip.
58. The patient interface of any one of clauses 46 to 57, wherein an outward facing surface of either or both of the non-air delivery side member and air delivery side member is roughened.
59. A patient interface comprising:
   a manifold and at least one nasal prong or an outlet extending from the manifold to be received by a user's nare or mouth;
   at least one non-gases delivery side member extending from a side of the manifold;
   wherein at least a part of the non-gases delivery side member comprises an outer wall for, in use, contact with a mask, and an inner wall for, in use, contact with a user's face, where a cross-sectional profile of the at least a part of the non-gases delivery side member comprises the inner wall having a more pronounced outward curve than the outer wall.
60. A system for providing a flow of respiratory gases to a user, the system comprising:
   a gases flow source;
   a patient interface according to any one of the preceding clauses 1 to 59;
   a conduit providing gases flow between the flow source and the patient interface.

This divisional application is divided from EP19754802.7 (the 'parent application') and the divisional specification as filed comprises the content of the parent application, including the original claims recited as 'representative features' above. The scope of this disclosure therefore includes the full content of the parent application. In any case, protection may be sought for any features disclosed in the parent application as filed.

## Claims

1. A patient interface comprising:
a manifold and at least one nasal prong or an outlet extending from the manifold to be received by a user's nare or mouth; and
at least one non-gases delivery side member extending from a side of the manifold, wherein at least a part of the non-gases delivery side member comprises an outer wall for, in use, contact with a mask, and an inner wall for, in use, contact with a user's face, where a cross-sectional profile of the at least a part of the non-gases delivery side member comprises the inner wall having a more pronounced outward curve than the outer wall.

2. The patient interface of claim 1, wherein the cross-sectional profile of the at least part of the non-gases delivery side member is an elongated asymmetric lens shape.

3. The patient interface of claim 1, wherein the inner wall has a curved profile with a central, flat region.

4. The patient interface of claim 1, wherein the outer wall has a curved profile with a central, flat region.

5. The patient interface of claim 1, wherein the outer wall has a flat profile relative to the outward curve of the inner wall.

6. The patient interface of claim 1, wherein, relative to a plane taken through meeting edges of the inner and outer walls, the inner wall has a greater depth compared to the outer wall.

7. The patient interface of claim 1, wherein, relative to a plane taken through meeting edges of the inner and outer walls, the outward curve of the inner wall extends from 1mm to 3mm and the outward curve of the outer wall extends from 0mm to 0.9mm.

8. The patient interface of claim 1, wherein edges of the inner and outer walls meet at a point, a taper or a rounded area.

9. The patient interface of claim 1, wherein the non-gases delivery side member is contoured for placement across a user's upper lip.

10. The patient interface of claim 1, wherein an outward facing surface of the non-gases delivery side member is roughened.

11. The patient interface of claim 1, wherein the non-gases delivery side member comprises a connection feature for connection of the non-gases delivery side member to a head strap and/or an associated headgear.

12. The patient interface of claim 11, wherein the connection feature comprises one or a pair or a plurality of slots.

13. The patient interface of claim 1, wherein the non-gases delivery side member comprises a solid arm.

14. The patient interface of claim 13, wherein the solid arm is in the form of a strap or part thereof.

15. The patient interface of claim 13, wherein the solid arm has a constant cross-section.
